# EUROPEAN PATENT APPLICATION

(11) **EP 3 219 329 A1**
(43) Date of publication of application: **20.09.2017**
(21) Application number: 16160962.3
(22) Date of filing: 17.03.2016
(51) Int. Cl.: A61K 45/06, A61K 31/4439, A61K 31/519, A61K 31/5377, A61P 35/00, A61P 35/02

(54) **COMBINATIONS OF COPANLISIB**

(71) Applicant: Bayer Pharma Aktiengesellschaft, 13353 Berlin (DE)
(72) Inventor: Lange, Martin, 10115 Berlin (DE)
(74) Representative: BIP Patents

(57) **Abstract**

The present invention relates to :
* combinations of :
component A: one or more 2,3-dihydroimidazo[1,2-c]quinazoline compounds of general formula (A1) or (A2) as defined herein, or a physiologically acceptable salt, solvate, hydrate or stereoisomer thereof ;
component B : one or more IRAK4-inhibiting compounds of general formula (I) as defined herein,or a physiologically acceptable salt, solvate, hydrate or stereoisomer thereof ;
in which optionally some or all of the components are in the form of a pharmaceutical formulation which is ready for use to be administered simultaneously, concurrently, separately or sequentially.
dependently of one another by the oral, intravenous, topical, local installations, intraperitoneal or nasal route ;
* use of such combinations for the preparation of a medicament for the treatment or prophylaxis of a cancer ; and
* a kit comprising such a combination.

## Description

The present invention relates to combinations of :
component A: one or more 2,3-dihydroimidazo[1,2-c]quinazoline compounds of general formula (A1) or (A2) as defined herein, or a physiologically acceptable salt, solvate, hydrate or stereoisomer thereof;
   and
component B : one or more IRAK4-inhibiting compounds of general formula (I) as defined herein, or a physiologically acceptable salt, solvate, hydrate or stereoisomer thereof; and, optionally,
component C : one or more further pharmaceutical agents ;
in which optionally either or both of components A and B in any of the above-mentioned combinations are in the form of a pharmaceutical formulation which is ready for use to be administered simultaneously, concurrently, separately or sequentially. The components may be administered independnently of one another by the oral, intravenous, topical, local installations, intraperitoneal or nasal route.

Another aspect of the present invention relates to the use of such combinations as described *supra* for the preparation of a medicament for the treatment or prophylaxis of a cancer, particularly non-Hodgkin's lymphoma (hereinafter abbreviated to "NHL"), particularly 1st line, 2nd line, relapsed, refractory, indolent or aggressive non-Hodgkin's lymphoma (NHL), in particular follicular lymphoma (hereinafter abbreviated to "FL"), chronic lymphocytic leukaemia (hereinafter abbreviated to "CLL"), marginal zone lymphoma (hereinafter abbreviated to "MZL"), diffuse large B-cell lymphoma (hereinafter abbreviated to "DLBCL"), mantle cell lymphoma (MCL), transformed lymphoma (hereinafter abbreviated to "TL"), or peripheral T-cell lymphoma (hereinafter abbreviated to "PTCL").

In a further aspect, the present invention relates to a kit comprising a combination of
component A : one or more 2,3-dihydroimidazo[1,2-c]quinazoline compounds of general formula (A1) or (A2) as defined herein, or a physiologically acceptable salt, solvate, hydrate or stereoisomer thereof;
   and
component B : one or more IRAK4-inhibiting compounds of general formula (I) as defined herein, or a physiologically acceptable salt, solvate, hydrate or stereoisomer thereof;
in which optionally either or both of said components A) and B) in any of the above-mentioned combinations are in the form of a pharmaceutical formulation which is ready for use to be administered simultaneously, concurrently, separately or sequentially. The components may be administered independnently of one another by the oral, intravenous, topical, local installations, intraperitoneal or nasal route.

### BACKGROUND OF THE INVENTION

### Combinations of PI3K Inhibitors and IRAK-4 inhibitors:

Cancer is the second most prevalent cause of death in the United States, causing 450,000 deaths per year. While substantial progress has been made in identifying some of the likely environmental and hereditary causes of cancer, there is a need for additional therapeutic modalities that target cancer and related diseases. In particular there is a need for therapeutic methods for treating diseases associated with dysregulated growth / proliferation.
Cancer is a complex disease arising after a selection process for cells with acquired functional capabilities like enhanced survival / resistance towards apoptosis and a limitless proliferative potential. Thus, it is preferred to develop drugs for cancer therapy addressing distinct features of established tumors.

The PI3K signaling pathway is one of the prominent pathways that promote tumor cell survival. PI3K is activated by many cancer related receptor tyrosine kinases (e.g. PDGFR, EGFR, HER2/3, or IGF-1R), cell adhesion molecules, GPCR, and oncogenic proteins (such as Ras). The PI3K pathway activation by genetic alteration of PI3K (activation mutation and/or amplification) and/or loss-of-function of the tumour suppressor PTEN are frequently found in many tumors. Furthermore, activation of PI3K is one of the major mechanisms causing the resistance of tumors to radio-, chemo- and targeted therapeutics.

Once PI3K is activated, it catalyzes the generation of PIP3 from PIP2. The biological active PIP3 binds to the pleckstrin homology (PH) domains of PDK-1, AKT, and other PH-domain containing proteins, such as Rho and PLC. As the consequence of binding to PIP3, these proteins are translocated to the cell membrane and are subsequently activated to induce tumor cell proliferation, survival, invation and migration.

Human IRAK4 (Interleukin-1 receptor-associated kinase 4) plays a key role in the activation of the immune system. It is for this reason that this kinase is an important therapeutic target molecule for the development of inflammation inhibiting substances. IRAK4 is investigated from a multiplicity of cells and mediates the signal transduction of Toll-like receptors (TLR), except TLR3, as well as Interleukin (IL)-1 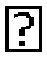 receptor family, comprising IL-1R (receptor), IL-18R, IL-33R und IL-36R (Janewayfamily consisting of the IL-1R (receptor), IL-18R, IL-33R and IL-36R (Janeway and Medzhitov, Annu. Rev. Immunol., 2002; Dinarello, Annu. Rev. Immunol., 2009; Flannery and Bowie, Biochemical Pharmacology, 2010).
Neither IRAK4 knockout mice nor human cells from patients lacking IRAK4 react to stimulation by TLRs (except for TLR3) and the IL-1β family (Suzuki, Suzuki, et al., Nature, 2002; Davidson, Currie, et al., The Journal of Immunology, 2006; Ku, von Bernuth, et al., JEM, 2007; Kim, Staschke, et al., JEM, 2007).

The binding of the TLR ligands or the ligands of the IL-1β family to the respective receptor leads to recruitment and binding of MyD88 [Myeloid differentiation primary response gene (88)] to the receptor. As a result, MyD88 interacts with IRAK4, resulting in the formation of an active complex which interacts with and activates the kinases IRAK1 or IRAK2 (Kollewe, Mackensen, et al., Journal of Biological Chemistry, 2004; Precious et al., J. Biol. Chem., 2009). As a result of this, the NF (nuclear factor)-kB signalling pathway and the MAPK (mitogen-activated protein kinase) signal pathway is activated (Wang, Deng, et al., Nature, 2001). The activation both of the NF-kB signal pathway and of the MAPK signal pathway leads to processes associated with different immune processes. For example, there is increased expression of various inflammatory signal molecules and enzymes such as cytokines, chemokines and COX-2 (cyclooxygenase-2), for example, and increased mRNA stability of inflammation-associated genes, for example COX-2, IL-6, IL-8 (Holtmann, Enninga, et al., Journal of Biological Chemistry, 2001; Datta, Novotny, et al., The Journal of Immunology, 2004). Furthermore, these processes may be associated with the proliferation and differentiation of particular cell types, for example monocytes, macrophages, dendritic cells, T cells and B cells (Wan, Chi, et al., Nat Immunol, 2006; McGettrick and J. O'Neill, British Journal of Haematology, 2007).

This also applies to some oncological disorders. Particular lymphomas, for example ABC-DLBCL (activated B-cell-like diffuse large-cell B-cell lymphoma), mantle cell lymphoma and Waldenstrom's disease, and also chronic lymphatic leukaemia, melanoma and liver cell carcinoma, are characterized by mutations in MyD88 or changes in MyD88 activity which can be treated by an IRAK4 inhibitor (Ngo, Young, et al., Nature, 2011; Puente, Pinyol, et al., Nature, 2011; Srivastava, Geng, et al., Cancer Research, 2012; Treon, Xu, et al., New England Journal of Medicine, 2012; Choi, Kim, et al., Human Pathology, 2013; Liang, Chen, et al., Clinical Cancer Research, 2013). In addition, MyD88 plays an important role in ras-dependent tumours, and so IRAK4 inhibitors are also suitable for treatment thereof (Kfoury, A., K. L. Corf, et al., Journal of the National Cancer Institute, 2013).

The prior art discloses a multitude of IRAK4 inhibitors (see, for example, Annual Reports in Medicinal Chemistry (2014), 49, 117 - 133).

US8293923 and US20130274241 disclose IRAK4 inhibitors having a 3-substituted indazole structure. There is no description of 2-substituted indazoles.

WO2013106254 and WO2011153588 disclose 2,3-disubstituted indazole derivatives.

WO2007091107 describes 2-substituted indazole derivatives for the treatment of Duchenne muscular dystrophy. The compounds disclosed do not have 6-hydroxyalkyl substitution.

The unpublished application EP13198463.5 (filing date 19 December 2013) describes indazoles wherein the alkyl radical and 2 position is substituted by a carboxamide structure.

Diffuse large B-cell lymphoma (DLBCL) is the most common subtype of aggressive Non-Hodgkin Lymphoma (NHL). DLBCL can be classified into three different molecular cell-of-origin subtypes: germinal centre B-cell (GCB), activated B-cell (ABC) and primary mediastinal B-cell lymphoma (PMBL). GCB DLBCL frequently has Bcl2 translocations and most result in activation of Bcl6. Mutations of EZH2 (21%) are specific for this subtype. Loss-of-PTEN (55% of GCB-DLBCL cases) results in activation of the PI3K pathway. Constitutive activation of the NF-kappaB and BCR oncogenic signalling pathways by mutations in *Myd88, CARD11* and *CD79* are the hallmark of ABC DLBCL. Thus, DLBCL has significant molecular heterogeneity (Table 1) and shows diverse prognosis. Therefore, identification of combinations of novel agents targeting the driver oncogenic signalling pathways is needed for increasing the cure rate of DLBCL.

**Table 1 Oncogenic signalling pathways in DLBCL**

| **DLBL Subtype** | **Cell of Origin** | **Oncogenic Mechanisms** | **Potential Targets** |
|---|---|---|---|
| GCB | Germinal centre B-cell | BCL2 translocation | BCL6 |
| | | EZH2 mutations | EZH₂ |
| | | PTEN deletions | PI3K/Akt |
| | | Loss of PTEN expression | Bcl2 |
| | | | Epigenetics |
| ABC | Post-germinal centre B-cell | NF-κB activation | BCR |
| | | CARD11 mutations | BTK |
| | | MYD88 mutations | PI3K |
| | | CD79B mutations | VBM complex |
| | | A20 deletions | IRAK-4 |
| | | | JAK-STAT |
| PMBL | Post-thymic B-cell | NF-κB activation | JAK-STAT |
| | | 9p24 amplification | PD-1 |
| | | REL amplification | |
| | | JAK2 mutations | |
| | | CIITA translocations | |

As described in the present invention, 2-amino-N-[7-methoxy-8-(3-morpholin-4-ylpropoxy)-2,3-dihydroimidazo[1,2-c]quinazolin-5-yl]pyrimidine-5-carboxamide dihydrochloride, (a highly selective pan-Class I PI3K inhibitor with predominant activity against PI3Kα and PI3Kδ) and IRAK4-inhibiting compounds of general formula (I) as defined herein, were investigated as combination in inhibiting aggressive NHL proliferation and apoptosis compared to each single agent.

Unexpectedly, and this represents a basis of the present invention, when combinations of:
- component A : one or more 2,3-dihydroimidazo[1,2-c]quinazoline compounds of general formula (A1) or (A2), or a physiologically acceptable salt, solvate, hydrate or stereoisomer thereof, as described and defined herein; with
- component B : one or more IRAK4-inhibiting compounds of general formula (I) as defined herein, or a physiologically acceptable salt, solvate, hydrate or stereoisomer thereof, as described and defined herein ;
were evaluated for the treatment of non-Hodgkin's lymphoma (hereinafter abbreviated to "NHL"), particularly 1st line, 2nd line, relapsed, refractory, indolent or aggressive non-Hodgkin's lymphoma (NHL), in particular follicular lymphoma (hereinafter abbreviated to "FL"), chronic lymphocytic leukaemia (hereinafter abbreviated to "CLL"), marginal zone lymphoma (hereinafter abbreviated to "MZL"), diffuse large B-cell lymphoma (hereinafter abbreviated to "DLBCL"), mantle cell lymphoma (MCL), transformed lymphoma (hereinafter abbreviated to "TL"), or peripheral T-cell lymphoma (hereinafter abbreviated to "PTCL"), synergistically increased anti-tumor activities were demonstrated with these combinations compared to each monotherapy, providing a fundamental rationale for the clinical combination therapy using PI3K inhibitors-IRAK-4 inhibitors.

To the Applicant's knowledge, no generic or specific disclosure or suggestion in the prior art is known that either combinations of:
component A : one or more 2,3-dihydroimidazo[1,2-c]quinazoline compounds of general formula (A1) or (A2) as defined herein, or a physiologically acceptable salt, solvate, hydrate or stereoisomer thereof;
component B : one or more IRAK4-inhibiting compounds of general formula (I) as defined herein, or a physiologically acceptable salt, solvate, hydrate or stereoisomer thereof;
in which optionally either or both of said components A and B of any of the above-mentioned combinations are in the form of a pharmaceutical formulation which is ready for use to be administered simultaneously, concurrently, separately or sequentially, would be effective in the treatment or prophylaxis of cancer, particularly non-Hodgkin's lymphoma (hereinafter abbreviated to "NHL"), particularly 1st line, 2nd line, relapsed, refractory, indolent or aggressive non-Hodgkin's lymphoma (NHL), in particular follicular lymphoma (hereinafter abbreviated to "FL"), chronic lymphocytic leukaemia (hereinafter abbreviated to "CLL"), marginal zone lymphoma (hereinafter abbreviated to "MZL"), diffuse large B-cell lymphoma (hereinafter abbreviated to "DLBCL"), mantle cell lymphoma (MCL), transformed lymphoma (hereinafter abbreviated to "TL"), or peripheral T-cell lymphoma (hereinafter abbreviated to "PTCL").

Based on the action of the testing compounds described in this invention, the combinations of the present invention as described and defined herein, show a beneficial effect in the treatment of cancer, particularly non-Hodgkin's lymphoma (hereinafter abbreviated to "NHL"), particularly 1st line, 2nd line, relapsed, refractory, indolent or aggressive non-Hodgkin's lymphoma (NHL), in particular follicular lymphoma (hereinafter abbreviated to "FL"), chronic lymphocytic leukaemia (hereinafter abbreviated to "CLL"), marginal zone lymphoma (hereinafter abbreviated to "MZL"), diffuse large B-cell lymphoma (hereinafter abbreviated to "DLBCL"), mantle cell lymphoma (MCL), transformed lymphoma (hereinafter abbreviated to "TL"), or peripheral T-cell lymphoma (hereinafter abbreviated to "PTCL").

Accordingly, in accordance with a first aspect, the present invention relates to combinations of :
component A : one or more 2,3-dihydroimidazo[1,2-c]quinazoline compounds of general formula (A1) or (A2) as defined herein, or a physiologically acceptable salt, solvate, hydrate or stereoisomer thereof;
component B : one or more IRAK4-inhibiting compounds of general formula (I) as defined herein, or a physiologically acceptable salt, solvate, hydrate or stereoisomer thereof;
in which optionally either or both of said components A and B) of any of the above-mentioned combinations are in the form of a pharmaceutical formulation which is ready for use to be administered simultaneously, concurrently, separately or sequentially. The components may be administered independnently of one another by the oral, intravenous, topical, local installations, intraperitoneal or nasal route.

In accordance with a second aspect, of the present invention relates to the use of any of such combinations as described *supra* for the preparation of a medicament for the treatment or prophylaxis of a cancer, particularly non-Hodgkin's lymphoma (hereinafter abbreviated to "NHL"), particularly 1st line, 2nd line, relapsed, refractory, indolent or aggressive non-Hodgkin's lymphoma (NHL), in particular follicular lymphoma (hereinafter abbreviated to "FL"), chronic lymphocytic leukaemia (hereinafter abbreviated to "CLL"), marginal zone lymphoma (hereinafter abbreviated to "MZL"), diffuse large B-cell lymphoma (hereinafter abbreviated to "DLBCL"), mantle cell lymphoma (MCL), transformed lymphoma (hereinafter abbreviated to "TL"), or peripheral T-cell lymphoma (hereinafter abbreviated to "PTCL").

Further, in accordance with a third aspect, the present invention relates to a kit comprising a combination of:
component A : one or more 2,3-dihydroimidazo[1,2-c]quinazoline compounds of general formula (A1) or (A2) as defined herein, or a physiologically acceptable salt, solvate, hydrate or stereoisomer thereof;
component B : one or more IRAK4-inhibiting compounds of general formula (I) as defined herein, or a physiologically acceptable salt, solvate, hydrate or stereoisomer thereof;
in which optionally either or both of components A and B in any of the above-mentioned combinations are in the form of a pharmaceutical formulation which is ready for use to be administered simultaneously, concurrently, separately or sequentially. The components may be administered independnently of one another by the oral, intravenous, topical, local installations, intraperitoneal or nasal route.

### Detailed description of the Invention

In accordance with an embodiment of the above-mentioned aspects of the present invention, said combinations are of:
component A : which is one or more 2,3-dihydroimidazo[1,2-c]quinazoline compounds of general formula (A1) : 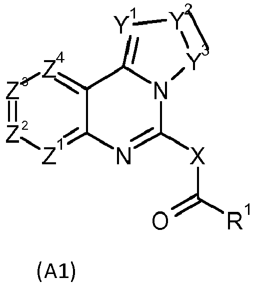 wherein
   - X: represents CR⁵R⁶ or NH;
   - Y¹: represents CR³ or N;
   Chemical bond between Y²------Y³ represents a single bond or double bond,
   with the proviso that when the Y²------Y³ represents a double bond,
   Y² and Y³ independently represent CR⁴ or N, and
   when Y²------Y³ represents a single bond, Y² and Y³ independently represent CR³R⁴ or NR⁴;
   Z¹, Z², Z³ and Z⁴ independently represent CH , CR² or N;
   - R¹: represents aryl optionally having 1 to 3 substituents selected from R¹¹, C₃₋₈ cycloalkyl optionally having 1 to 3 substituents selected from R¹¹,
   C₁₋₆ alkyl optionally substituted by
   aryl, heteroaryl, C₁₋₆ alkoxyaryl, aryloxy, heteroaryloxy or one or more halogen,
   C₁₋₆ alkoxy optionally substituted by
   carboxy, aryl, heteroaryl, C₁₋₆ alkoxyaryl, aryloxy, heteroaryloxy or one or more halogen,
   or
   a 3 to 15 membered mono- or bi-cyclic heterocyclic ring that is saturated or unsaturated, and contains 1 to 3 heteroatoms selected from the group consisting of N, O and S, and optionally having 1 to 3 substituents selected from R¹¹ wherein
   R¹¹ represents
   halogen, nitro, hydroxy, cyano, carboxy, amino, N-(C₁₋₆alkyl)amino, N-(hydroxyC₁₋₆alkyl)amino, N,N-di(C₁₋₆alkyl)amino, N-(C₁₋₆acyl)amino, N-(formyl)-N-(C₁₋₆alkyl)amino, N-(C₁₋₆alkanesulfonyl) amino, N-(carboxyC₁₋₆alkyl)-N-(C₁₋₆alkyl)amino, N-(C₁₋₆alkoxycabonyl)amino, N-[N,N-di(C₁₋₆alkyl)amino methylene]amino, N-[N,N-di(C₁₋₆alkyl)amino (C₁₋₆ alkyl)methylene]amino, N-[N,N-di(C₁₋₆alkyl)amino C₂₋₆alkenyl]amino, aminocarbonyl, N-(C₁₋₆alkyl)aminocarbonyl, N,N-di(C₁₋₆alkyl)aminocarbonyl, C₃₋₈cycloalkyl, C₁₋₆ alkylthio, C₁₋₆alkanesulfonyl, sulfamoyl, C₁₋₆alkoxycarbonyl,
   N-arylamino wherein said aryl moiety is optionally having 1 to 3 substituents selected from R¹⁰¹, N-(aryl C₁₋₆alkyl)amino wherein said aryl moiety is optionally having 1 to 3 substituents selected from R¹⁰¹, aryl C₁₋₆alkoxycarbonyl wherein said aryl moiety is optionally having 1 to 3 substituents selected from R¹⁰¹,
   C₁₋₆alkyl optionally substituted by
   mono-, di- or tri- halogen, amino, N-(C₁₋₆alkyl)amino or N,N-di(C₁₋₆alkyl)amino,
   C₁₋₆alkoxy optionally substituted by
   mono-, di- or tri- halogen, N-(C₁₋₆alkyl)sulfonamide, or N-(aryl)sulfonamide,
   or
   a 5 to 7 membered saturated or unsaturated ring having 1 to 3 heteroatoms selected from the group consisting of O, S and N, and optionally having 1 to 3 substituents selected from R¹⁰¹
   wherein
   R¹⁰¹ represents
   halogen, carboxy, amino, N-(C₁₋₆ alkyl)amino, N,N-di(C₁₋₆alkyl)amino, aminocarbonyl, N-(C₁₋₆alkyl)aminocarbonyl, N,N-di(C₁₋₆alkyl)aminocarbonyl, pyridyl,
   C₁₋₆ alkyl optionally substituted by cyano or mono- di- or tri- halogen,
   or
   C₁₋₆alkoxy optionally substituted by cyano, carboxy, amino, N-(C₁₋₆ alkyl)amino, N,N-di(C₁₋₆alkyl)amino, aminocarbonyl, N-(C₁₋₆alkyl)aminocarbonyl, N,N-di(C₁₋₆alkyl)aminocarbonyl or mono-, di- or tri- halogen;
   - R²: represents hydroxy, halogen, nitro, cyano, amino, N-(C₁₋₆alkyl)amino, N,N-di(C₁₋₆alkyl)amino, N-(hydroxyC₁₋₆alkyl)amino, N-(hydroxyC₁₋₆alkyl)-N-(C₁₋₆alkyl)amino, C₁₋₆acyloxy, aminoC₁₋₆acyloxy, C₂₋₆alkenyl, aryl,
   a 5-7 membered saturated or unsaturated heterocyclic ring having 1 to 3 heteroatoms selected from the group consisting O, S and N, and optionally substituted by
   hydroxy, C₁₋₆ alkyl, C₁₋₆ alkoxy, oxo, amino, amino C₁₋₆alkyl, N-(C₁₋₆alkyl)amino, N,N-di(C₁₋₆alkyl)amino, N-(C₁₋₆ acyl)amino, N-(C₁₋₆alkyl)carbonylamino, phenyl, phenyl C₁₋₆ alkyl, carboxy, C₁₋₆alkoxycarbonyl, aminocarbonyl, N-(C₁₋₆alkyl)aminocarbonyl, or N,N-di(C₁₋₆alkyl)amino,
   -C(O)- R²⁰
   wherein
   R²⁰ represents C₁₋₆ alkyl, C₁₋₆ alkoxy, amino, N-(C₁₋₆alkyl)amino, N,N-di(C₁₋₆alkyl)amino, N-(C₁₋₆ acyl)amino, or a 5-7 membered saturated or unsaturated heterocyclic ring having 1 to 3 heteroatoms selected from the group consisting O, S and N, and optionally substituted by
   C₁₋₆ alkyl, C₁₋₆ alkoxy, oxo, amino, N-(C₁₋₆alkyl)amino, N,N-di(C₁₋₆alkyl)amino, N-(C₁₋₆ acyl)amino, phenyl, or benzyl,
   C₁₋₆ alkyl optionally substituted by R²¹
   or
   C₁₋₆ alkoxy optionally substituted by R²¹
   wherein
   - R²¹: represents cyano, mono-, di or tri- halogen, hydroxy, amino, N-(C₁₋₆alkyl)amino, N,N-di(C₁₋₆alkyl)amino, N- (hydroxyC₁₋₆ alkyl) amino, N- (halophenylC₁₋₆ alkyl) amino, amino C₂₋₆ alkylenyl, C₁₋₆ alkoxy, hydroxyC₁₋₆ alkoxy, -C(O)- R²⁰¹,-NHC(O)- R²⁰¹, C₃₋₈cycloalkyl, isoindolino, phthalimidyl, 2-oxo-1,3-oxazolidinyl, aryl or a 5 or 6 membered saturated or unsaturated heterocyclic ring having 1 to 4 heteroatoms selected from the group consisting O, S and N optionally substituted by
   hydroxy, C₁₋₆ alkyl, C₁₋₆ alkoxy, C₁₋₆ alkoxycarbonyl, hydroxyC₁₋₆ alkoxy, oxo, amino, aminoC₁₋₆alkyl, N-(C₁₋₆alkyl)amino, N,N-di(C₁₋₆alkyl)amino, N-(C₁₋₆ acyl)amino, or benzyl,
   wherein
   R²⁰¹ represents hydroxy, amino, N-(C₁₋₆alkyl)amino, N,N-di(C₁₋₆alkyl)amino, N-(halophenylC₁₋₆ alkyl) amino, C₁₋₆alkyl, aminoC₁₋₆ alkyl, aminoC₂₋₆ alkylenyl, C₁₋₆ alkoxy, a 5 or 6 membered saturated or unsaturated heterocyclic ring having 1 to 4 heteroatoms selected from the group consisting O, S and N optionally substituted by
   hydroxy, C₁₋₆ alkyl, C₁₋₆ alkoxy, C₁₋₆ alkoxycarbonyl, hydroxyC₁₋₆ alkoxy, oxo, amino, N-(C₁₋₆alkyl)amino, N,N-di(C₁₋₆alkyl)amino, N-(C₁₋₆ acyl)amino or benzyl;
   - R³: represents hydrogen, halogen, aminocarbonyl, or C₁₋₆ alkyl optionally substituted by aryl C₁₋₆ alkoxy or mono-, di- or tri- halogen;
   - R⁴: represents hydrogen or C₁₋₆ alkyl;
   - R⁵: represents hydrogen or C₁₋₆ alkyl; and
   - R⁶: represents halogen, hydrogen or C₁₋₆ alkyl ;
or a physiologically acceptable salt, solvate, hydrate or stereoisomer thereof;
said compounds are published as compounds of general formulae I, I-a, and I-b in International patent application PCT/EP2003/010377, published as WO 04/029055 A1 on April 08, 2004, which is incorporated herein by reference in its entirety. In WO 04/029055, said compounds of general formula I, I-a and I-b are described on pp. 6 *et seq.,* they may be synthesized according to the methods given therein on pp. 26 *et seq.,* and are exemplified as specific compound Examples 1-1 to 1-210 on pp. 47 to 106, specific compound Examples 2-1 to 2-368 on pp. 107 to 204, specific compound Examples 3-1 to 3-2 on pp. 205 to 207, and as specific compound Examples 4-1 to 4-2 on pp. 208 to 210, therein.

Such a compound, 2-amino-N-[7-methoxy-8-(3-morpholin-4-ylpropoxy)-2,3-dihydroimidazo[1,2-c]quinazolin-5-yl]pyrimidine-5-carboxamide dihydrochloride, (which is hereinafter referred to as "compound A" or "cpd. A") is published in international patent application PCT/EP2012/055600, published as WO 2012/136553 on October 11, 2012, (which is incorporated herein by reference in its entirety), as the compound of Examples 1 and 2 : 2-amino-N-[7-methoxy-8-(3-morpholin-4-ylpropoxy)-2,3-dihydroimidazo[1,2-c]quinazolin-5-yl]pyrimidine-5-carboxamide dinydrochloride: it may be synthesized according to the methods given in said Examples 1 and 2.

Said component A may be in the form of a pharmaceutical formulation which is ready for use to be administered simultaneously, concurrently, separately or sequentially. The components may be administered independnently of one another by the oral, intravenous, topical, local installations, intraperitoneal or nasal route.

In accordance with another embodiment of the above-mentioned aspects of the present invention, said combinations are of :
component A : which is one or more 2,3-dihydroimidazo[1,2-c]quinazoline compounds of general formula (A1), *supra,* which is selected from the list consisting of specific compound Examples 1-1 to 1-210 on pp. 47 to 106, specific compound Examples 2-1 to 2-368 on pp. 107 to 204, specific compound Examples 3-1 to 3-2 on pp. 205 to 207, and specific compound Examples 4-1 to 4-2 on pp. 208 to 210, of in International patent application PCT/EP2003/010377, published as WO 04/029055 A1 on April 08, 2004, which is incorporated herein by reference in its entirety;
or a physiologically acceptable salt, solvate, hydrate or stereoisomer thereof.

Such a component A may be : 2-amino-N-[7-methoxy-8-(3-morpholin-4-ylpropoxy)-2,3-dihydroimid-azo[1,2-c]quinazolin-5-yl]pyrimidine-5-carboxamide dihydrochloride, (which is hereinafter referred to as "compound A" or "cpd. A") is published in international patent application PCT/EP2012/055600, published as WO 2012/136553 on October 11, 2012, (which is incorporated herein by reference in its entirety), as the compound of Examples 1 and 2 : 2-amino-N-[7-methoxy-8-(3-morpholin-4-ylpropoxy)-2,3-dihydroimidazo[1,2-c]quinazolin-5-yl]pyrimidine-5-carboxamide dinydrochloride: it may be synthesized according to the methods given in said Examples 1 and 2.

Said component A may be in the form of a pharmaceutical formulation which is ready for use to be administered simultaneously, concurrently, separately or sequentially. The components may be administered independnently of one another by the oral, intravenous, topical, local installations, intraperitoneal or nasal route.

As mentioned *supra,* said specific compound Examples may be synthesized according to the methods given in WO 04/029055 A1 on pp. 26 *et seq..*

As mentioned *supra,* 2-amino-N-[7-methoxy-8-(3-morpholin-4-ylpropoxy)-2,3-dihydroimidazo[1,2-c]quinazolin-5-yl]pyrimidine-5-carboxamide dihydrochloride, (which is hereinafter referred to as "compound A" or "cpd. A") may be synthesized as described in Examples 1 and 2 of international patent application PCT/EP2012/055600, published as WO 2012/136553 on October 11, 2012, (which is incorporated herein by reference in its entirety).

In accordance with another embodiment of the above-mentioned aspects of the present invention, said combinations are of :
component A : which is one or more 2,3-dihydroimidazo[1,2-c]quinazoline compounds of general formula (A2): 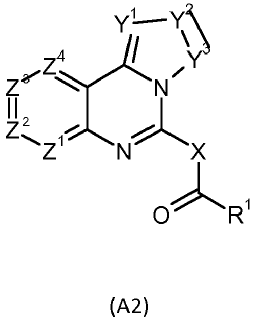 in which:
   - X: represents CR⁵R⁶or NH;
   - Y¹: represents CR³ or N;
   the chemical bond between Y²------Y³ represents a single bond or double bond,
   with the proviso that when the Y²------Y³ represents a double bond, Y² and Y³ independently represent CR⁴ or N, and
   when Y²------Y³ represents a single bond, Y² and Y³ independently represent CR³R⁴ or NR⁴;
   Z¹, Z², Z³ and Z⁴ independently represent CH , CR² or N;
   - R¹: represents aryl optionally having 1 to 3 substituents selected from R¹¹, C₃₋₈ cycloalkyl optionally having 1 to 3 substituents selected from R¹¹,
   C₁₋₆ alkyl optionally substituted by aryl, heteroaryl, C₁₋₆ alkoxyaryl, aryloxy, heteroaryloxy or one or more halogen,
   C₁₋₆ alkoxy optionally substituted by carboxy, aryl, heteroaryl, C₁₋₆ alkoxyaryl, aryloxy, heteroaryloxy or one or more halogen,
   or
   a 3 to 15 membered mono- or bi-cyclic heterocyclic ring that is saturated or unsaturated, optionally having 1 to 3 substituents selected from R¹¹, and contains 1 to 3 heteroatoms selected from the group consisting of N, O and S,
   wherein
   R¹¹ represents halogen, nitro, hydroxy, cyano, carboxy, amino, N-(C₁₋₆alkyl)amino, N-(hydroxyC₁₋₆alkyl)amino, N,N-di(C₁₋₆alkyl)amino, N-(C₁₋₆acyl)amino, N-(formyl)-N-(C₁₋₆alkyl)amino, N-(C₁₋₆alkanesulfonyl) amino, N-(carboxyC₁₋₆alkyl)-N-(C₁₋₆alkyl)amino, N-(C₁₋₆alkoxycabonyl)amino, N-[N,N-di(C₁₋₆alkyl)amino methylene]amino, N-[N,N-di(C₁₋₆alkyl)amino (C₁₋₆alkyl)methylene]amino, N-[N,N-di(C₁₋₆alkyl)amino C₂₋₆alkenyl]amino, aminocarbonyl, N-(C₁₋₆alkyl)aminocarbonyl, N,N-di(C₁₋₆alkyl)aminocarbonyl, C₃₋₈cycloalkyl, C₁₋₆ alkylthio, C₁₋₆alkanesulfonyl, sulfamoyl, C₁₋₆alkoxycarbonyl, N-arylamino wherein said aryl moiety is optionally having 1 to 3 substituents selected from R¹⁰¹, N-(aryl C₁₋₆alkyl)amino wherein said aryl moiety is optionally having 1 to 3 substituents selected from R¹⁰¹, aryl C₁₋₆alkoxycarbonyl wherein said aryl moiety is optionally having 1 to 3 substituents selected from R¹⁰¹,
   C₁₋₆alkyl optionally substituted by mono-, di- or tri- halogen, amino, N-(C₁₋₆alkyl)amino or N,N-di(C₁₋₆alkyl)amino,
   C₁₋₆alkoxy optionally substituted by mono-, di- or tri- halogen, N-(C₁₋₆alkyl)sulfonamide, or N-(aryl)sulfonamide,
   or
   a 5 to 7 membered saturated or unsaturated ring having 1 to 3 heteroatoms selected from the group consisting of O, S and N, and optionally having 1 to 3 substituents selected from R¹⁰¹
   wherein
   R¹⁰¹ represents halogen, carboxy, amino, N-(C₁₋₆ alkyl)amino, N,N-di(C₁₋₆alkyl)amino, aminocarbonyl, N-(C₁₋₆alkyl)aminocarbonyl, N,N-di(C₁₋₆alkyl)aminocarbonyl, pyridyl,
   C₁₋₆ alkyl optionally substituted by cyano or mono- di- or tri- halogen, and
   C₁₋₆alkoxy optionally substituted by cyano, carboxy, amino, N-(C₁₋₆ alkyl)amino, N,N-di(C₁₋₆alkyl)amino, aminocarbonyl, N-(C₁₋₆alkyl)aminocarbonyl, N,N-di(C₁₋₆alkyl)aminocarbonyl or mono-, di- or tri- halogen;
   - R²: represents hydroxy, halogen, nitro, cyano, amino, N-(C₁₋₆alkyl)amino, N,N-di(C₁₋₆alkyl)amino, N-(hydroxyC₁₋₆alkyl)amino, N-(hydroxyC₁₋₆alkyl)-N-(C₁₋₆alkyl)amino, C₁-₆acyloxy, aminoC₁₋₆acyloxy, C₂₋₆alkenyl, aryl,
   a 5-7 membered saturated or unsaturated heterocyclic ring having 1 to 3 heteroatoms selected from the group consisting O, S and N, and optionally substituted by
   hydroxy, C₁₋₆ alkyl, C₁₋₆ alkoxy, oxo, amino, amino C₁₋₆alkyl, N-(C₁₋₆alkyl)amino, N,N-di(C₁₋₆alkyl)amino, N-(C₁₋₆ acyl)amino, N-(C₁₋₆alkyl)carbonylamino, phenyl, phenyl C₁₋₆ alkyl, carboxy, C₁₋₆alkoxycarbonyl, aminocarbonyl, N-(C₁₋₆alkyl)aminocarbonyl, or N,N-di(C₁₋₆alkyl)amino, -C(O)- R²⁰ wherein
   R²⁰ represents C₁₋₆ alkyl, C₁₋₆ alkoxy, amino, N-(C₁₋₆alkyl)amino, N,N-di(C₁₋₆alkyl)amino, N-(C₁₋₆ acyl)amino, or a 5-7 membered saturated or unsaturated heterocyclic ring having 1 to 3 heteroatoms selected from the group consisting O, S and N, and optionally substituted by C₁₋₆ alkyl, C₁₋₆ alkoxy, oxo, amino, N-(C₁₋₆alkyl)amino, N,N-di(C₁₋₆alkyl)amino, N-(C₁₋₆ acyl)amino, phenyl, or benzyl,
   C₁₋₆ alkyl optionally substituted by R²¹,
   or
   C₁₋₆ alkoxy optionally substituted by R²¹,
   wherein
   R²¹ represents cyano, mono-, di or tri- halogen, hydroxy, amino, N-(C₁₋₆alkyl)amino, N,N-di(C₁₋₆alkyl)amino, N- (hydroxyC₁₋₆ alkyl) amino, N- (halophenylC₁₋₆ alkyl) amino, amino C₂₋₆ alkylenyl, C₁₋₆ alkoxy, hydroxyC₁₋₆ alkoxy, -C(O)- R²⁰¹, -NHC(O)- R²⁰¹, C₃₋₈cycloalkyl, isoindolino, phthalimidyl, 2-oxo-1,3-oxazolidinyl, aryl or a 5 or 6 membered saturated or unsaturated heterocyclic ring having 1 to 4 heteroatoms selected from the group consisting O, S and N , and optionally substituted by hydroxy, C₁₋₆ alkyl, C₁₋₆ alkoxy, C₁₋₆ alkoxycarbonyl, hydroxyC₁₋₆ alkoxy, oxo, amino, aminoC₁₋₆alkyl, N-(C₁₋₆alkyl)amino, N,N-di(C₁₋₆alkyl)amino, N-(C₁₋₆ acyl)amino, or benzyl,
   wherein
   R²⁰¹ represents hydroxy, amino, N-(C₁₋₆alkyl)amino, N,N-di(C₁₋₆alkyl)amino, N- (halophenylC₁₋₆ alkyl) amino, C₁₋₆alkyl, aminoC₁₋₆ alkyl, aminoC₂₋₆ alkylenyl, C₁₋₆ alkoxy, a 5 or 6 membered saturated or unsaturated heterocyclic ring having 1 to 4 heteroatoms selected from the group consisting O, S and N, and optionally substituted by hydroxy, C₁₋₆ alkyl, C₁₋₆ alkoxy, C₁₋₆ alkoxycarbonyl, hydroxyC₁₋₆ alkoxy, oxo, amino, N-(C₁₋₆alkyl)amino, N,N-di(C₁₋₆alkyl)amino, N-(C₁₋₆ acyl)amino or benzyl;
   - R³: represents hydrogen, halogen, aminocarbonyl, or C₁₋₆ alkyl optionally substituted by aryl C₁₋₆ alkoxy or mono-, di- or tri- halogen;
   - R⁴: represents hydrogen or C₁₋₆ alkyl;
   - R⁵: represents hydrogen or C₁₋₆ alkyl; and
   - R⁶: represents halogen, hydrogen or C₁₋₆ alkyl ;
   or a physiologically acceptable salt, solvate, hydrate or stereoisomer thereof;
   said compounds are published as compounds of general formulae I, la, Ib, Ic, Id and Ie in International patent application PCT/US2007/024985, published as WO 2008/070150 A1 on June 12, 2008, which is incorporated herein by reference in its entirety. In WO 2008/070150, said compounds of general formula I, la, Ib, Ic, Id and Ie are described on pp. 9 *et seq.,* they may be synthesized according to the methods given therein on pp. 42, *et seq.,* and are exemplified as specific compound Examples 1 to 103 therein on pp. 65 to 101. Biological test data for certain of said compounds are given therein on pp. 101 to 107.

2-amino-N-[7-methoxy-8-(3-morpholin-4-ylpropoxy)-2,3-dihydroimid-azo[1,2-c]quinazolin-5-yl]pyrimidine-5-carboxamide dihydrochloride, (which is hereinafter referred to as "compound A" or "cpd. A") is published in international patent application PCT/EP2012/055600, published as WO 2012/136553 on October 11, 2012, (which is incorporated herein by reference in its entirety), as the compound of Examples 1 and 2 : 2-amino-N-[7-methoxy-8-(3-morpholin-4-ylpropoxy)-2,3-dihydroimidazo[1,2-c]quinazolin-5-yl]pyrimidine-5-carboxamide dinydrochloride: it may be synthesized according to the methods given in said Examples 1 and 2.

The definitions used in relation to the structure (A) in this text are as follows :

The term 'alkyl' refers to a straight or branched hydrocarbon chain radical consisting solely of carbon and hydrogen atoms, containing solely of carbon and hydrogen atoms, containing no unsaturation, having from one to eight carbon atoms, and which is attached to the rest of the molecule by a single bond, such as illustratively, methyl, ethyl, n-propyl 1-methylethyl (isopropyl), n-butyl, n-pentyl, and 1,1-dimethylethyl (t-butyl).

The term "alkenyl" refers to an aliphatic hydrocarbon group containing a carbon-carbon double bond and which may be a straight or branched or branched chain having about 2 to about 10 carbon atoms, e.g., ethenyl, 1-propenyl, 2-propenyl (allyl), iso-propenyl, 2-methyl-I-propenyl, 1-butenyl, 2-and butenyl.

The term "alkynyl" refers to a straight or branched chain hydrocarbonyl radicals having at least one carbon-carbon triple bond, and having in the range of about 2 up to 12 carbon atoms (with radicals having in the range of about 2 up to 10 carbon atoms presently being preferred) e.g., ethynyl.

The term "alkoxy" denotes an alkyl group as defined herein attached via oxygen linkage to the rest of the molecule. Representative examples of those groups are methoxy and ethoxy.

The term "alkoxyakyl" denotes an alkoxy group as defined herein attached via oxygen linkage to an alkyl group which is then attached to the main structure at any carbon from alkyl group that results in the creation of a stable structure the rest of the molecule. Representative examples of those groups are -CH₂OCH₃ and -CH₂OC₂H₅.

The term "cycloalkyl" denotes a non-aromatic mono or multicyclic ring system of about 3 to 12 carbon atoms such as cyclopropyl, cyclobutyl, cyclopentyl, cyclohexyl and examples of multicyclic cycloalkyl groups include perhydronapththyl, adamantyl and norbornyl groups bridged cyclic group or sprirobicyclic groups e.g sprio (4,4) non-2-yl.

The term "cycloalkylalkyl" refers to cyclic ring-containing radicals containing in the range of about about 3 up to 8 carbon atoms directly attached to alkyl group which is then also attached to the main structure at any carbon from the alkyl group that results in the creation of a stable structure such as cyclopropylmethyl, cyclobuyylethyl, cyclopentylethyl.

The term "aryl" refers to aromatic radicals having in the range of 6 up to 14 carbon atoms such as phenyl, naphthyl, tetrahydronapthyl, indanyl, biphenyl.

The term "arylalkyl" refers to an aryl group as defined herein directly bonded to an alkyl group as defined herein which is then attached to the main structure at any carbon from alkyl group that results in the creation of a stable structure the rest of the molecule. e.g., --CH₂C₆H₅,-C₂H₅C₆H₅.

The term "heterocyclic ring" refers to a stable 3- to 15 membered ring radical which consists of carbon atoms and from one to five heteroatoms selected from the group consisting of nitrogen, phosphorus, oxygen and sulfur. For purposes of this invention, the heterocyclic ring radical may be a monocyclic, bicyclic or tricyclic ring system, which may include fused, bridged or spiro ring systems, and the nitrogen, phosphorus, carbon, oxygen or sulfur atoms in the heterocyclic ring radical may be optionally oxidized to various oxidation states. In addition, the nitrogen atom may be optionally quaternized; and the ring radical may be partially or fully saturated (i.e., heteroaromatic or heteroaryl aromatic). Examples of such heterocyclic ring radicals include, but are not limited to, azetidinyl, acridinyl, benzodioxolyl, benzodioxanyl, benzofurnyl, carbazolyl cinnolinyl dioxolanyl, indolizinyl, naphthyridinyl, perhydroazepinyl, phenazinyl, phenothiazinyl, phenoxazinyl, phthalazil, pyridyl, pteridinyl, purinyl, quinazolinyl, quinoxalinyl, quinolinyl, isoquinolinyl, tetrazoyl, imidazolyl tetrahydroisouinolyl, piperidinyl, piperazinyl, 2-oxopiperazinyl, 2-oxopiperidinyl, 2-oxopyrrolidinyl, 2-oxoazepinyl, azepinyl, pyrrolyl, 4-piperidonyl, pyrrolidinyl, pyrazinyl, pyrimidinyl pyridazinyl, oxazolyl oxazolinyl oxasolidinyl, triazolyl, indanyl, isoxazolyl, isoxasolidinyl, morpholinyl, thiazolyl, thiazolinyl, thiazolidinyl, isothiazolyl, quinuclidinyl, isothiazolidinyl, indolyl, isoindolyl, indolinyl, isoindolinyl, octahydroindolyl, octahydroisoindolyl quinolyl, isoquinolyl, decahydroisoquinolyl, benzimidazolyl, thiadiazolyl, benzopyranyl, benzothiazolyl, benzooxazolyl, furyl, tetrahydrofurtyl, tetrahydropyranyl, thienyl, benzothienyl, thiamorpholinyl, thiamorpholinyl sulfoxide thiamorpholinyl sulfone, dioxaphospholanyl, oxadiazolyl, chromanyl, isochromanyl.

The term "heteroaryl" refers to heterocyclic ring radical as defined herein which are aromatic. The heteroaryl ring radical may be attached to the main structure at any heteroatom or carbon atom that results in the creation of a stable structure.

The heterocyclic ring radical may be attached to the main structure at any heteroatom or carbon atom that results in the creation of a stable structure.

The term "heteroarylalkyl" refers to heteroaryl ring radical as defined herein directly bonded to alkyl group. The heteroarylalkyl radical may be attached to the main structure at any carbon atom from alkyl group that results in the creation of a stable structure.

The term "heterocyclyl" refers to a heterocylic ring radical as defined herein. The heterocylyl ring radical may be attached to the main structure at any heteroatom or carbon atom that results in the creation of a stable structure.

The term "heterocyclylalkyl" refers to a heterocylic ring radical as defined herein directly bonded to alkyl group. The heterocyclylalkyl radical may be attached to the main structure at carbon atom in the alkyl group that results in the creation of a stable structure.

The term "carbonyl" refers to an oxygen atom bound to a carbon atom of the molecule by a double bond.

The term "halogen" refers to radicals of fluorine, chlorine, bromine and iodine.

Said component A may be in the form of a pharmaceutical formulation which is ready for use to be administered simultaneously, concurrently, separately or sequentially. The components may be administered independnently of one another by the oral, intravenous, topical, local installations, intraperitoneal or nasal route.

In accordance with another embodiment of the above-mentioned aspects of the present invention, said combinations are of :
component A : which is one or more 2,3-dihydroimidazo[1,2-c]quinazoline compounds of general formula (A2), *supra,* which is selected from the list consisting of :
   Example 1 : N-[7-methoxy-8-(3-morpholin-4-ylpropoxy)-2,3-dihydroimidazo[1,2-c]quinazolin-5-yl]pyrimidine-5-carboxamide
   Example 2 : N-(8-{3-[(2R,6S)-2,6-dimethylmorpholin-4-yl]propoxy}-7-methoxy-2,3-dihydroimidazo[1,2-c]quinazolin-5-yl)nicotinamide
   Example 3 : N-(8-{3-[(2R,6S)-2,6-dimethylmorpholin-4-yl]propoxy}-7-methoxy-2,3-dihydroimidazo[1,2-c]quinazolin-5-yl)-2,4-dimethyl-1,3-thiazole-5-carboxamide
   Example 4 : 2-amino-N-[7-methoxy-8-(3-morpholin-4-ylpropoxy)-2,3-dihydroimidazo[1,2-c]quinazolin-5-yl]-1,3-thiazole-5-carboxamide.
   Example 5 : 2-amino-N-[7-methoxy-8-(3-morpholin-4-ylpropoxy)-2,3-dihydroimidazo[1,2-c]quinazolin-5-yl]isonicotinamide
   Example 6 : 2-amino-N-[7-methoxy-8-(3-morpholin-4-ylpropoxy)-2,3-dihydroimidazo[1,2-c]quinazolin-5-yl]-4-methyl-1,3-thiazole-5-carboxamide
   Example 7 : 2-amino-N-[7-methoxy-8-(3-morpholin-4-ylpropoxy)-2,3-dihydroimidazo[1,2-c]quinazolin-5-yl]-4-propylpyrimidine-5-carboxamide
   Example 8 : N-{8-[2-(4-ethylmorpholin-2-yl)ethoxy]-7-methoxy-2,3-dihydroimidazo[1,2-c]quinazolin-5-yl}nicotinamide
   Example 9 : N-18-[2-(dimethylamino)ethoxy]-7-methoxy-2,3-dihydroimidazo[1,2-c]quinazolin-5-yl}pyrimidine-5-carboxamide
   Example 10 : N-(8-{3-[2-(hydroxymethyl)morpholin-4-yl]propoxy}-7-methoxy-2,3-dihydroimidazo[1,2-c]quinazolin-5-yl)nicotinamide
   Example 11 : N-(8-{3-[2-(hydroxymethyl)morpholin-4-yl]propoxy}-7-methoxy-2,3-dihydroimidazo[1,2-c]quinazolin-5-yl)nicotinamide
   Example 12 : N-{8-[3-(dimethylamino)propoxy]-7-methoxy-2,3-dihydroimidazo[1,2-c]quinazolin-5-yl}nicotinamide 1-oxide
   Example 13 : 2-amino-N-[7-methoxy-8-(3-morpholin-4-ylpropoxy)-2,3-dihydroimidazo[1,2-c]quinazolin-5-yl]pyrimidine-5-carboxamide.
   Example 14 : N-[7-methoxy-8-(3-morpholin-4-ylpropoxy)-2,3-dihydroimidazo[1,2-c]quinazolin-5-yl]-6-(2-pyrrolidin-1-ylethyl)nicotinamide.
   Example 15 : 6-(cyclopentylamino)-N-[7-methoxy-8-(3-morpholin-4-ylpropoxy)-2,3-dihydroimidazo[1,2-c]quinazolin-5-yl]nicotinamide

| Example | Structure |
|---|---|
| 16 | 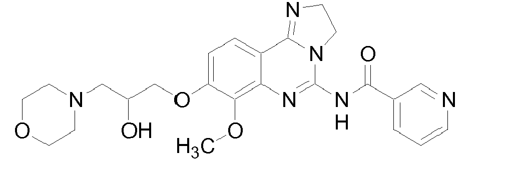 |
| 17 | 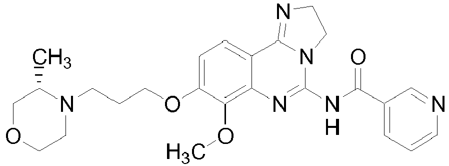 |
| 18 | 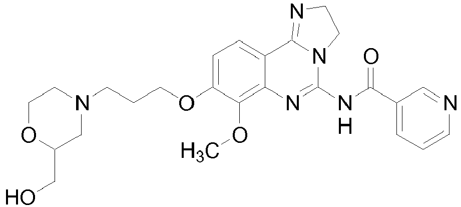 |
| 19 | 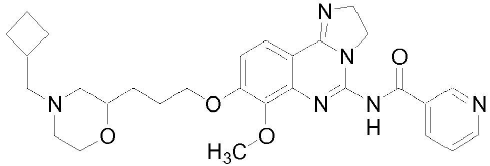 |
| 20 | 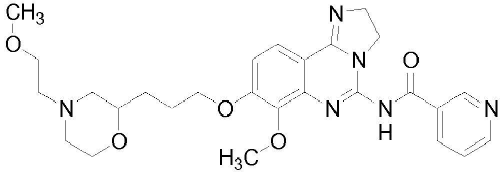 |
| 21 | 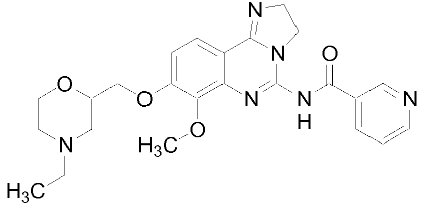 |
| 22 | 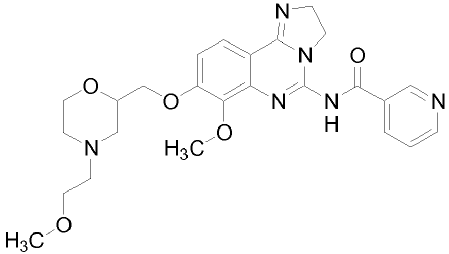 |
| 23 | 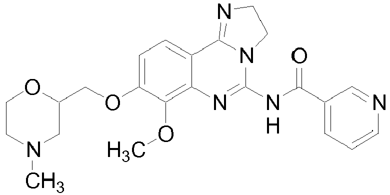 |
| 24 | 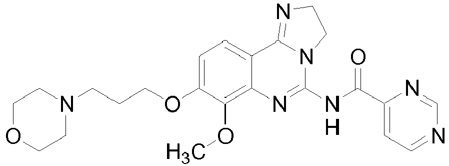 |
| 25 | 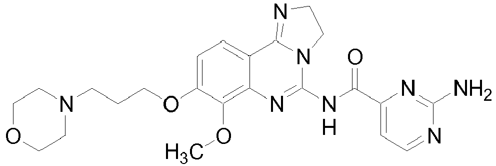 |
| 26 | 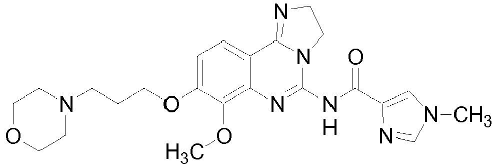 |
| 27 | 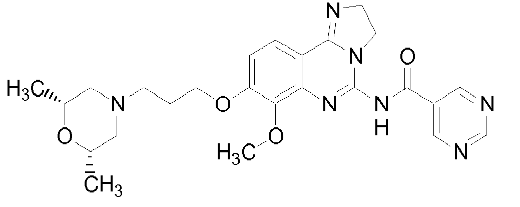 |
| 28 | 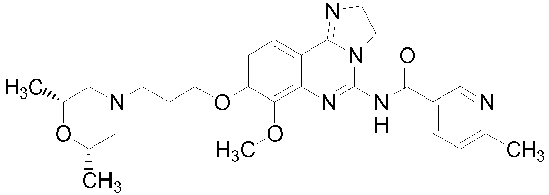 |
| 29 | 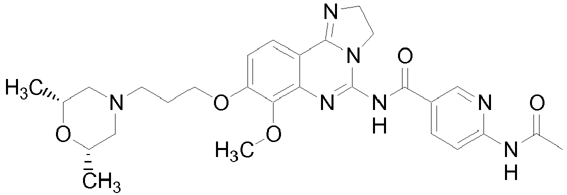 |
| 30 | 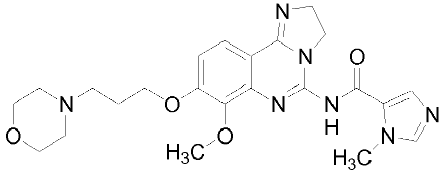 |
| 31 | 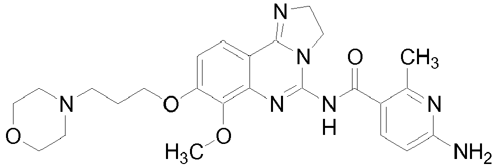 |
| 32 | 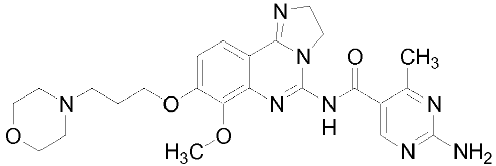 |
| 33 | 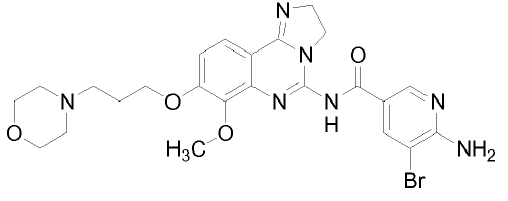 |
| 34 | 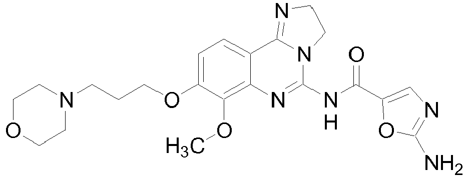 |
| 35 | 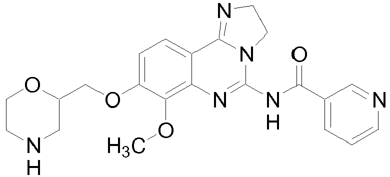 |
| 36 | 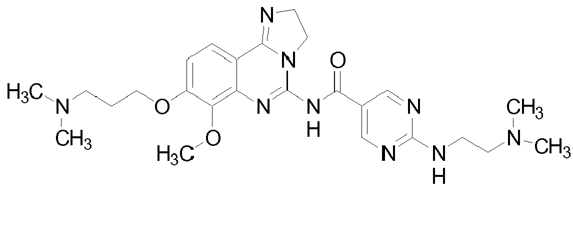 |
| 37 | 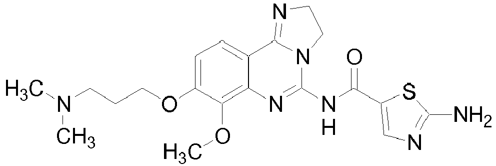 |
| 38 | 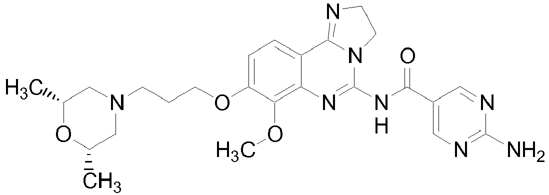 |
| 39 | 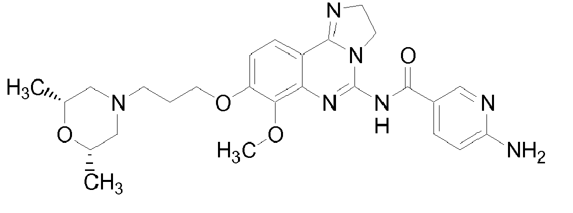 |
| 40 | 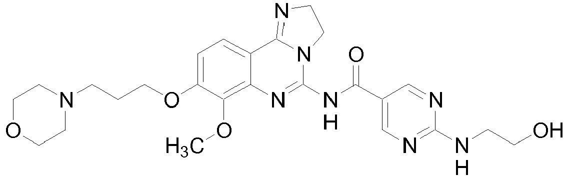 |
| 41 | 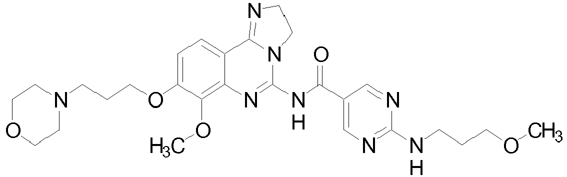 |
| 42 | 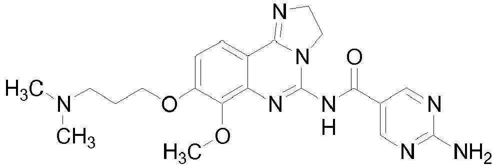 |
| 43 | 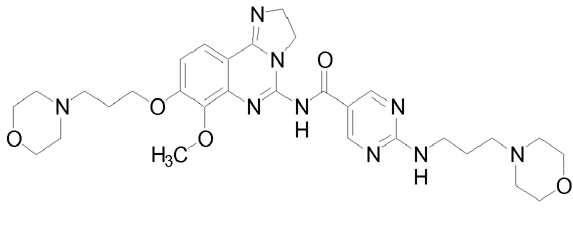 |
| 44 | 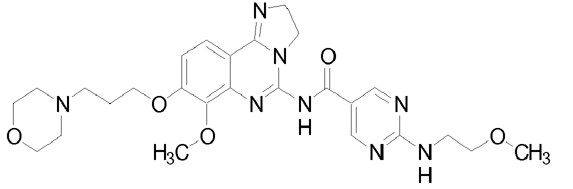 |
| 45 | 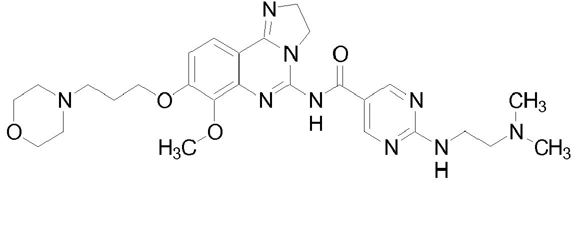 |
| 46 | 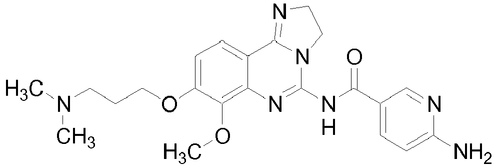 |
| 47 | 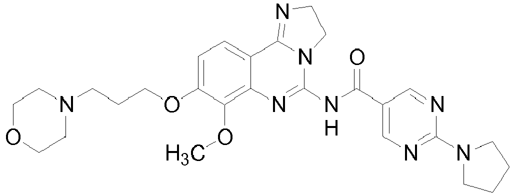 |
| 48 | 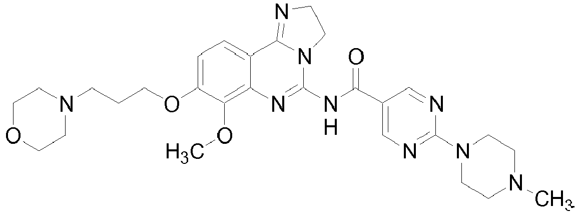 |
| 49 | 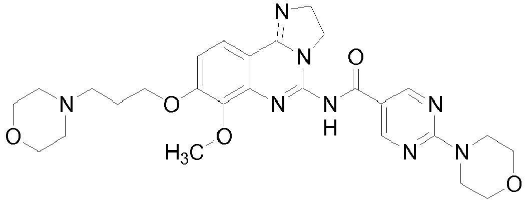 |
| 50 | 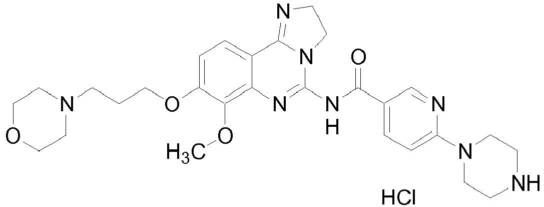 |
| 51 | 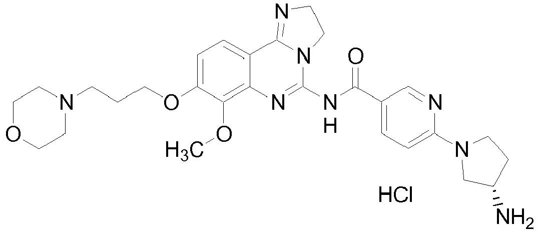 |
| 52 | 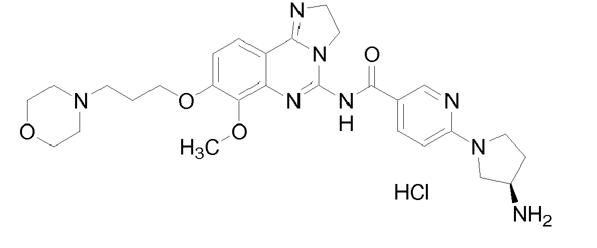 |
| 53 | 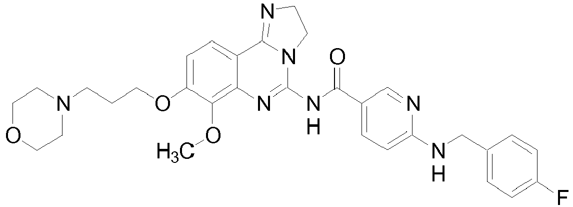 |
| 54 | 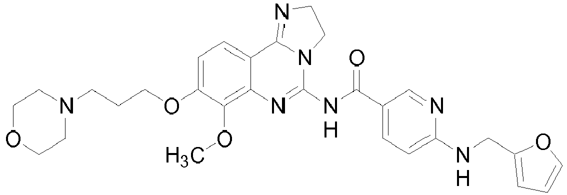 |
| 55 | 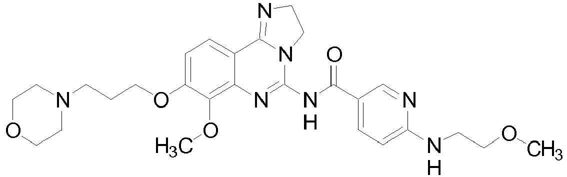 |
| 56 | 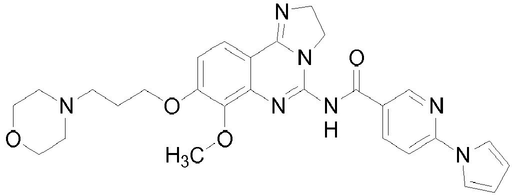 |
| 57 | 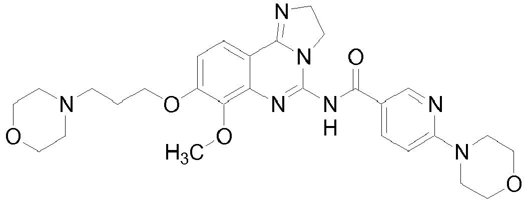 |
| 58 | 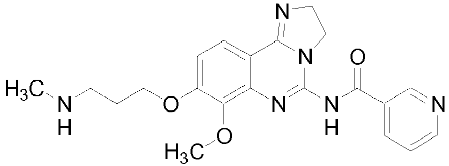 |
| 59 | 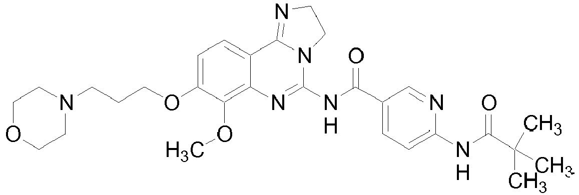 |
| 60 | 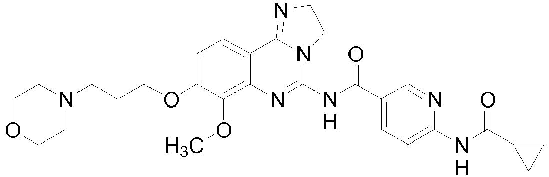 |
| 61 | 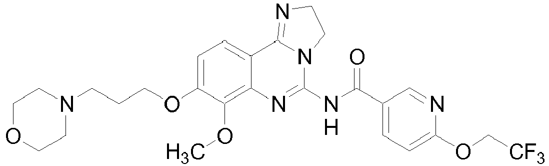 |
| 62 | 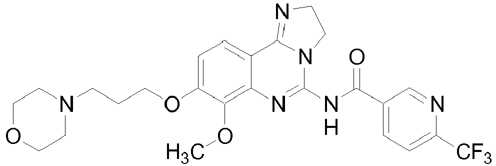 |
| 63 | 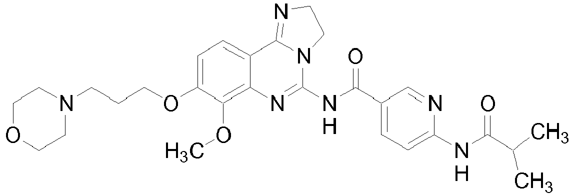 |
| 64 | 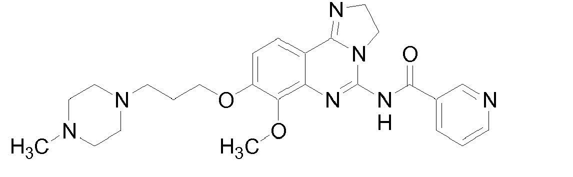 |
| 65 | 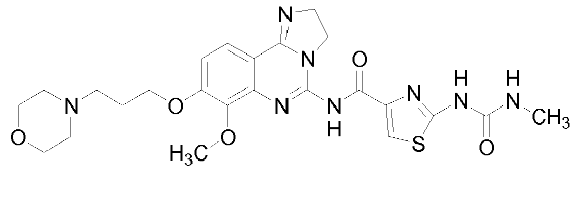 |
| 66 | 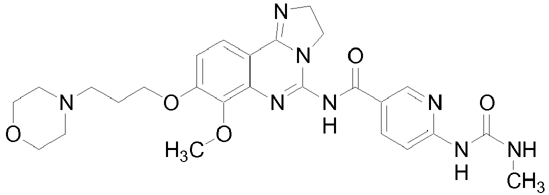 |
| 67 | 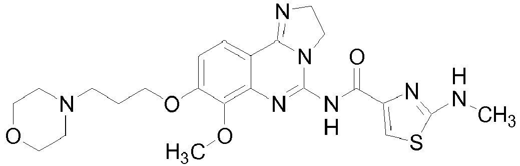 |
| 68 | 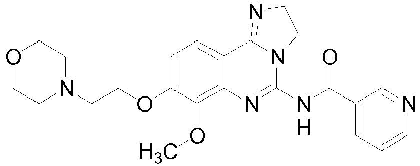 |
| 69 | 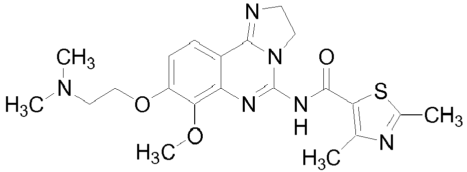 |
| 70 | 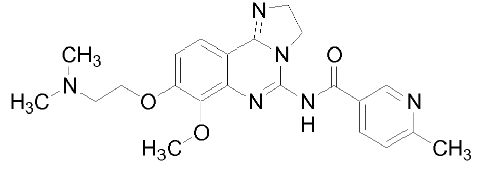 |
| 71 | 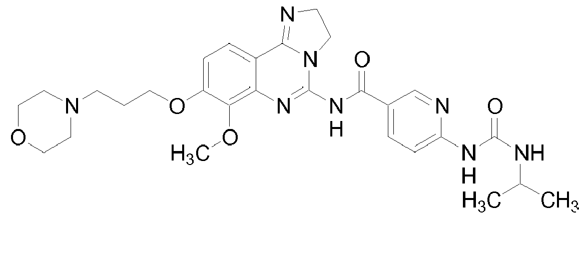 |
| 72 | 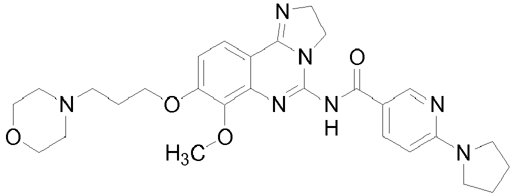 |
| 73 | 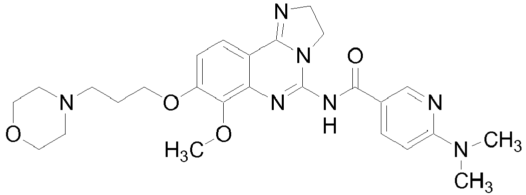 |
| 74 | 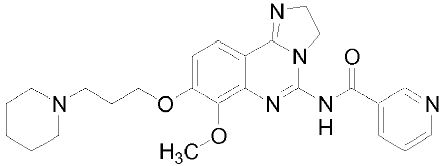 |
| 75 | 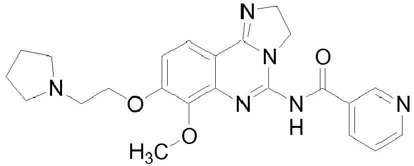 |
| 76 | 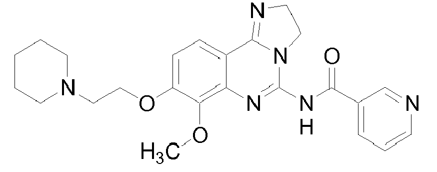 |
| 77 | 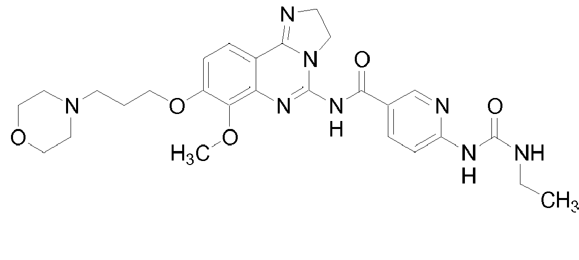 |
| 78 | 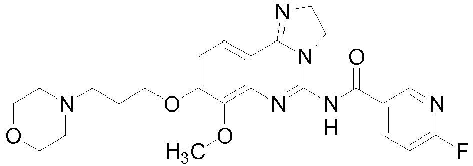 |
| 79 | 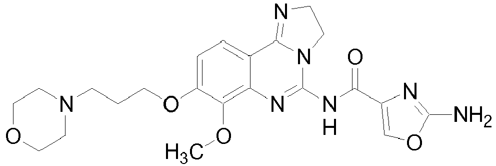 |
| 80 | 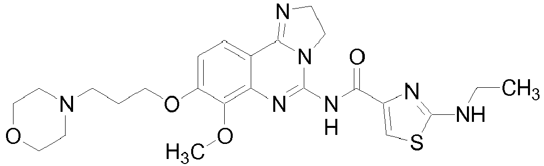 |
| 81 | 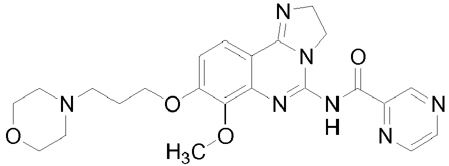 |
| 82 | 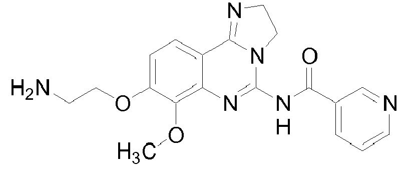 |
| 83 | 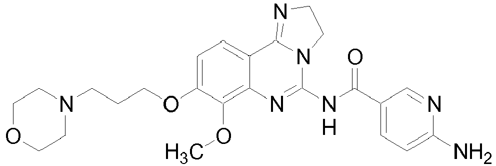 |
| 84 | 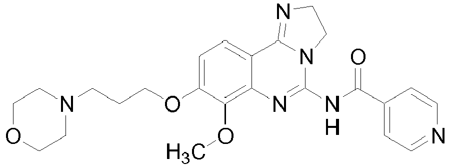 |
| 85 | 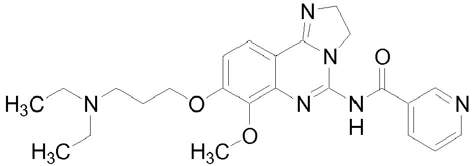 |
| 86 | 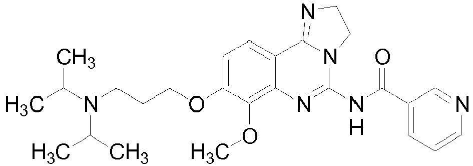 |
| 87 | 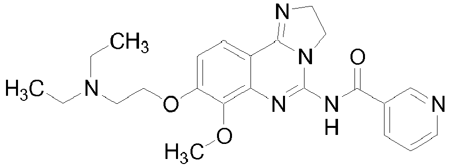 |
| 88 | 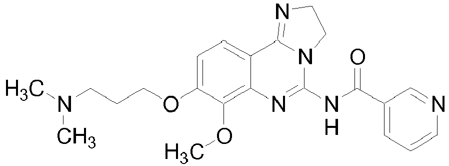 |
| 89 | 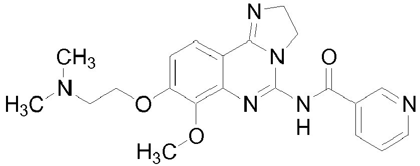 |
| 90 | 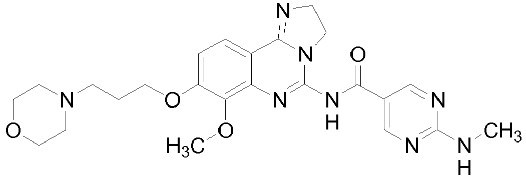 |
| 91 | 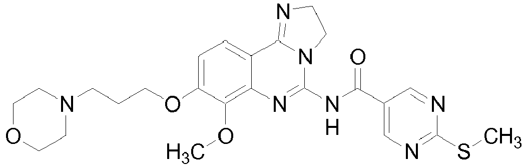 |
| 92 | 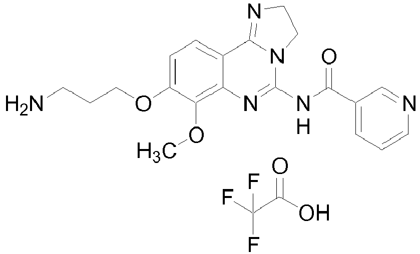 |
| 93 | 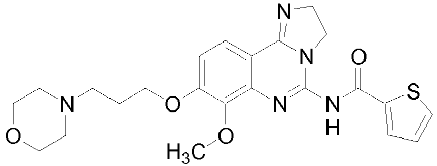 |
| 94 | 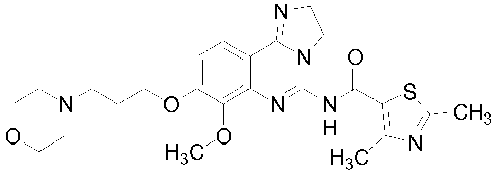 |
| 95 | 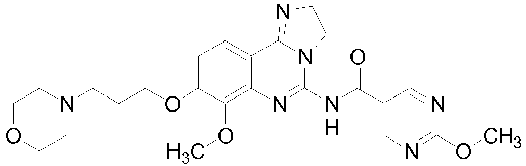 |
| 96 | 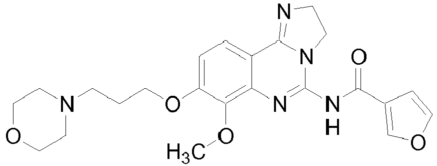 |
| 97 | 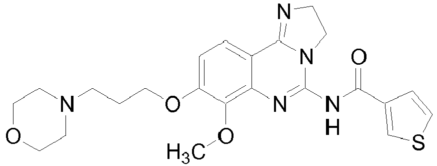 |
| 98 | 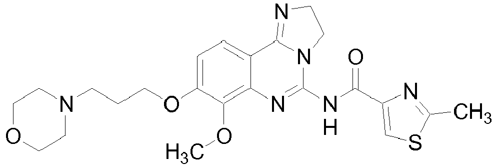 |
| 99 | 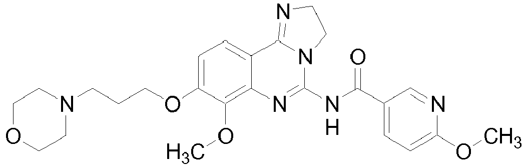 |
| 100 | 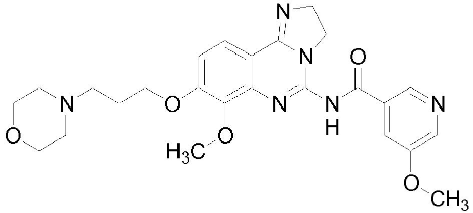 |
| 101 | 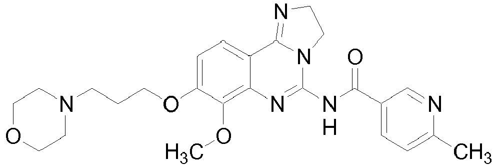 |
| 102 | 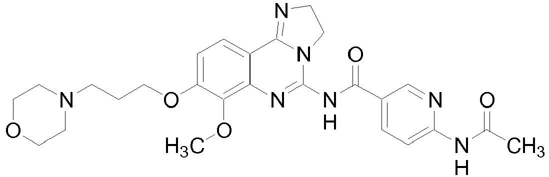 |
| 103 | 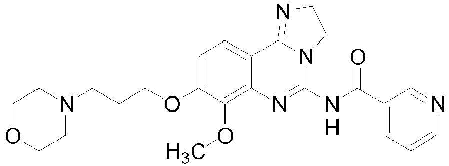 |

or a physiologically acceptable salt, solvate, hydrate or stereoisomer thereof,

Said compounds are published as specific compound Examples 1 to 103 in International patent application PCT/US2007/024985, published as WO 2008/070150 A1 on June 12, 2008, which is incorporated herein by reference in its entirety. In WO 2008/070150, said specific compound Examples may be synthesized according to the Examples. Biological test data for certain of said compounds are given therein on pp. 101 to 107.

Specifically, component A may be 2-amino-N-[7-methoxy-8-(3-morpholin-4-ylpropoxy)-2,3-dihydroimid-azo[1,2-c]quinazolin-5-yl]pyrimidine-5-carboxamide dihydrochloride, (which is hereinafter referred to as "compound A" or "cpd. A") is published in international patent application PCT/EP2012/055600, published as WO 2012/136553 on October 11, 2012, (which is incorporated herein by reference in its entirety), as the compound of Examples 1 and 2 : 2-amino-N-[7-methoxy-8-(3-morpholin-4-ylpropoxy)-2,3-dihydroimidazo[1,2-c]quinazolin-5-yl]pyrimidine-5-carboxamide dinydrochloride: it may be synthesized according to the methods given in said Examples 1 and 2.

Said component A may be in the form of a pharmaceutical formulation which is ready for use to be administered simultaneously, concurrently, separately or sequentially. The components may be administered independnently of one another by the oral, intravenous, topical, local installations, intraperitoneal or nasal route.

In accordance with an embodiment of the above-mentioned aspects of the present invention, said combinations are of :
component B : which is one or more IRAK4-inhibiting compounds of general formula (I) : 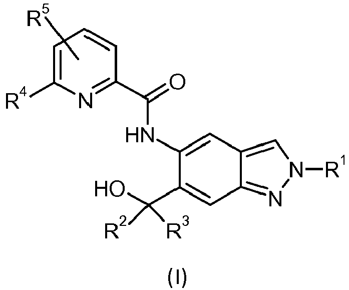 in which:
   - R¹: is C₁-C₆-alkyl, where the C₁-C₆-alkyl radical is unsubstituted or mono- or polysubstituted identically or differently by
   halogen, hydroxyl, an unsubstituted or mono- or poly-halogen-substituted C₃-C₆-cycloalkyl, or an R⁶, R⁷SO₂, R⁷SO or R⁸O radical,
   or a group selected from: 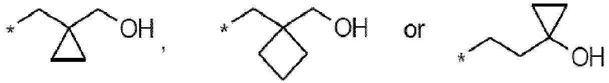 where * represents the bonding site of the group to the rest of the molecule;
   - R² and R³: always have the same definition and are both either hydrogen or C₁-C₆-alkyl;
   - R⁴: is halogen, cyano, an unsubstituted or a singly or multiply, identically or differently substituted C₁-C₆-alkyl or an unsubstituted or a singly or multiply, identically or differently substituted C₃-C₆-cycloalkyl, and the substituents are selected from the group of halogen and hydroxyl;
   - R⁵: is hydrogen, halogen or an unsubstituted or poly-halogen-substituted C₁-C₆-alkyl;
   - R⁶: is an unsubstituted or mono- or di-methyl-substituted monocyclic saturated heterocycle having 4 to 6 ring atoms, which contains a heteroatom or a heterogroup from the group of O, S, SO and SO₂;
   - R⁷: is C₁-C₆-alkyl, where the C₁-C₆-alkyl radical is unsubstituted or mono- or polysubstituted identically or differently by halogen, hydroxyl or C₃-C₆-cycloalkyl; or R⁷ is C₃-C₆-cycloalkyl,
   - R⁸: is C₁-C₆-alkyl, where the C₁-C₆-alkyl radical is unsubstituted or mono- or polysubstituted identically or differently by halogen;
   and the diastereomers, enantiomers, metabolites, salts, solvates or solvates of the salts thereof.

The definitions of the substituents in said IRAK4-inhibiting compounds of general formula (I) of the inventive combinations of the present invention, unless specified otherwise, are defined as follows:
Alkyl in the context of the invention is a linear or branched alkyl radical having the particular number of carbon atoms specified. Examples include methyl, ethyl, n-propyl, isopropyl, n-butyl, isobutyl, 1-methylpropyl, 2-methylpropyl, tert-butyl, n-pentyl, 1-ethylpropyl, 1-methylbutyl, 2-methylbutyl, 3-methylbutyl, 2,2-dimethylpropyl, n-hexyl, 1-methylpentyl, 2-methylpentyl, 3-methylpentyl, 4-methylpentyl, 1-ethylbutyl and 2-ethylbutyl. Preference is given to methyl, ethyl, n-propyl, n-butyl, 2-methylbutyl, 3-methylbutyl and 2,2-dimethylpropyl.
Cycloalkyl in the context of the invention is a monocyclic saturated alkyl radical having the number of carbon atoms specified in each case. Preferred examples include cyclopropyl, cyclobutyl, cyclopentyl, cyclohexyl and cycloheptyl.
Alkoxy in the context of the invention is a linear or branched alkoxy radical having the particular number of carbon atoms specified. 1 to 6 carbon atoms are preferred. Examples include methoxy, ethoxy, n-propoxy, isopropoxy, 1-methylpropoxy, n-butoxy, isobutoxy, tert-butoxy, n-pentoxy, isopentoxy, 1-ethylpropoxy, 1-methylbutoxy, 2-methylbutoxy, 3-methylbutoxy and n-hexoxy. Particular preference is given to a linear or branched alkoxy radical having 1 to 4 carbon atoms. Examples which may be mentioned as being preferred are methoxy, ethoxy, n-propoxy, 1-methylpropoxy, n-butoxy and isobutoxy.
Halogen in the context of the invention is fluorine, chlorine and bromine. Preference is given to fluorine.
Hydroxyl in the context of the invention is OH.
A monocyclic saturated heterocycle is a monocyclic saturated heterocycle which has 4 to 6 ring atoms and contains a heteroatom or a heterogroup from the group of O, S, SO and SO₂. A heterocycle having a heteroatom or a heterogroup from the group of O, SO and SO₂ is preferred. Examples include: oxetane, tetrahydrofuran, tetrahydro-2H-pyran-4-yl, 1,1-dioxidotetrahydro-2H-thiopyran-3-yl, 1,1-dioxidotetrahydro-2H-thiopyran-2-yl, 1,1-dioxidotetrahydro-2H-thiopyran-4-yl, 1,1-dioxidotetrahydrothiophen-3-yl, 1,1-dioxidotetrahydrothiophen-2-yl, 1,1-dioxidothietan-2-yl or 1,1-dioxidothietan-3-yl. Particular preference is given here to oxetane and tetrahydrofuran. Very particular preference is given to oxetan-3-yl.

A symbol * at a bond denotes the bonding site in the molecule.

When radicals in the compounds of the formula (I) as constituents of the inventive combinations are substituted, the radicals may be mono- or polysubstituted, unless specified otherwise. In the context of the present invention, all radicals which occur more than once are defined independently of one another. Substitution by one, two or three identical or different substituents is preferred.

Said IRAK4-inhibiting compounds of general formula (I) of the inventive combinations of the present invention are prepared as follows:

Starting materials used for synthesis of the compounds of the formula (I) as constituents of the inventive combinations are carboxylic acids (Intermediate V3), which are commercially available or can be prepared by routes known from the literature or analogously to routes known from the literature (see, for example, European Journal of Organic Chemistry 2003, 8, 1559 - 1568, Chemical and Pharmaceutical Bulletin, 1990, 38, 9, 2446 - 2458, Synthetic Communications 2012, 42, 658 - 666, Tetrahedron, 2004, 60, 51, 11869 - 11874) (see, for example, Synthesis Scheme 1). Some carboxylic acids V3 can be prepared proceeding from carboxylic esters (Intermediate V2) by hydrolysis (cf., for example, the reaction of ethyl 6-(hydroxymethyl)pyridine-2-carboxylate with aqueous sodium hydroxide solution in methanol, WO200411328) or - in the case of a *tert*-butyl ester - by reaction with an acid, for example hydrogen chloride or trifluoroacetic acid (cf., for example, Dalton Transactions, 2014, 43, 19, 7176 - 7190). The carboxylic acids V3 can also be used in the form of their alkali metal salts. The Intermediates V2 can optionally also be prepared from the Intermediates V1 which bear a chlorine, bromine or iodine as substituent X¹ by reaction in a carbon monoxide atmosphere, optionally under elevated pressure, in the presence of a phosphine ligand, for example 1,3-bis(diphenylphoshino)propane, a palladium compound, for example palladium(II) acetate, and a base, for example triethylamine, with addition of methanol or methanol in a solvent, for example dimethyl sulphoxide (for preparation methods see, for example, WO2012112743, WO 2005082866, Chemical Communications (Cambridge, England), 2003, 15, 1948 - 1949, WO200661715). The Intermediates V1 are either commercially available or can be prepared by routes known from the literature. Illustrative preparation methods are detailed in WO 2012061926, European Journal of Organic Chemistry, 2002, 2, 327 - 330, Synthesis, 2004, 10, 1619 - 1624, Journal of the American Chemical Society, 2013, 135, 32, 12122 - 12134, Bioorganic and Medicinal Chemistry Letters, 2014, 24, 16, 4039 - 4043, US2007185058, WO2009117421.

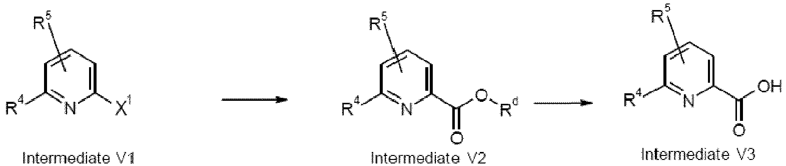

### Synthesis Scheme 1

X¹ is chlorine, bromine or iodine.
R^{d} is methyl, ethyl, benzyl or *tert*-butyl.
R⁴, R⁵ are each as defined in the general formula (I).

Methyl 5-amino-1H-indazole-6-carboxylate (Intermediate 2) can be obtained proceeding from methyl 1H-indazole-6-carboxylate (Intermediate 0) according to Synthesis Scheme 2 by nitration and reduction of the nitro group of Intermediate 1 with hydrogen in the presence of palladium on charcoal analogously to *Ohrai, Kazuhiko Chiba* WO 2008/001883. For preparation of the Intermediates 3 proceeding from Intermediate 2, it is possible to use various coupling reagents known from the literature (Amino Acids, Peptides and Proteins in Organic Chemistry, Vol.3 - Building Blocks, Catalysis and Coupling Chemistry, Andrew B. Hughes, Wiley, Chapter 12 - Peptide-Coupling Reagents, 407-442; Chem. Soc. Rev., 2009, 38, 606). For example, it is possible to use 1-(3-dimethylaminopropyl)-3-ethylcarbodiimide hydrochloride in combination with 1-hydroxy-1H-benzotriazole hydrate (HOBt, WO2012107475; Bioorg. Med. Chem. Lett., 2008 , 18, 2093), (1H-benzotriazol-1-yloxy)(dimethylamino)-N,N-dimethylmethaniminium tetrafluoroborate (TBTU, CAS 125700-67-6), (dimethylamino)-N,N-dimethyl(3H-[1,2,3]triazolo[4,5-b]pyridin-3-yloxy)methanaminium hexafluorophosphate (HATU, CAS 148893-10-1), propanephosphonic anhydride (as solution in ethyl acetate or DMF, CAS68957-94-8) or di-1H-imidazol-1-ylmethanone (CDI) as coupling reagents, with addition of a base such as triethylamine or N-ethyl-N-isopropylpropan-2-amine in each case to the reaction mixture. Preference is given to the use of TBTU and N-ethyl-N-isopropylpropan-2-amine in THF. 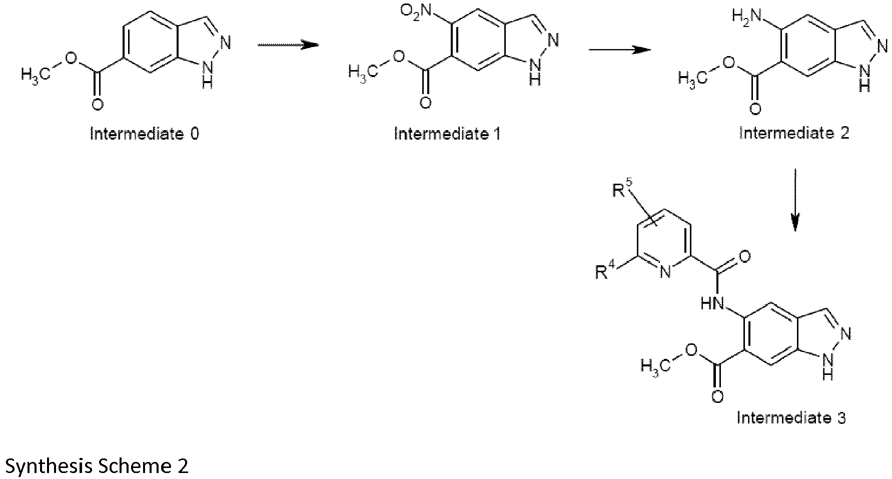 The substituents R⁴, R⁵ are each as defined in the general formula (I).

Proceeding from the Intermediates 3, it is possible to prepare 2-substituted indazole derivatives (Intermediate 4) (see Synthesis Scheme 3). Useful reactions for this purpose include those with optionally substituted alkyl chlorides, alkyl bromides, alkyl iodides or alkyl 4-methylbenzenesulphonates. The alkyl halides or alkyl 4-methylbenzenesulphonates used are commercially available or can be prepared analogously to routes known from literature (for the preparation of alkyl 4-methylbenzenesulphonates, one example is the reaction of an appropriate alcohol with 4-methylbenzenesulphonyl chloride in the presence of triethylamine or pyridine; see, for example, Bioorganic and Medicinal Chemistry, 2006, 14, 12 4277 - 4294). Optionally, in the case of use of alkyl chlorides or alkyl bromides, it is also possible to add an alkali metal iodide such as potassium iodide or sodium iodide. Bases used may, for example, be potassium carbonate, caesium carbonate or sodium hydride. In the case of reactive alkyl halides, it is also possible in some cases to use N-cyclohexyl-N-methylcyclohexanamine. Useful solvents include, for example, 1-methylpyrrolidin-2-one, DMF, DMSO or THF. Optionally, the alkyl halides or alkyl 4-methylbenzenesulphonates used may have functional groups which have optionally been protected with a protecting group beforehand (see also P. G. M. Wuts, T. W. Greene, Greene's Protective Groups in Organic Synthesis, Fourth Edition, ISBN: 9780471697541). If, for example, alkyl halides or alkyl 4-methylbenzenesulphonates having one or more hydroxyl groups are used, these hydroxyl groups may optionally be protected by a *tert-*butyl(dimethyl)silyl group or a similar silicon-containing protecting group familiar to those skilled in the art. Alternatively, the hydroxyl groups may also be protected by the tetrahydro-2H-pyran (THP) group or by the acetyl or benzoyl group. The protecting groups used can then be detached subsequently to the synthesis of Intermediate 4, or else after the synthesis of (I). If, for example, a *tert*-butyl(dimethylsilyl) group is used as protecting group, it can be detached using tetrabutylammonium fluoride in a solvent such as THF, for example. A THP protecting group can be detached, for example, using 4-methylbenzenesulphonic acid (optionally in monohydrate form). Acetyl groups or benzoyl groups can be detached by treatment with aqueous sodium hydroxide solution.

Optionally, the alkyl halides or alkyl 4-methylbenzenesulphonates used may contain functional groups which can be converted by oxidation or reduction reactions known to those skilled in the art (see, for example, Science of Synthesis, Georg Thieme Verlag). If, for example, the functional group is a sulphide group, this can be oxidized by methods known in the literature to a sulphoxide or sulphone group. In the case of a sulphoxide group, this can likewise be oxidized to a sulphone group. For these oxidation steps, it is possible to use, for example, 3-chloroperbenzoic acid (CAS 937-14-4) (in this regard, see also, for example, US201094000 for the oxidation of a 2-(methylsulphanyl)ethyl-1H-pyrazole derivative to a 2-(methylsulphinyl)ethyl-1H-pyrazole derivative and the oxidation of a further 2-(methylsulphanyl)ethyl-1H-pyrazole derivative to a 2-(methylsulphonyl)ethyl-1H-pyrazole derivative). If the alkyl halides or tosylates used contain a keto group, this can be reduced by reduction methods known to those skilled in the art to an alcohol group (see, for example, Chemische Berichte, 1980, 113, 1907 - 1920 for the use of sodium borohydride). These oxidation or reduction steps can be effected subsequently to the synthesis of Intermediate 4, or else after the synthesis of the compounds of the general formula (I). Alternatively, Intermediate 4 can be prepared via Mitsunobu reaction (see, for example, K. C. K. Swamy et. al. Chem. Rev. 2009, 109, 2551 - 2651) of Intermediate 3 with optionally substituted alkyl alcohols. It is possible to utilize various phosphines such as triphenylphosphine, tributylphosphine or 1,2-diphenylphosphinoethane in combination with diisopropyl azodicarboxylate (CAS 2446-83-5) or further diazene derivatives mentioned in the literature (K. C. K. Swamy et. al. Chem. Rev. 2009, 109, 2551 - 2651). Preference is given to the use of triphenylphosphine and diisopropyl azodicarboxylate. If the alkyl alcohol bears a functional group it is possible - as in the case of the abovementioned reactions with alkyl halides - for known protecting group strategies (further pointers can be found in P. G. M. Wuts, T. W. Greene, Greene's Protective Groups in Organic Synthesis, Fourth Edition, ISBN: 9780471697541) and - as in the case of the abovementioned reactions with alkyl halides - for oxidation or reduction steps to be effected subsequently to the synthesis of Intermediate 4, or else after the synthesis of the compounds of the general formula (I). Proceeding from Intermediate 4, inventive compounds of the general formula (I) where R² and R³ are defined as C₁-C₆-alkyl (where R² and R³ have the same definition) may be obtained by a Grignard reaction (cf., for example, the reaction of a methyl 1H-indazole-6-carboxylate derivative with methylmagnesium bromide in EP 2489663). For the Grignard reaction, it is possible to use alkylmagnesium halides. Particular preference is given to methylmagnesium chloride or methylmagnesium bromide in THF or diethyl ether, or else in mixtures of THF and diethyl ether. Alternatively, proceeding from Intermediate 4, inventive compounds of the general formula (I) where R² and R³ are defined as C₁-C₆-alkyl (where R² and R³ have the same definition) may be obtained by a reaction with an alkyllithium reagent (cf., for example, the reaction of a methyl 2-amino-4-chloro-1-methyl-1H-benzimidazole-7-carboxylate derivative with isopropyllithium or tert-butyllithium in WO2006116412). Proceeding from Intermediate 4, it is possible to prepare inventive compounds of the general formula (I) where R² and R³ are defined as H by reduction with lithium aluminium hydride in THF, lithium borohydride in THF or sodium borohydride in THF, optionally with addition of methanol, or mixtures of lithium borohydride and sodium borohydride.

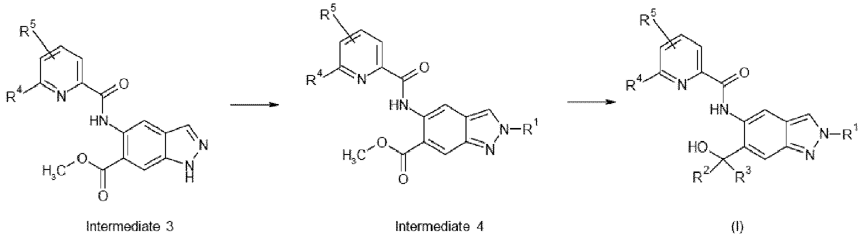

### Synthesis Scheme 3:

The substituents R¹, R², R³, R⁴, R^{S} are each as defined in the general formula (I).

Proceeding from Intermediate 3, Intermediate 5 where R² and R³ are defined as C₁-C₆-alkyl (where R² and R³ have the same definition) may be obtained by a Grignard reaction (cf., for example, Synthesis Scheme 4). For this purpose, it is possible to use suitable alkylmagnesium halides, for example methylmagnesium chloride or methylmagnesium bromide in THF or in diethyl ether or else in mixtures of THF and diethyl ether.

Proceeding from Intermediate 5, it is then possible to prepare a portion (I-a) of the compounds (I) where R² and R³ are defined as C₁-C₆-alkyl (where R² and R³ have the same definition). For this purpose, analogously to Synthesis Scheme 3 (preparation of Intermediate 3), useful reactions are those of Intermediate 5 with optionally substituted alkyl chlorides, alkyl bromides, alkyl iodides or alkyl 4-methylbenzenesulphonates. It is possible to use protecting group strategies analogously to those described in Synthesis Scheme 3.

Alternatively, for preparation of a portion (I-a) of the compounds (I) where R² and R³ are defined as C₁-C₆-alkyl (where R² and R³ have the same definition), it is possible to use the Mitsunobu reaction of Intermediate 5 with optionally substituted alkyl alcohols (analogously to Synthesis Scheme 3).

If R¹ in the compounds of the formula (I-a) includes a suitable functional group, it is optionally possible subsequently, in analogy to Synthesis Scheme 3, to use oxidation or reduction reactions for preparation of further inventive compounds.

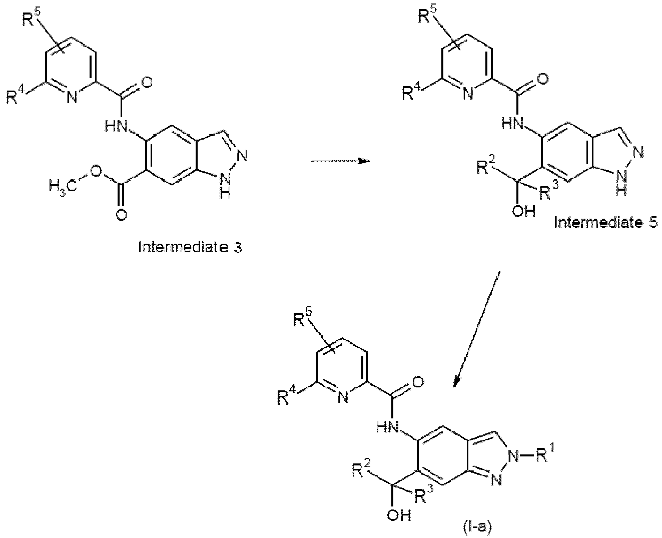

### Synthesis Scheme 4

The substituents R¹, R⁴, R⁵ are each as defined in the general formula (I). R² and R³ always have the same definition and are both C₁-C₆-alkyl.

Proceeding from Intermediate 1, it is possible to prepare Intermediate 4 in an alternative manner (see Synthesis Scheme 5). First of all, Intermediate 1 is converted to Intermediate 6 by methods as in Synthesis Scheme 3 (preparation of Intermediate 4 from Intermediate 3). Intermediate 6 can then be converted to Intermediate 7 by reduction of the nitro group. For example, the nitro group can be reduced with palladium on carbon under a hydrogen atmosphere (cf., for example, WO2013174744 for the reduction of 6-isopropoxy-5-nitro-1H-indazole to 6-isopropoxy-1H-indazol-5-amine) or by the use of iron and ammonium chloride in water and ethanol (see, for example, also Journal of the Chemical Society, 1955, 2412-2419), or by the use of tin(II) chloride (CAS 7772-99-8). The use of iron and ammonium chloride in water and ethanol is preferred. The preparation of Intermediate 4 from Intermediate 7 can be effected analogously to Synthesis Scheme 2 (preparation of Intermediate 3 from Intermediate 2).

As described for Synthesis Scheme 3, it is optionally possible to use protecting group strategies in the case of Synthesis Scheme 5 as well. Optionally, it is additionally possible, proceeding from Intermediate 6 for Intermediate 7, as described for Synthesis Scheme 3, to conduct oxidation for reduction reactions known to those skilled in the art (cf., for example Science of Synthesis, Georg Thieme Verlag).

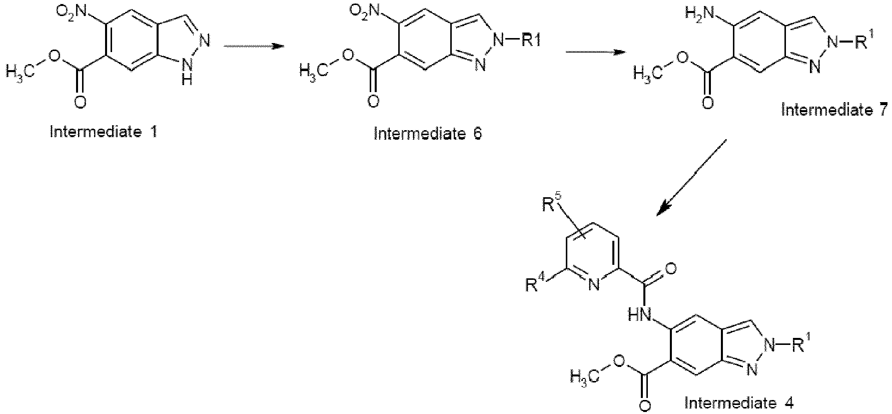

### Synthesis Scheme 5

The substituents R1, R4, R5 are each as defined in the general formula (I).

### Synthesis of the example compounds of the formula (I) as constituents of the inventive combinations

**Abbreviations and elucidations**

| | |
|---|---|
| DMF | *N,N*-dimethylformamide |
| DMSO | dimethyl sulphoxide |
| THF | Tetrahydrofuran |
| RT | room temperature |
| HPLC | high-performance liquid chromatography |
| H | hour(s) |
| min | minute(s) |
| UPLC | ultrahigh-performance liquid chromatography |
| DAD | diode array detector |
| ELSD | evaporating light scattering detector |
| ESI | electrospray ionization |
| SQD | single quadrupole detector |
| CPG | core-pulled precision glass |

The term sodium chloride solution always means a saturated aqueous sodium chloride solution.

The chemical names of the intermediates and examples were generated using the ACD / LABS (Batch Version 12.01.) software.

### Methods

In some cases, the compounds and precursors and/or intermediates thereof were analysed by LC-MS.

### Method A1: UPLC (MeCN-HCOOH):

Instrument: Waters Acquity UPLC-MS SQD 3001; column: Acquity UPLC BEH C18 1.7 50 x 2.1 mm; eluent A: water + 0.1% by vol. of formic acid (99%), eluent B: acetonitrile; gradient: 0-1.6 min 1-99% B, 1.6-2.0 min 99% B; flow rate 0.8 ml/min; temperature: 60°C; injection: 2 µl; DAD scan: 210-400 nm; MS ESI+, ESI-, scan range 160-1000 m/z; ELSD.

### Method A2: UPLC (MeCN-NH₃):

Instrument: Waters Acquity UPLC-MS SQD 3001; column: Acquity UPLC BEH C18 1.7 50 x 2.1 mm; eluent A: water + 0.2% by vol. of ammonia (32%), eluent B: acetonitrile; gradient: 0-1.6 min 1-99% B, 1.6-2.0 min 99% B; flow rate 0.8 ml/min; temperature: 60°C; injection: 2 µl; DAD scan: 210-400 nm; MS ESI+, ESI-, scan range 160-1000 m/z; ELSD.

### Method A3: (LC-MS)

Instrument: Agilent 1290 Infinity LC; column: Acquity UPLC BEH C18 1.7 50 x 2.1 mm; eluent A: water + 0.05% by vol. of formic acid, eluent B: acetonitrile + 0.05% by vol. of formic acid; gradient: 0-1.7 min 2-90% B, 1.7-2.0 min 90% B; flow rate 1.2 ml/min; temperature: 60°C; injection: 2 µl; DAD scan: 190-390 nm; MS: Agilent TOF 6230.

### Method A4: (LC-MS)

Instrument: Waters Acquity; column: Kinetex (Phenomenex), 50 x 2 mm; eluent A: water + 0.05% by vol. of formic acid, eluent B: acetonitrile + 0.05% by vol. of formic acid; gradient: 0-1.9 min 1-99% B, 1.9-2.1 min 99% B; flow rate 1.5 ml/min; temperature: 60°C; injection: 0.5 µl; DAD scan: 200-400 nm.

In some cases, the compounds of the formula (I) as constituents of the inventive combinations and the precursors and/or intermediates thereof were purified by the following preparative HPLC methods:
Method P1: system: Waters Autopurification system: Pump 2545, Sample Manager 2767, CFO, DAD 2996, ELSD 2424, SQD; column: XBridge C18 5 µm 100 x 30 mm; eluent A: water + 0.1% by vol. of formic acid, eluent B: acetonitrile; gradient: 0-8 min 10-100% B, 8-10 min 100% B; flow: 50 ml/min; temperature: room temperature; solution: max. 250 mg / max. 2.5 ml DMSO or DMF; injection: 1 x 2.5 ml; detection: DAD scan range 210-400 nm; MS ESI+, ESI-, scan range 160-1000 m/z.
Method P2: system: Waters Autopurification system: Pump 254, Sample Manager 2767, CFO, DAD 2996, ELSD 2424, SQD 3100; column: XBridge C18 5 µm 10 x 30 mm; eluent A: water + 0.2% by vol. of ammonia (32%), eluent B: methanol; gradient: 0-8 min 30-70% B; flow: 50 ml/min; temperature: room temperature; detection: DAD scan range 210-400 nm; MS ESI+, ESI-, scan range 160-1000 m/z; ELSD.
Method P3: system: Labomatic, pump: HD-5000, fraction collector: LABOCOL Vario-4000, UV detector: Knauer UVD 2.1S; column: XBridge C18 5 µm 100x30 mm; eluent A: water + 0.2% by vol. of ammonia (25%), eluent B: acetonitrile; gradient: 0-1 min 15% B, 1-6.3 min 15-55% B, 6.3-6.4 min 55-100% B, 6.4-7.4 min 100% B; flow: 60 ml/min; temperature: room temperature; solution: max. 250 mg / 2 ml DMSO; injection: 2 x 2 ml; detection: UV 218 nm; Software: SCPA PrepCon5.
Method P4: system: Labomatic, pump: HD-5000, fraction collector: LABOCOL Vario-4000, UV detector: Knauer UVD 2.1S; column: Chromatorex RP C18 10 µm 125 x 30 mm; eluent A: water + 0.1% by vol. of formic acid, eluent B: acetonitrile; gradient: 0-15 min 65 - 100% B; flow: 60 ml/min; temperature: room temperature; solution: max. 250 mg / 2 ml DMSO; injection: 2 x 2 ml; detection: UV 254 nm; Software: SCPA PrepCon5.
Method P5: system: Sepiatec: Prep SFC100, column: Chiralpak IA 5 µm 250x20 mm; eluent A: carbon dioxide, eluent B: ethanol; gradient: isocratic 20% B; flow: 80 ml/min; temperature: 40°C; solution: max. 250 mg / 2 ml DMSO; injection: 5 x 0.4 mL; detection: UV 254 nm.
Method P6: system: Agilent: Prep 1200, 2 x prep pump, DLA, MWD, Gilson: Liquid Handler 215; column: Chiralcel OJ-H 5 µm 250 x 20 mm; eluent A: hexane, eluent B: ethanol; gradient: isocratic 30% B; flow: 25 ml/min; temperature: 25°C; solution: 187 mg / 8 ml ethanol/methanol; injection: 8 x 1.0 ml; detection: UV 280 nm.
Method P7: system: Labomatic, pump: HD-5000, fraction collector: LABOCOL Vario-4000, UV detector: Knauer UVD 2.1S; column: XBridge C18 5 µm 100 x 30 mm; eluent A: water + 0.1% by vol. of formic acid, eluent B: acetonitrile; gradient: 0-3 min: 65% B isocratic, 3-13 min: 65-100% B; flow: 60 ml/min; temperature: room temperature; solution: max. 250 mg / 2 ml DMSO; injection: 2 x 2 ml; detection: UV 254 nm.
Method P8: system: Agilent: Prep 1200, 2 x prep pump, DLA, MWD, Gilson: Liquid Handler 215; column: Chiralpak IF 5 µm 250 x 20 mm; eluent A: ethanol, eluent B: methanol; gradient: isocratic 50% B; flow: 25 ml/min; temperature: 25°C; solution: 600 mg / 7 ml N,N-dimethylformamide; injection: 10 x 0.7 ml; detection: UV 254 nm.

In some cases, substance mixtures were purified by column chromatography on silica gel.

For preparation of the compounds of the formula (I) as constituents of the inventive combinations and the precursors and/or intermediates thereof, a column chromatography purification ("flash chromatography") was conducted on silica gel using lsolera^{®} devices from Biotage. This involved using cartridges from Biotage, for example the "SNAP Cartridge, KP_SIL" cartridge of different size and "Interchim Puriflash Silica HP 15UM flash column" cartridges from Interchim of different size.

### Starting materials

### Intermediate V2-1

### Methyl 6-(2-hydroxypropan-2-yl)pyridine-2-carboxylate

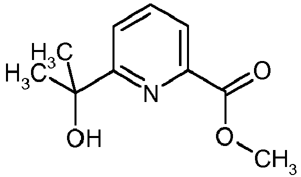

2.00 g (9.26 mmol) of 2-(6-bromopyridin-2-yl)propan-2-ol (CAS 638218-78-7) were dissolved in 20 ml of methanol and 20 ml of DMSO. Subsequently, 250 mg of 1,3-bis(diphenylphosphino)propane, 130 mg of palladium(II) acetate and 3 ml of triethylamine were added. The reaction mixture was purged three times with carbon monoxide at room temperature and stirred under a 13 bar carbon monoxide atmosphere for 30 min. The carbon monoxide atmosphere was removed by applying a vacuum and the mixture was stirred under a 14 bar carbon monoxide atmosphere at 100°C for 24 h. The autoclave was decompressed, water was added to the reaction mixture, and the reaction mixture was extracted three times with ethyl acetate, washed with saturated aqueous sodium hydrogencarbonate solution and sodium chloride solution, filtered through a hydrophobic filter and concentrated. This gave 1.60 g of a crude product.
UPLC-MS (Method A1): Rₜ = 0.76 min (UV detector: TIC), mass found 195.00.

### Intermediate V3-1

### Potassium 6-(2-hydroxypropan-2-yl)pyridine-2-carboxylate

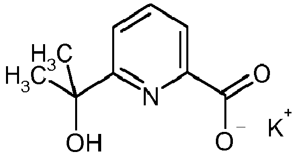

1.60 g of the crude product of Intermediate 0-1 were initially charged in 15 ml of methanol, 0.74 g of potassium hydroxide was added and the mixture was stirred at 50°C for 16.5 h. After concentration, this gave 2.1 g of a solid which was used without further purification.
UPLC-MS (Method A1): Rₜ = 0.47 min (UV detector: TIC), mass found 181.00.

### Intermediate 1-1

### Methyl 5-nitro-1H-indazole-6-carboxylate

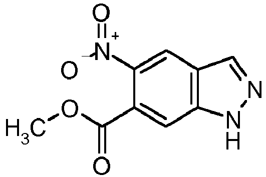

4.60 g (26.1 mmol) of methyl 1H-indazole-6-carboxylate (CAS No: 170487-40-8) were dissolved in 120 ml of sulphuric acid (96%) and cooled to -15°C in a three-neck flask having a CPG stirrer, dropping funnel and internal thermometer. Over a period of 15 min, the nitrating acid (10 ml of 96% sulphuric acid in 5 ml of 65% nitric acid), which had been prepared and cooled beforehand, was added dropwise to this solution. After the dropwise addition had ended, the mixture was stirred for a further 1 h (internal temperature at -13°C). The reaction mixture was added to ice, and the precipitate formed was filtered off with suction, washed with water and dried in a drying cabinet at 50°C under reduced pressure. 5.49 g of the title compound were obtained.
UPLC-MS (Method A2): Rₜ = 0.75 min
MS (ESlpos): m/z = 222(M+H)⁺
¹H NMR (400 MHz, DMSO-d6): δ [ppm] = 3.87 (s, 3 H), 7.96 (s, 1 H), 8.44 (s, 1 H), 8.70 (s, 1 H), 13.98 (br. s., 1 H).

### Intermediate 2-1

### Methyl 5-amino-1H-indazole-6-carboxylate

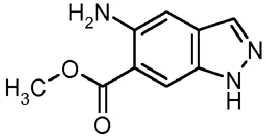

4.40 g (19.8 mmol) of methyl 5-nitro-1H-indazole-6-carboxylate (Intermediate 1-1) were dissolved in 236 ml of methanol and hydrogenated with 1.06 g (0.99 mmol) of palladium on activated carbon under standard hydrogen pressure at 25°C for 3 h. The reaction mixture was filtered through Celite, the filter was washed with methanol, and the filtrate was concentrated. 3.53 g of the title compound were obtained.
¹H NMR (300 MHz, DMSO-d6): δ [ppm] = 3.85 (s, 3 H) 6.01 (s, 2 H) 6.98 (s, 1 H) 7.79 - 7.91 (m, 1 H) 7.99 (s, 1 H) 12.84 (br. s., 1 H).

### Intermediate 3-1

### Methyl 5-({[6-(trifluoromethyl)pyridin-2-yl]carbonyl}amino)-1H-indazole-6-carboxylate

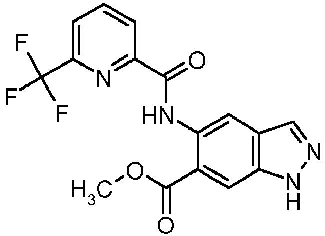

4.95 g (25.9 mmol) of 6-(trifluoromethyl)pyridine-2-carboxylic acid were initially charged in 45 ml of THF. 9.07 g (28.2 mmol) of O-(benzotriazol-1-yl)-N,N,N',N'-tetramethyluronium tetrafluoroborate and 4.92 ml (28.2 mmol) of N-ethyl-N-isopropylpropan-2-amine were added and the mixture was stirred at 25°C for 30 min. Subsequently, 4.50 g (23.5 mmol) of methyl 5-amino-1H-indazole-6-carboxylate (Intermediate 2-1) were added and the mixture was stirred at 25°C for 24 h. The reaction mixture was filtered with suction through a membrane filter and washed with THF and with water, and dried in a drying cabinet overnight. 7.60 g of the title compound were obtained.
UPLC-MS (Method A2): Rₜ = 1.16 min
MS (ESlpos): m/z = 365 (M+H)⁺
¹H NMR (400 MHz, DMSO-d6): δ [ppm] = 3.97 (s, 3 H), 8.13 - 8.27 (m, 2 H), 8.30 (s, 1 H), 8.33 - 8.45 (m, 1 H), 8.45 - 8.51 (m, 1 H), 9.15 (s, 1 H), 12.57 (s, 1 H), 13.44 (s, 1 H).

### Intermediate 3-2

### Methyl 5-({[6-(difluoromethyl)pyridin-2-yl]carbonyl}amino)-1H-indazole-6-carboxylate

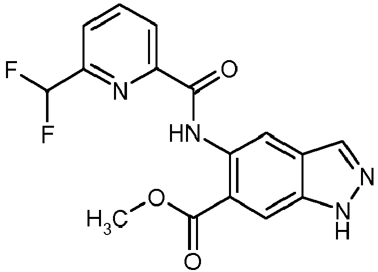

2.85 g (23.5 mmol) of 6-(difluoromethyl)pyridine-2-carboxylic acid were initially charged in 30 ml of THF. 6.05 g (18.8 mmol) of O-(benzotriazol-1-yl)-N,N,N',N'-tetramethyluronium tetrafluoroborate and 3.3 ml of N-ethyl-N-isopropylpropan-2-amine were added and the mixture was stirred at room temperature for 10 minutes. Subsequently, 3.00 g (15.7 mmol) of methyl 5-amino-1H-indazole-6-carboxylate were added and the mixture was stirred at room temperature overnight. The reaction mixture was admixed with water, and the precipitate was filtered off with suction and washed repeatedly with water and dichloromethane. This gave 1.53 g (27% of theory) of the title compound. The phases of the filtrate were separated, the organic phase was concentrated, admixed with a little dichloromethane and suspended in an ultrasound bath, and the precipitate was filtered off with suction. This gave a further 1.03 g of the title compound.
1H-NMR (first product fraction, 300MHz, DMSO-d6): δ [ppm]= 3.99 (s, 3H), 7.09 (t, 1H), 8.00 (d, 1H), 8.21 - 8.40 (m, 4H), 9.14 (s, 1H), 12.53 (s, 1H), 13.44 (s, 1H).

### Intermediate 3-3

### Methyl 5-({[6-(2-hydroxypropan-2-yl)pyridin-2-yl]carbonyl}amino)-1H-indazole-6-carboxylate

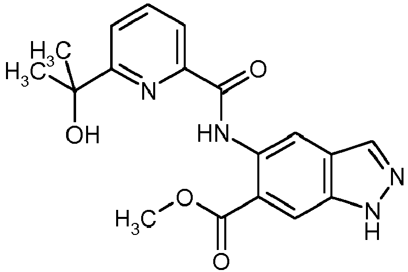

2.10 g of potassium 6-(2-hydroxypropan-2-yl)pyridine-2-carboxylate (Intermediate V3-1) were initially charged in 15 ml of THF. 3.69 g (11.5 mmol) of O-(benzotriazol-1-yl)-N,N,N',N'-tetramethyluronium tetrafluoroborate and 2.00 ml of N-ethyl-N-isopropylpropan-2-amine were added and the mixture was stirred at room temperature for 15 min. Subsequently, 1.83 g (9.58 mmol) of methyl 5-amino-1H-indazole-6-carboxylate (Intermediate 2-1) were added and the mixture was stirred at room temperature for 19 h. The mixture was admixed with water and ethyl acetate, the undissolved solids were filtered off, the phases of the filtrate were separated, and the aqueous phase was extracted twice with ethyl acetate, washed with sodium chloride solution, filtered through a hydrophobic filter, concentrated and purified by column chromatography on silica gel (hexane/ethyl acetate). After the solvents had been removed, 1.56 g of the title compound were obtained as a yellow foam.
UPLC-MS (Method A1): Rₜ = 1.00 min (UV detector: TIC Smooth), mass found 354.00.
1H-NMR (500MHz,DMSO-d6): δ [ppm] = 1.63 (s, 6H), 3.97 (s, 3H), 5.37(s ,1H), 7.90 - 7.95 (m, 1H), 8.03-8.07 (m, 2H), 8.23(s, 1H),8.29 (s, 1H), 9.19 (s, 1H), 12.79 (s, 1H), 13.41 (br.s., 1H).

### Intermediate 4-1

### Methyl 2-(oxetan-3-ylmethyl)-5-({[6-(trifluoromethyl)pyridin-2-yl]carbonyl}amino)-2H-indazole-6-carboxylate

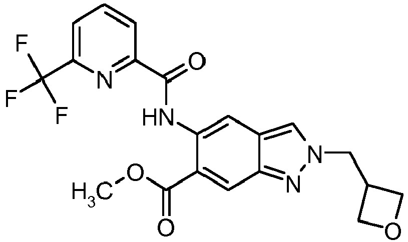

1.00 mg (2.66 mmol) of methyl 5-({[6-(trifluoromethyl)pyridin-2-yl]carbonyl}amino)-1H-indazole-6-carboxylate (Intermediate 3-1) was dissolved in 10 ml of DMF and, after addition of 1.10 mg (7.99 mmol) of potassium carbonate and 221 mg (1.33 mmol) of potassium iodide, the mixture was stirred at 25°C for 30 min. 603 mg (3.99 mmol) of 3-bromomethyloxetane were added, and the mixture was stirred at 25°C for 24 h. The reaction mixture was partitioned between water and ethyl acetate. The mixture was extracted twice with ethyl acetate, and the combined organic phases were filtered through a hydrophobic filter and concentrated. The residue was purified by column chromatography on silica gel (hexane/ethyl acetate). This gave 260 mg of the title compound.
UPLC-MS (Method A2): Rₜ = 1.24 min
MS (ESlpos): m/z = 435(M+H)⁺
¹H NMR (400 MHz, DMSO-d6): δ [ppm] = 3.49 - 3.64 (m, 1 H), 3.95 (s, 3 H), 4.49 (t, 2 H), 4.68 (dd, 2 H), 4.81 (d, 2 H), 8.20 (dd, 1 H), 8.35 - 8.41 (m, 1 H), 8.43 - 8.49 (m, 2 H), 8.55 - 8.58 (m, 1 H), 9.06 (s, 1 H), 12.53 (s, 1 H).

### Intermediate 4-2

### Methyl 2-(2-methoxyethyl)-5-({[6-(trifluoromethyl)pyridin-2-yl]carbonyl}amino)-2H-indazole-6-carboxylate

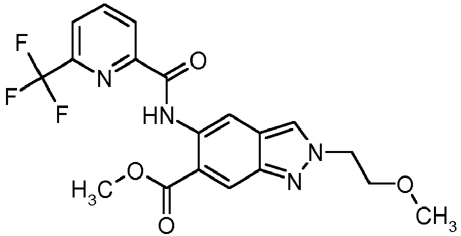

1.00 mg (2.75 mmol) of methyl 5-({[6-(trifluoromethyl)pyridin-2-yl]carbonyl}amino)-1H-indazole-6-carboxylate (Intermediate 3-1) was dissolved in 5 ml of DMF, and 387 µl (4.12 mmol) of 2-bromoethyl methyl ether, 1.14 g (8.23 mmol) of potassium carbonate and 228 mg (1.37 mmol) of potassium iodide were added while stirring. The reaction mixture was stirred at 25°C for 24 h, diluted with water and extracted twice with ethyl acetate. The combined organic phases were filtered through a hydrophobic filter and concentrated. The residue was purified by column chromatography on silica gel (hexane/ethyl acetate). This gave 12 mg of the title compound.
UPLC-MS (Method A1): Rₜ = 1.24 min
MS (ESlpos): m/z = 423 (M+H)⁺
¹H NMR (400 MHz, DMSO-d6): δ [ppm] = 3.24 (s, 3 H), 3.86 (t, 2 H), 3.96 (s, 3 H), 4.65 (t, 2 H), 8.21 (dd, 1 H), 8.35 - 8.42 (m, 1 H), 8.43 - 8.51 (m, 2 H), 8.52 (d, 1 H), 9.06 (s, 1 H), 12.53 (s, 1 H).

### Intermediate 4-3

### Methyl 2-(3-methoxypropyl)-5-({[6-(trifluoromethyl)pyridin-2-yl]carbonyl}amino)-2H-indazole-6-carboxylate

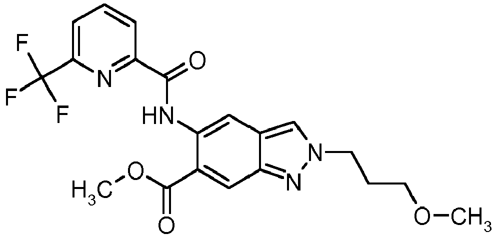

1.00 mg (2.75 mmol) of methyl 5-({[6-(trifluoromethyl)pyridin-2-yl]carbonyl}amino)-1H-indazole-6-carboxylate (Intermediate 3-1) was dissolved in 5 ml of DMF, and 460 µl (4.12 mmol) of 1-bromo-3-methoxypropane, 1.14 g (8.23 mmol) of potassium carbonate and 228 mg (1.37 mmol) of potassium iodide were added while stirring. The reaction mixture was stirred at 25°C for 72 h, diluted with water and extracted twice with ethyl acetate. The combined organic phases were filtered through a hydrophobic filter and concentrated. The residue was purified by column chromatography on silica gel (hexane/ethyl acetate). This gave 28 mg of the title compound.
UPLC-MS (Method A1): Rₜ = 1.29 min
MS (ESlpos): m/z = 437 (M+H)⁺
¹H NMR (400 MHz, DMSO-d6): δ [ppm] = 2.17 (quin, 2 H), 3.24 (s, 3 H), 3.33 - 3.36 (m, 2 H), 3.96 (s, 3 H), 4.53 (t, 2 H), 8.21 (dd, 1 H), 8.35 - 8.42 (m, 1 H), 8.45 - 8.49 (m, 2 H), 8.54 (d, 1 H), 9.06 (s, 1 H), 12.54 (s, 1 H).

### Intermediate 4-4

### Methyl 2-(3-hydroxy-3-methylbutyl)-5-({[6-(trifluoromethyl)pyridin-2-yl]carbonyl}amino)-2H-indazole-6-carboxylate

### Preparation Method 1

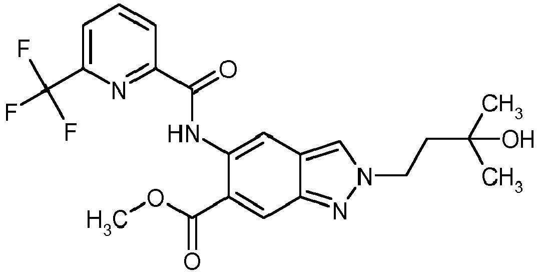

930 mg (2.55 mmol) of methyl 5-({[6-(trifluoromethyl)pyridin-2-yl]carbonyl}amino)-1H-indazole-6-carboxylate (Intermediate 3-1), 1.06 g of potassium carbonate and 212 mg of potassium iodide were initially charged in 9 ml of DMF and the mixture was stirred for 15 min. Then 0.62 ml of 4-bromo-2-methylbutan-2-ol was added and the mixture was stirred at 60°C overnight. The mixture was admixed with water and extracted twice with ethyl acetate, and the extract was washed three times with saturated sodium chloride solution, filtered and concentrated. Column chromatography purification on silica gel (hexane/ethyl acetate) gave 424 g of the title compound.
UPLC-MS (Method A2): Rₜ = 1.21 min (UV detector: TIC), mass found 450.00.
¹H-NMR (400MHz, DMSO-d₆): δ [ppm]= 1.16 (s, 6 H) 2.02 - 2.11 (m, 2 H) 3.96 (s, 3 H) 4.51 - 4.60 (m, 3 H) 8.20 (dd, *J*=7.83, 1.01 Hz, 1 H) 8.39 (s, 1 H) 8.45 (s, 2 H) 8.55 (d, *J*=0.76 Hz, 1 H) 9.05 (s, 1 H) 12.52 (s, 1 H).

### Preparation Method 2

1.95 g (7.03 mmol) of methyl 5-amino-2-(3-hydroxy-3-methylbutyl)-2H-indazole-6-carboxylate (Intermediate 7-1) were initially charged in 30 ml of THF. 1.45 g (7.73 mmol) of 6-(trifluoromethyl)pyridine-2-carboxylic acid, 2.71 g (8.44 mmol) of O-(benzotriazol-1-yl)-N,N,N',N'-tetramethyluronium tetrafluoroborate and 1.47 ml (8.44 mmol) of N-ethyl-N-isopropylpropan-2-amine were added and the mixture was stirred at 25°C for 20.5 h. Water was added, the mixture was extracted three times with ethyl acetate and the extracts were washed with sodium chloride solution, filtered through a hydrophobic filter and concentrated. The residue was separated by column chromatography on silica gel (hexane/ethyl acetate). This gave 2.79 g of the title compound.
UPLC-MS (Method A1): Rₜ = 1.23 min (UV detector: TIC), mass found 450.00.

### Intermediate 4-5

### Methyl 2-(2-{[tert-butyl(dimethyl)silyl]oxy}ethyl)-5-({[6-(trifluoromethyl)pyridin-2-yl]carbonyl}amino)-2H-indazole-6-carboxylate

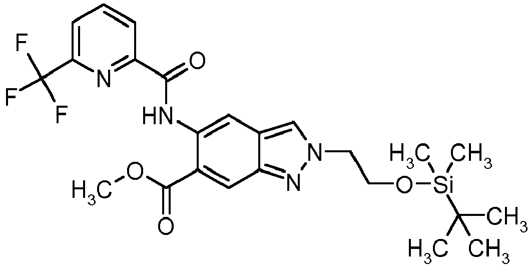

1.00 g (2.66 mmol, 97%) of methyl 5-({[6-(trifluoromethyl)pyridin-2-yl]carbonyl}amino)-1H-indazole-6-carboxylate (Intermediate 3-1) was initially charged in 50 ml of DMF, 1.10 g (7.99 mmol) of potassium carbonate and 221 mg (1.33 mmol) of potassium iodide were added while stirring, and the mixture was stirred at 25°C for 30 min. Subsequently, 857 µl (3.99 mmol) of (2-bromoethoxy)(tert-butyl)dimethylsilane were added and the mixture was stirred at 25°C for 24 h. The reaction mixture was diluted with water and extracted with ethyl acetate. The combined organic phases were filtered through a hydrophobic filter and concentrated. The residue was purified by column chromatography on silica gel (hexane/ethyl acetate). This gave 400 mg of the title compound.
UPLC-MS (Method A1): Rₜ = 1.58 min
MS (ESlpos): m/z = 523(M+H)⁺
¹H NMR (300 MHz, DMSO-d6): δ [ppm] = -0.18 - -0.13 (m, 6 H), 0.74 (s, 9 H), 3.96 (s, 3 H), 4.08 (t, 2 H), 4.57 (t, 2 H), 8.15 - 8.25 (m, 1 H), 8.32 - 8.43 (m, 1 H), 8.43 - 8.52 (m, 3 H), 9.07 (s, 1 H), 12.53 (s, 1 H).

### Intermediate 4-6

### Methyl 2-(3-{[tert-butyl(dimethyl)silyl]oxy}propyl)-5-({[6-(trifluoromethyl)pyridin-2-yl]carbonyl}amino)-2H-indazole-6-carboxylate

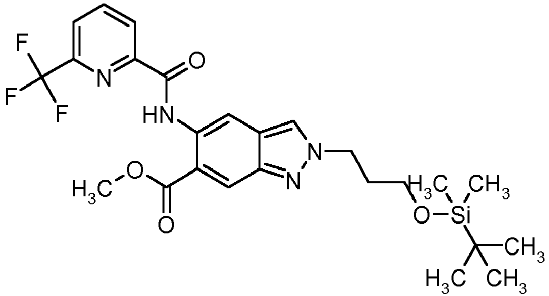

Analogously to Intermediate 4-5, 1.00 g (2.75 mmol) of methyl 5-({[6-(trifluoromethyl)pyridin-2-yl]carbonyl}amino)-1H-indazole-6-carboxylate (Intermediate 7-1) was dissolved in 10 ml of DMF 1.14 g (8.24 mmol) of potassium carbonate and 228 mg (1.37 mmol) of potassium iodide were added while stirring, and the mixture was stirred at 25°C for 30 min. Subsequently, 1.04 g (4.12 mmol) of (3-bromopropoxy)(tert-butyl)dimethylsilane were added and the mixture was stirred at 25°C for 24 h. The reaction mixture was filtered and the filtercake was washed with ethyl acetate. The reaction mixture was partitioned between water and ethyl acetate and the aqueous phase was extracted twice with ethyl acetate. The combined organic phases were filtered through a hydrophobic filter and concentrated. Purification of the residue by preparative HPLC gave 428 mg of the title compound.
UPLC-MS (Method A1): Rₜ = 1.63 min
MS (ESlpos): m/z = 537 (M+H)⁺
¹H NMR (400 MHz, DMSO-d6): δ [ppm] = -0.02 - 0.06 (m, 6 H), 0.87 (s, 9 H), 2.14 (quin, 2 H), 3.62 (t, 2 H), 3.96 (s, 3 H), 4.54 (t, 2 H), 8.20 (d, 1 H), 8.35 - 8.42 (m, 1 H), 8.43 - 8.48 (m, 2 H), 8.49 - 8.53 (m, 1 H), 9.06 (s, 1 H).

### Intermediate 4-7

### Methyl 5-({[6-(2-hydroxypropan-2-yl)pyridin-2-yl]carbonyl}amino)-2-(4,4,4-trifluorobutyl)-2H-indazole-6-carboxylate

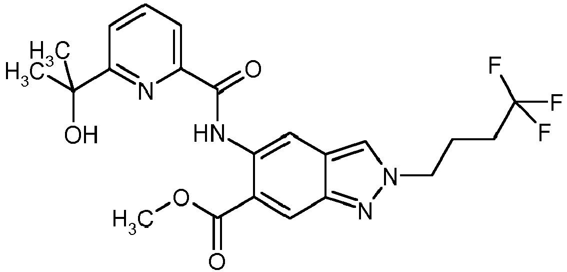

300 mg of methyl 5-({[6-(2-hydroxypropan-2-yl)pyridin-2-yl]carbonyl}amino)-1H-indazole-6-carboxylate (Intermediate 3-3) were initially charged in 4.5 ml of DMF. 287 mg of 1,1,1-trifluoro-4-iodobutane and 333 mg of potassium carbonate were added and the mixture was stirred at 100°C for 23 h. Water was added, and the mixture was extracted three times with ethyl acetate. The mixture was concentrated and the product was purified by preparative HPLC. This gave 72 mg of the title compound.
UPLC-MS (Method A1): Rₜ = 1.26 min (UV detector: TIC), mass found 464.17.

### Intermediate 4-8

### Methyl 5-{[(5-fluoro-6-methylpyridin-2-yl)carbonyl]amino}-2-(3-hydroxy-3-methylbutyl)-2H-indazole-6-carboxylate

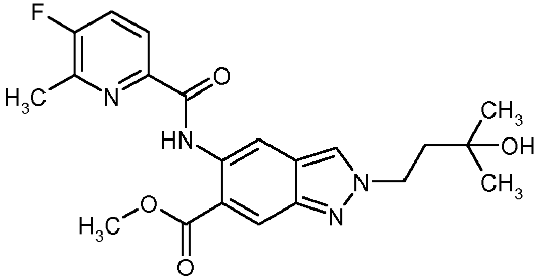

195 mg of methyl 5-amino-2-(3-hydroxy-3-methylbutyl)-2H-indazole-6-carboxylate (Intermediate 7-1) were reacted with 78 mg of 5-fluoro-6-methylpyridine-2-carboxylic acid analogous to Intermediate 4-4 (Preparation Method 2) within 19.5 h. 228 mg of a crude product were obtained after analogous aqueous workup.
UPLC-MS (Method A1): Rₜ = 1.20 min (UV detector: TIC), mass found 414.00.

### Intermediate 4-9

### Methyl 2-(3-hydroxy-3-methylbutyl)-5-{[(6-methylpyridin-2-yl)carbonyl]amino}-2H-indazole-6-carboxylate

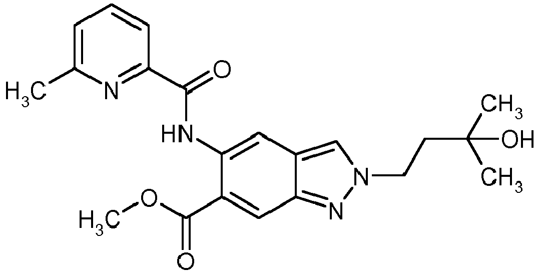

195 mg of methyl 5-amino-2-(3-hydroxy-3-methylbutyl)-2H-indazole-6-carboxylate (Intermediate 7-1) were reacted with 70 mg of 6-methylpyridine-2-carboxylic acid analogous to the preparation of Intermediate 4-4 (Preparation Method 2) within 19.5 h. 278 mg of the title compound as crude product were obtained after analogous aqueous workup.
UPLC-MS (Method A1): Rₜ = 1.14 min (UV detector: TIC), mass found 396.00.

### Intermediate 4-10

### Methyl 2-[3-(2,2,2-trifluoroethoxy)propyl]-5-({[6-(trifluoromethyl)pyridin-2-yl]carbonyl}amino)-2H-indazole-6-carboxylate

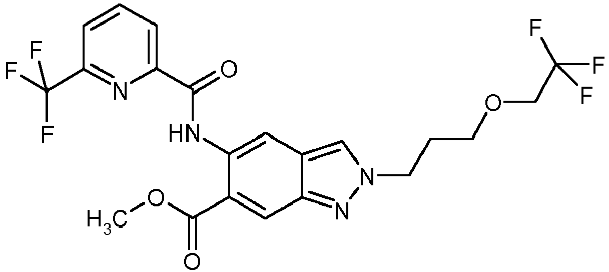

A mixture of 250 mg of methyl 5-({[6-(trifluoromethyl)pyridin-2-yl]carbonyl}amino)-1H-indazole-6-carboxylate (Intermediate 7-1), 193 mg of 3-bromopropyl 2,2,2-trifluoroethyl ether, 242 mg of potassium carbonate and 145 mg of potassium iodide in 3 ml of DMF was stirred at 100°C for 20 h. Water was added, the mixture was extracted with ethyl acetate and the extract was washed with sodium chloride solution and concentrated. Purification by preparative HPLC gave 52 mg of the title compound.
UPLC-MS (Method A1): Rₜ = 1.39 min (UV detector: TIC), mass found 504.12.

### Intermediate 5-1

### N-[6-(2-Hydroxypropan-2-yl)-1H-indazol-5-yl]-6-(trifluoromethyl)pyridine-2-carboxamide

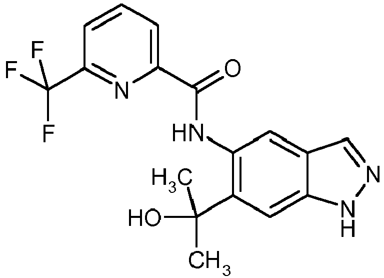

To a solution, cooled in an ice-water cooling bath, of 1.50 g (4.12 mmol) of methyl 5-({[6-(trifluoromethyl)pyridin-2-yl]carbonyl}amino)-1H-indazole-6-carboxylate (Intermediate 3-1) in 20 ml of THF were cautiously added 6.9 ml (5 equivalents) of a 3M methylmagnesium bromide solution in diethyl ether. The mixture was stirred while cooling with an ice bath for 1 h and at room temperature for 19.5 h. Another 2 equivalents of methylmagnesium bromide solution were added and the mixture was stirred at room temperature for a further 24 h. Saturated aqueous ammonium chloride solution was added and the mixture was stirred and extracted three times with ethyl acetate. The combined organic phases were washed with sodium chloride solution, filtered through a hydrophobic filter and concentrated. The residue was purified by column chromatography on silica gel (hexane/ethyl acetate). This gave 763 mg of the title compound.
¹H-NMR (400MHz, DMSO-d₆): δ [ppm]= 1.63 (s, 6H), 5.99 (s, 1H), 7.49 (s, 1H), 8.06 (s, 1H), 8.14 - 8.19 (m, 1H), 8.37 (t, 1H), 8.46 (d, 1H), 8.78 (s, 1H), 12.32 (s, 1H), 12.97 (s, 1H).

### Intermediate 5-2

### 6-(Difluoromethyl)-N-[6-(2-hydroxypropan-2-yl)-1H-indazol-5-yl]pyridine-2-carboxamide

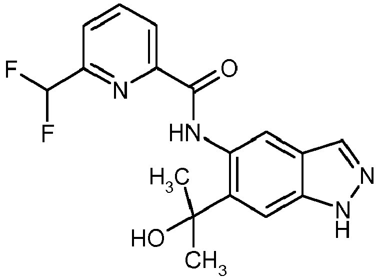

Analogously to the preparation of Intermediate 5-1, 2.40 g (6.93 mmol) of methyl 5-({[6-(difluoromethyl)pyridin-2-yl]carbonyl}amino)-1H-indazole-6-carboxylate (Intermediate 3-2) in 10 ml of THF were reacted with three portions of 3M methylmagnesium bromide solution in diethyl ether (6.9 ml, then stirring at room temperature for 45 min; 11.6 ml, then stirring at room temperature for 2 h; 6.9 ml, then stirring at room temperature for 2 h). After the workup as for Intermediate 5-1, 2.39 g of a crude product were obtained, which were used further without further purification.

### Intermediate 6-1

### Methyl 2-(3-hydroxy-3-methylbutyl)-5-nitro-2H-indazole-6-carboxylate

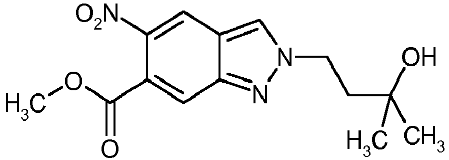

5.00 g (22.6 mmol) of methyl 5-nitro-1H-indazole-6-carboxylate (Intermediate 1-1) were initially charged in 40 ml of DMF. 5.65 g (33.9 mmol) of 4-bromo-2-methylbutan-2-ol, 9.37 g (67.8 mmol) of potassium carbonate and 5.63 g (33.9 mmol) of potassium iodide were added and the mixture was stirred at 100°C for 20 h. Water was added, the mixture was extracted three times with ethyl acetate and the extracts were washed with sodium chloride solution, filtered through a hydrophobic filter and concentrated. The residue was purified by column chromatography on silica gel (hexane/ethyl acetate). The solids obtained were extracted by stirring with diethyl ether, filtered off with suction, washed with diethyl ether and dried. This gave 2.49 g of the title compound.
UPLC-MS (Method A1): Rₜ = 0.93 min (UV detector: TIC), mass found 307.00.
¹H-NMR (400MHz, DMSO-d₆): δ [ppm]= 1.15 (s, 6H), 2.02 - 2.11 (m, 2H), 3.84 (s, 3H), 4.54 (s, 1H), 4.58 - 4.65 (m, 2H), 8.05 (s, 1H), 8.69 (s, 1H), 8.86 (s, 1H).

### Intermediate 7-1

### Methyl 5-amino-2-(3-hydroxy-3-methylbutyl)-2H-indazole-6-carboxylate

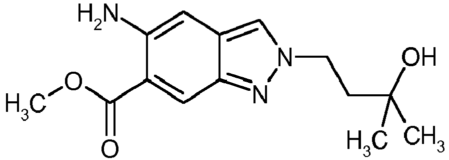

4.53 g of iron and 217 mg of ammonium chloride were added to 2.49 g (8.10 mmol) of methyl 2-(3-hydroxy-3-methylbutyl)-5-nitro-2H-indazole-6-carboxylate (Intermediate 6-1) in 30 ml of ethanol and 10 ml of water, and the mixture was stirred at 90°C for 21.5 h. The mixture was filtered through Celite and washed through with ethanol three times, and the filtrate was concentrated and the residue was admixed with water. Extraction was effected three times with ethyl acetate (to improve the phase separation, sodium chloride solution was added). The combined organic phases were washed with sodium chloride solution, filtered through a hydrophobic filter and concentrated. This gave 1.95 g (85% of theory) of the title compound.
UPLC-MS (Method A1): Rₜ = 0.67 min (UV detector: TIC), mass found 277.00.
¹H-NMR (400MHz, DMSO-d₆): δ [ppm]= 1.14 (s, 6H), 1.96 - 2.08 (m, 2H), 3.85 (s, 3H), 4.39 - 4.51 (m, 3H), 5.81 (s, 2H), 6.80 (s, 1H), 8.05 (s, 1H), 8.18 (s, 1H).

### Example 1

### N-[6-(2-Hydroxypropan-2-yl)-2-(2-methoxyethyl)-2H-indazol-5-yl]-6-(trifluoromethyl)pyridine-2-carboxamide

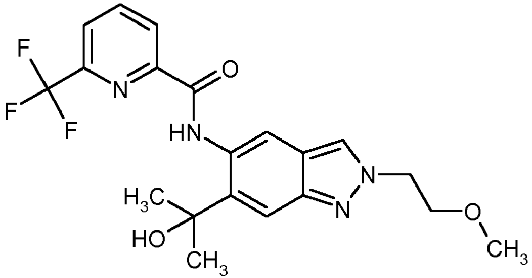

75 mg (0.18 mmol) of methyl 2-(2-methoxyethyl)-5-({[6-(trifluoromethyl)pyridin-2-yl]carbonyl}amino)-2H-indazole-6-carboxylate (Intermediate 4-2) were dissolved in 500 µl of THF and admixed with 887 µl (0.89 mmol) of a 1 M methylmagnesium bromide solution in THF. The reaction mixture was stirred at 25°C for 60 min. Subsequently, 1 ml of a saturated aqueous ammonium chloride solution was added cautiously and the mixture was filtered. The aqueous phase was extracted twice with ethyl acetate, and the organic phases were combined, filtered through a hydrophobic filter and concentrated. The residue was dissolved in 3 ml of DMSO and purified by preparative HPLC. The product-containing fractions were freeze-dried. This gave 20 mg of the title compound.
UPLC-MS (Method A1): Rₜ = 1.08 min
MS (ESlpos): m/z = 423 (M+H)⁺
¹H NMR (300 MHz, DMSO-d6): δ [ppm] = 1.62 (s, 6 H), 3.22 (s, 3 H), 3.82 (t, J=5.2 Hz, 2 H), 4.55 (t, J=5.2 Hz, 2 H), 5.96 (s, 1 H), 7.57 (s, 1 H), 8.16 (d, J=7.2 Hz, 1 H), 8.29 - 8.42 (m, 2 H), 8.42 - 8.50 (m, 1 H), 8.71 (s, 1 H), 12.36 (s, 1 H)

### Example 2

### N-[6-(Hydroxymethyl)-2-(2-methoxyethyl)-2H-indazol-5-yl]-6-(trifluoromethyl)pyridine-2-carboxamide

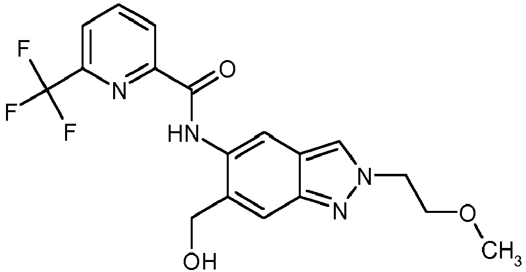

13 mg (0.36 mmol) of lithium aluminium hydride were suspended in 1 ml of THF and the mixture was cooled to 0°C. 75 mg (0.17 mmol) of methyl 2-(2-methoxyethyl)-5-({[6-(trifluoromethyl)pyridin-2-yl]carbonyl}amino)-2H-indazole-6-carboxylate (Intermediate 4-2) dissolved in 500 µl of THF were added dropwise and the mixture was stirred at 25°C for 60 min. The mixture was diluted with water and extracted twice with ethyl acetate, and the combined organic phases were washed with sodium chloride solution, filtered through a hydrophobic filter, concentrated and dried under reduced pressure. This gave 36 mg of the title compound.
UPLC-MS (Method A2): Rₜ = 0.97 min
MS (ESlpos): m/z = 409 (M+H)⁺
¹H NMR (400 MHz, DMSO-d6): δ [ppm] = 1.62 (s, 6 H), 3.86 (q, 2 H), 4.43 (t, 2 H), 4.95 (t, 1 H), 5.94 (s, 1 H), 7.57 (s, 1 H), 8.16 (dd, 1 H), 8.30 (s, 1 H), 8.37 (t, 1 H), 8.45 (d, 1 H), 8.72 (s, 1 H), 12.36 (s, 1 H).

### Example 3

### N-[6-(2-Hydroxypropan-2-yl)-2-(3-methoxypropyl)-2H-indazol-5-yl]-6-(trifluoromethyl)pyridine-2-carboxamide

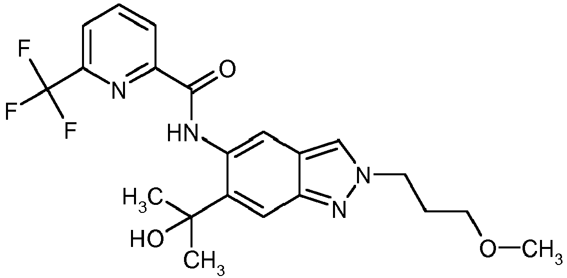

75 mg (0.17 mmol) of methyl 2-(3-methoxypropyl)-5-({[6-(trifluoromethyl)pyridin-2-yl]carbonyl}amino)-2H-indazole-6-carboxylate (Intermediate 4-3) were dissolved in 500 µl of THF and admixed with 859 µl (0.86 mmol) of a 1 M methylmagnesium bromide solution in THF. The reaction mixture was stirred at 25°C for 60 min. Subsequently, 1 ml of a saturated ammonium chloride solution was added cautiously and the mixture was filtered. The aqueous phase was extracted twice with ethyl acetate, and the organic phases were combined, filtered through a hydrophobic filter and concentrated. The residue was dissolved in 3 ml of DMSO and purified by preparative HPLC. The product-containing fractions were freeze-dried. This gave 25 mg of the title compound.
UPLC-MS (Method A1): Rₜ = 1.13 min
MS (ESlpos): m/z = 437 (M+H)⁺
¹H NMR (400 MHz, DMSO-d6): δ [ppm] = 1.62 (s, 6 H), 2.14 (quin, 2 H), 3.23 (s, 3 H), 3.26 - 3.32 (m, 2 H), 4.44 (t, 2 H), 5.95 (s, 1 H), 7.58 (s, 1 H), 8.16 (d, 1 H), 8.31 - 8.40 (m, 2 H), 8.43 - 8.48 (m, 1 H), 8.72 (s, 1 H), 12.36 (s, 1 H).

### Example 4

### N-[6-(Hydroxymethyl)-2-(3-methoxypropyl)-2H-indazol-5-yl]-6-(trifluoromethyl)pyridine-2-carboxamide

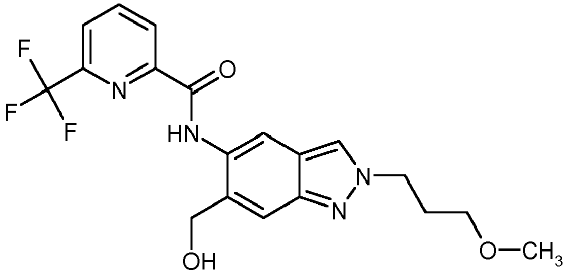

13 mg of lithium aluminium hydride were suspended in THF and the mixture was cooled to 0°C. 75 mg (0.17 mmol) of methyl 2-(3-methoxypropyl)-5-({[6-(trifluoromethyl)pyridin-2-yl]carbonyl}amino)-2H-indazole-6-carboxylate (Intermediate 4-3) in THF were added dropwise and the mixture was allowed to come to room temperature within 30 min. The mixture was diluted with water and filtered, the residue was washed with ethyl acetate and the filtrate was extracted with ethyl acetate. The combined ethyl acetate phases were washed with sodium chloride solution, filtered through a hydrophobic filter and concentrated. The residue was purified by preparative HPLC.
¹H NMR (300 MHz, DMSO-*d₆*): δ [ppm] = 2.14 (quin, 2 H), 3.23 (s, 3 H), 3.29 (t, 2 H), 4.45 (t, *J*=7.0 Hz, 2 H), 4.68 (d, 2 H), 5.77 (t, 1 H), 7.58 (s, 1 H), 8.18 (d, 1 H), 8.32 - 8.48 (m, 3 H), 8.51 (s, 1 H), 11.21 (s, 1 H).

### Example 5

### N-[2-(2-Hydroxyethyl)-6-(2-hydroxypropan-2-yl)-2H-indazol-5-yl]-6-(trifluoromethyl)pyridine-2-carboxamide

### Stage A:

### Preparation of N-[2-(2-{[tert-butyl(dimethyl)silyl]oxy}ethyl)-6-(2-hydroxypropan-2-yl)-2H-indazol-5-yl]-6-(trifluoromethyl)pyridine-2-carboxamide

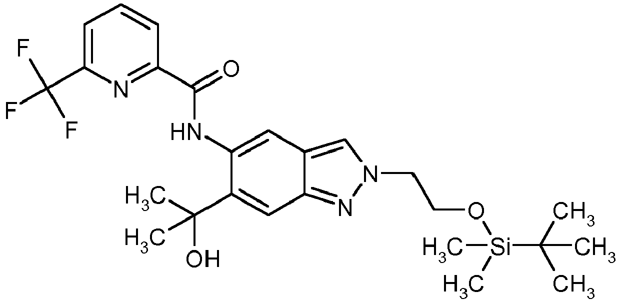

100 mg (0.19 mmol) of methyl 2-(2-{[tert-butyl(dimethyl)silyl]oxy}ethyl)-5-({[6-(trifluoromethyl)pyridin-2-yl]carbonyl}amino)-2H-indazole-6-carboxylate (Intermediate 4-5) were dissolved in 1 ml of THF and admixed with 669 µl (0.67 mmol) of a 1 M methylmagnesium bromide solution in THF. The reaction mixture was stirred at 25°C for 60 min. Another 287 µl (0.29 mmol) of a 1 M methylmagnesium bromide solution in THF were added and the mixture was stirred at 25°C for 3 h. Subsequently, 20 ml of a saturated ammonium chloride solution were added cautiously and the mixture was filtered. The aqueous phase was extracted twice with ethyl acetate, and the organic phases were combined, dried over magnesium sulphate, filtered, concentrated and dried under reduced pressure. This gave 50 mg of N-[2-(2-{[tert-butyl(dimethyl)silyl]oxy}ethyl)-6-(2-hydroxypropan-2-yl)-2H-indazol-5-yl]-6-(trifluoromethyl)pyridine-2-carboxamide.
UPLC-MS (Method A2): Rₜ = 1.51 min
MS (ESlpos): m/z = 523(M+H)⁺
¹H NMR (300 MHz, DMSO-d6): δ [ppm] = -0.17 - -0.09 (m, 6 H), 0.78 (s, 9 H), 1.62 (s, 6 H), 4.04 (t, 2H), 4.47 (t, 2 H), 5.98 (s, 1 H), 7.57 (s, 1 H), 8.16 (d, 1 H), 8.29 (s, 1 H), 8.37 (t, 1H), 8.45 (d, 1 H), 8.73 (s, 1 H), 12.38 (s, 1 H).

### Stage B:

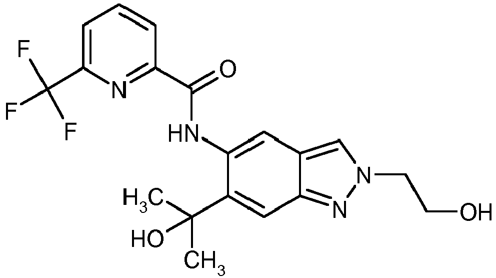

50 mg (96 µmol) of N-[2-(2-{[tert-butyl(dimethyl)silyl]oxy}ethyl)-6-(hydroxymethyl)-2H-indazol-5-yl]-6-(trifluoromethyl)pyridine-2-carboxamide were dissolved in 1.0 ml of THF and admixed with 144 µl (0.14 mmol) of a 1 M solution of tetrabutylammonium fluoride in THF. The reaction mixture was stirred at room temperature for 1 h. The mixture was diluted with water and extracted twice with ethyl acetate, and the combined organic phases were washed with saturated sodium chloride solution, filtered through a hydrophobic filter and concentrated. This gave 36 mg of N-[2-(2-hydroxyethyl)-6-(2-hydroxypropan-2-yl)-2H-indazol-5-yl]-6-(trifluoromethyl)pyridine-2-carboxamide (Example 5).
¹H-NMR (400MHz, DMSO-d₆): d [ppm]= 1.62 (s, 6H), 3.86 (q, 2H), 4.43 (t, 2H), 4.95 (t, 1H), 5.94 (s, 1H), 7.57 (s, 1H), 8.16 (dd, 1H), 8.30 (s, 1H), 8.37 (t, 1H), 8.45 (d, 1H), 8.72 (s, 1H), 12.36 (s, 1H).
UPLC-MS (Method A2): Rₜ = 0.97 min (UV detector: TIC), mass found 408.00.

### Example 6

### N-[6-(2-Hydroxypropan-2-yl)-2-(3-hydroxypropyl)-2H-indazol-5-yl]-6-(trifluoromethyl)pyridine-2-carboxamide

### Stage A:

### Preparation of N-[2-(3-{[tert-butyl(dimethyl)silyl]oxy}propyl)-6-(2-hydroxypropan-2-yl)-2H-indazol-5-yl]-6-(trifluoromethyl)pyridine-2-carboxamide

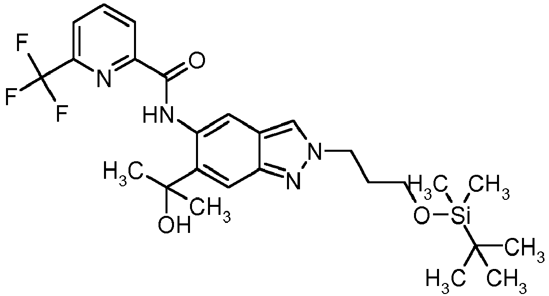

50 mg (0.09 mmol) of methyl 2-(3-{[tert-butyl(dimethyl)silyl]oxy}propyl)-5-({[6-(trifluoromethyl)pyridin-2-yl]carbonyl}amino)-2H-indazole-6-carboxylate (Intermediate 4-6) were dissolved in 500 ml of THF and admixed with 326 µl (0.33 mmol) of a 1 M methylmagnesium bromide solution in THF. The reaction mixture was stirred at 25°C for 60 min. Subsequently, 20 ml of a saturated ammonium chloride solution were added cautiously and the mixture was extracted twice with ethyl acetate. The combined organic phases were filtered through a hydrophobic filter, concentrated and dried under reduced pressure. The residue was purified by preparative HPLC. 40 mg of N-[2-(3-{[tert-butyl(dimethyl)silyl]oxy}propyl)-6-(2-hydroxypropan-2-yl)-2H-indazol-5-yl]-6-(trifluoromethyl)pyridine-2-carboxamide were obtained.
UPLC-MS (Method A1): Rₜ = 1.58 min
MS (ESlpos): m/z = 537(M+H)⁺
¹H NMR (300 MHz, DMSO-d6): δ [ppm] = 0.02 - 0.05 (m, 6 H), 0.84 - 0.91 (m, 9 H), 1.62 (s, 6 H), 2.02 - 2.18 (m, 2 H), 3.55 - 3.62 (m, 2 H), 4.45 (t, 2 H), 5.96 (s, 1 H), 7.57 (s, 1 H), 8.16 (d, 1 H), 8.31 (s, 1 H), 8.33 - 8.42 (m, 1 H), 8.45 (d, 1 H), 8.72 (s, 1 H), 12.37 (s, 1 H).

### Stage B:

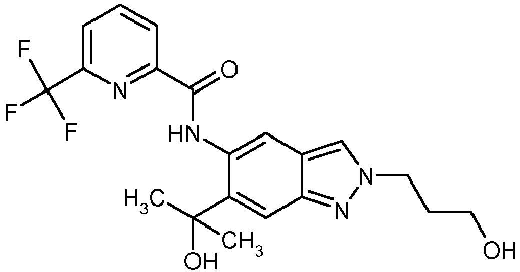

37 mg (0.07 mmol) of N-[2-(3-{[tert-butyl(dimethyl)silyl]oxy}propyl)-6-(2-hydroxypropan-2-yl)-2H-indazol-5-yl]-6-(trifluoromethyl)pyridine-2-carboxamide were dissolved in 500 µl of THF and admixed with 207 µl (0.21 mmol) of a 1 M solution of tetrabutylammonium fluoride in THF. The reaction mixture was stirred at 25°C for 2 h. The mixture was diluted with water and extracted twice with ethyl acetate, and the combined organic phases were washed with saturated sodium chloride solution, filtered and concentrated. After purification by preparative HPLC, 10 mg of N-[6-(2-hydroxypropan-2-yl)-2-(3-hydroxypropyl)-2H-indazol-5-yl]-6-(trifluoromethyl)pyridine-2-carboxamide (Example 6) were obtained.
UPLC-MS (Method A2): Rₜ = 1.00 min
MS (ESlpos): m/z = 423 (M+H)⁺
¹H NMR (400 MHz, DMSO-d6): δ [ppm] = 1.62 (s, 6 H), 2.00 - 2.07 (m, 2 H), 3.07 - 3.22 (m, 1 H), 3.39 (t, 2 H), 4.45 (t, 2 H), 4.63 (br. s., 1 H), 5.94 (br. s., 1 H), 7.56 (s, 1 H), 8.14 (d, 1 H), 8.28 - 8.39 (m, 2 H), 8.41 - 8.47 (m, 1 H), 8.72 (s, 1 H), 12.31 (br. s., 1 H).

### Example 7

### N-[2-(2-Hydroxyethyl)-6-(hydroxymethyl)-2H-indazol-5-yl]-6-(trifluoromethyl)pyridine-2-carboxamide

### Stage A:

### N-[2-(2-{[tert-Butyl(dimethyl)silyl]oxy}ethyl)-6-(hydroxymethyl)-2H-indazol-5-yl]-6-(trifluoromethyl)pyridine-2-carboxamide

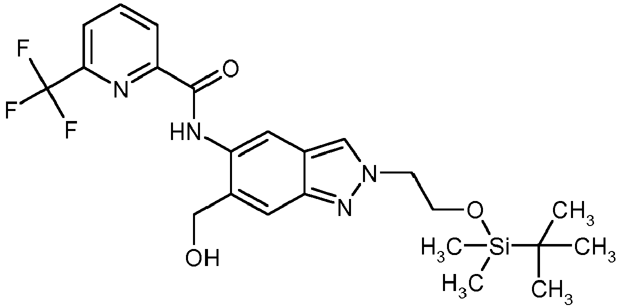

100 mg (0.19 mmol) of methyl 2-(2-{[tert-butyl(dimethyl)silyl]oxy}ethyl)-5-({[6-(trifluoromethyl)pyridin-2-yl]carbonyl}amino)-2H-indazole-6-carboxylate (Intermediate 4-5) were dissolved in 1 ml of THF and admixed with 191 µl (0.38 mmol) of a 2 M lithium borohydride solution. The mixture was left to stir at 25°C for 24 h. 14 mg (0.38 mmol) of sodium borohydride and 500 µl of methanol were added, and the mixture was stirred at 25°C for 4 h. Another 14 mg (0.38 mmol) of sodium borohydride were added, and the mixture was stirred at 25°C for 24 h. Water was added cautiously and the mixture was concentrated. The mixture was then extracted twice with ethyl acetate, and the combined organic phases were washed with saturated sodium chloride solution, filtered through a hydrophobic filter and concentrated. The residue was taken up in 2 ml of DMSO and purified by preparative HPLC. This gave 30 mg of N-[2-(2-{[tert-butyl(dimethyl)silyl]oxy}ethyl)-6-(hydroxymethyl)-2H-indazol-5-yl]-6-(trifluoromethyl)pyridine-2-carboxamide.
UPLC-MS (Method A2): Rₜ = 1.44 min
MS (ESlpos): m/z = 495(M+H)⁺
¹H NMR (300 MHz, DMSO-d6): δ [ppm] = -0.16 - -0.12 (m, 6 H), 0.75 - 0.79 (m, 9 H), 4.05 (t, 2 H), 4.48 (t, 2 H), 4.69 (d, 2 H), 5.75 - 5.77 (m, 1 H), 7.57 (s, 1 H), 8.18 (dd, 1 H), 8.30 - 8.33 (m, 1 H), 8.38 (t, 1 H), 8.45 (d, 1 H), 8.51 (s, 1 H), 11.20 (s, 1 H).

### Stage B:

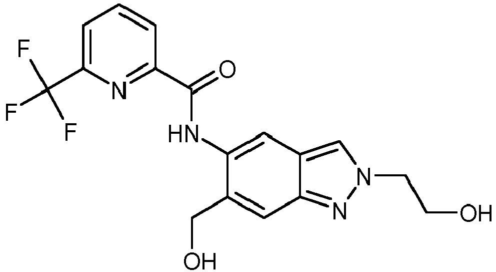

33 mg (0.07 mmol) of N-[2-(2-{[tert-butyl(dimethyl)silyl]oxy}ethyl)-6-(hydroxymethyl)-2H-indazol-5-yl]-6-(trifluoromethyl)pyridine-2-carboxamide were dissolved in 1 ml of THF and admixed with 100 µl (0.10 mmol) of a 1 M solution of tetrabutylammonium fluoride in THF. The reaction mixture was stirred at 25°C for 1 h. The mixture was diluted with water and extracted twice with ethyl acetate, and the combined organic phases were washed with saturated sodium chloride solution, filtered through a hydrophobic filter, concentrated and dried under reduced pressure. 25 mg of N-[2-(2-hydroxyethyl)-6-(hydroxymethyl)-2H-indazol-5-yl]-6-(trifluoromethyl)pyridine-2-carboxamide (Example 7) were obtained.
UPLC-MS (Method A2): Rₜ = 0.87 min
MS (ESlpos): m/z = 381 (M+H)⁺
¹H NMR (300 MHz, DMSO-d6): δ [ppm] = 3.87 (q, 2 H), 4.44 (t, 2 H), 4.69 (d, 2 H), 4.98 (t, 1 H), 5.70 - 5.81 (m, 1 H), 7.57 (s, 1 H), 8.11 - 8.23 (m, 1 H), 8.31 - 8.42 (m, 2 H), 8.43 - 8.49 (m, 1 H), 8.51 (s, 1 H), 11.20 (s, 1 H).

### Example 8

### N-[6-(2-Hydroxypropan-2-yl)-2-(oxetan-3-ylmethyl)-2H-indazol-5-yl]-6-(trifluoromethyl)pyridine-2-carboxamide

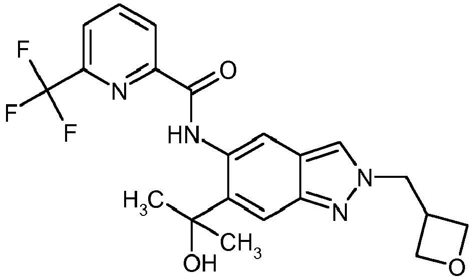

50 mg (0.12 mmol) of methyl 2-(oxetan-3-ylmethyl)-5-({[6-(trifluoromethyl)pyridin-2-yl]carbonyl}amino)-2H-indazole-6-carboxylate (Intermediate 4-1) were dissolved in 500 µl of THF and admixed with 576 µl (0.58 mmol) of a 1 M methylmagnesium bromide solution in THF. The reaction mixture was stirred at 25°C for 60 min. Subsequently, 20 ml of a saturated aqueous ammonium chloride solution were added cautiously and the mixture was concentrated. The aqueous phase was extracted twice with ethyl acetate, and the organic phases were combined, dried over magnesium sulphate, filtered and concentrated. The residue was dissolved in 2.0 ml of DMSO and purified by preparative HPLC. The product-containing fractions were freeze-dried. This gave 30 mg of the title compound.
UPLC-MS (Method A2): Rₜ = 1.03 min
MS (ESlpos): m/z = 435 (M+H)⁺
¹H NMR (400 MHz, DMSO-d6): δ [ppm] = 1.62 (s, 6 H), 3.45 - 3.61 (m, 1 H), 4.48 (t, 2 H), 4.66 (dd, 2 H), 4.72 (d, 2 H), 5.94 (s, 1 H), 7.57 (s, 1 H), 8.16 (d, 1 H), 8.33 - 8.42 (m, 2 H), 8.42 - 8.47 (m, 1 H), 8.72 (s, 1 H), 12.36 (s, 1 H).

### Example 9

### N-[6-(Hydroxymethyl)-2-(oxetan-3-ylmethyl)-2H-indazol-5-yl]-6-(trifluoromethyl)pyridine-2-carboxamide

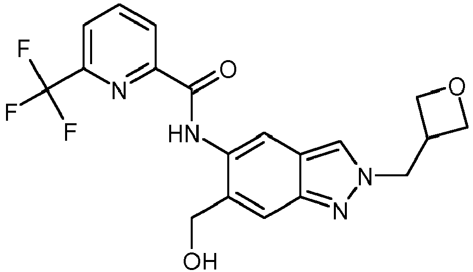

75 mg (0.17 mmol) of methyl 2-(oxetan-3-ylmethyl)-5-({[6-(trifluoromethyl)pyridin-2-yl]carbonyl}amino)-2H-indazole-6-carboxylate (Intermediate 4-1) were dissolved in 1 ml of a mixture of THF/methanol (1:1), and 8 mg (0.21 mmol) of sodium borohydride were added. The mixture was left to stir at 25°C for 60 min. The reaction mixture was concentrated, and the residue was admixed with water. The suspension was stirred vigorously for 15 min, and the solids were filtered off with suction, washed twice with water and twice with diethyl ether, and dried under reduced pressure. This gave 48 mg of the title compound.
UPLC-MS (Method A2): Rₜ = 0.94 min
MS (ESlpos): m/z = 407 (M+H)⁺
¹H NMR (300 MHz, DMSO-d6): δ [ppm] = 3.55 (s, 1 H), 4.48 (t, 2 H), 4.61 - 4.77 (m, 6 H), 7.57 (s, 1 H), 8.18 (dd, 1 H), 8.33 - 8.49 (m, 3 H), 8.51 (s, 1 H), 11.21 (s, 1 H).

### Example 10

### N-{6-(2-Hydroxypropan-2-yl)-2-[3-(methylsulphonyl)propyl]-2H-indazol-5-yl}-6-(trifluoromethyl)pyridine-2-carboxamide

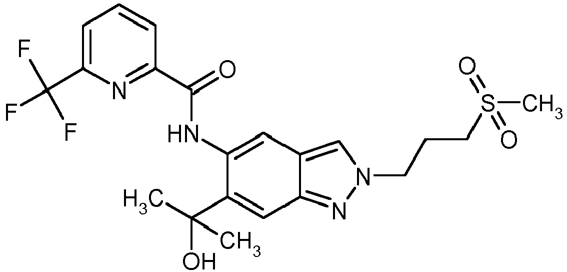

A mixture of 500 mg (1.32 mmol) of N-[6-(2-hydroxypropan-2-yl)-1H-indazol-5-yl]-6-(trifluoromethyl)pyridine-2-carboxamide (Intermediate 5-1), 569 mg of potassium carbonate and 114 mg of potassium iodide in 5.0 ml of DMF was stirred at room temperature for 15 min. 414 mg of 1-bromo-3-(methylsulphonyl)propane were added and the mixture was stirred at room temperature overnight. Water was added, the mixture was twice extracted with ethyl acetate and the extracts were washed with sodium chloride solution and concentrated. The residue was purified by column chromatography (dichloromethane/methanol gradient). Extracting the product fraction by stirring with diethyl ether gave 59 mg of the title compound.
UPLC-MS (Method A2): Rₜ = 1.02 min
MS (ESlpos): m/z = 485 (M+H)+
¹H-NMR (300MHz, DMSO-d₆): δ [ppm]= 1.63 (s, 6H), 2.26 - 2.42 (m, 2H), 2.99 (s, 3H), 3.06 - 3.16 (m, 2H), 4.55 (t, 2H), 5.96 (s, 1H), 7.60 (s, 1H), 8.16 (d, 1H), 8.33 - 8.48 (m, 3H), 8.73 (s, 1H), 12.37 (s, 1H).

### Example 11

### N-[2-(3-Hydroxy-3-methylbutyl)-6-(2-hydroxypropan-2-yl)-2H-indazol-5-yl]-6-(trifluoromethyl)pyridine-2-carboxamide

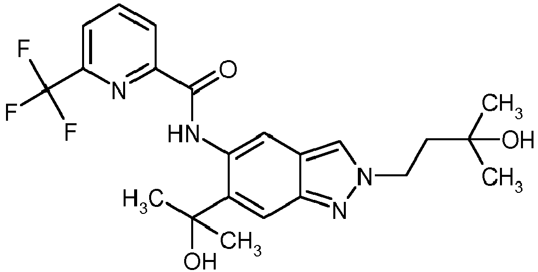

### Preparation Method 1

705 mg (1.57 mmol) of methyl 2-(3-hydroxy-3-methylbutyl)-5-({[6-(trifluoromethyl)pyridin-2-yl]carbonyl}amino)-2H-indazole-6-carboxylate (Intermediate 4-4) were initially charged in 10 ml of THF and cooled in an ice-water cooling bath. 2.6 ml (5.0 equivalents) of 3M methylmagnesium bromide solution (in diethyl ether) were added and the mixture was left to stir while cooling with an ice bath for 1 h and at room temperature for 4.5 h. Another 1 equivalent of the methylmagnesium bromide solution was added and the mixture was left to stir at room temperature for 20.5 h. Another 1 equivalent again of the methylmagnesium bromide solution was added and the mixture was left to stir at room temperature for 22 h. The reaction mixture was admixed with saturated aqueous ammonium chloride solution, stirred and extracted three times with ethyl acetate. The combined organic phases were washed with sodium chloride solution, filtered through a hydrophobic filter and concentrated. This gave 790 mg of a residue which was purified by means of preparative HPLC. This gave 234 mg of the title compound and 164 mg of a product fraction which was extracted by stirring with diethyl ether. After filtration with suction followed by drying, a further 146 mg of the title compound were obtained.
UPLC-MS (Method A1): Rₜ = 1.10 min (UV detector: TIC), mass found 450.00.
¹H-NMR (400MHz, DMSO-d₆): δ [ppm]= 1.14 (s, 6H), 1.61 (s, 6H), 1.99 - 2.08 (m, 2H), 4.42 - 4.55 (m, 3H), 5.93 (s, 1H), 7.56 (s, 1H), 8.15 (dd, 1H), 8.32 - 8.39 (m, 2H), 8.41 - 8.47 (m, 1H), 8.70 (s, 1H), 12.34 (s, 1H).

### Preparation Method 2

A mixture of 500 mg (1.37 mmol) of N-[6-(2-hydroxypropan-2-yl)-1H-indazol-5-yl]-6-(trifluoromethyl)pyridine-2-carboxamide (Intermediate 5-1), 569 mg of potassium carbonate and 114 mg of potassium iodide in 5 ml of DMF was stirred at room temperature for 15 min. 344 mg (1.5 equivalents) of 4-bromo-2-methylbutan-2-ol were added and the mixture was heated to 100°C for 2 h. Another 0.5 equivalent of 4-bromo-2-methylbutan-2-ol was added and the mixture was stirred at room temperature overnight. The mixture was admixed with water and extracted twice with ethyl acetate, and the combined organic phases were washed with saturated sodium chloride solution and filtered through a hydrophobic filter and concentrated. The residue was purified by column chromatography purification on silica gel (hexane/ethyl acetate). This gave 100 mg of a product fraction which was extracted by stirring with diethyl ether. After drying, 60 mg of the title compound were obtained.
¹H-NMR (400MHz, DMSO-d₆): δ [ppm]= 1.14 (s, 6 H), 1.61 (s, 6H), 1.99 - 2.07 (m, 2 H), 4.43 - 4.52 (m, 3 H) 5.94 (s, 1 H) 7.57 (s, 1 H) 8.15 (dd, 1H) 8.33 - 8.40 (m, 2 H), 8.42 - 8.48 (m, 1 H), 8.71 (s, 1 H), 12.35 (s, 1 H).

### Example 12

### N-{6-(2-Hydroxypropan-2-yl)-2-[2-(methylsulphonyl)ethyl]-2H-indazol-5-yl}-6-(trifluoromethyl)pyridine-2-carboxamide

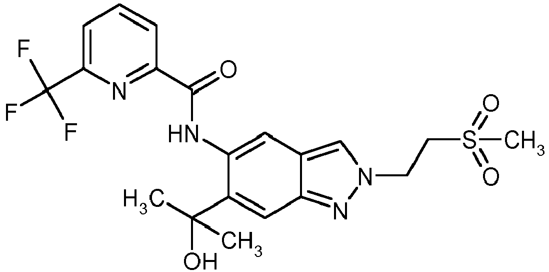

160 mg (0.44 mmol) of N-[6-(2-hydroxypropan-2-yl)-1H-indazol-5-yl]-6-(trifluoromethyl)pyridine-2-carboxamide (Intermediate 5-1) were suspended together with 182 mg of potassium carbonate and 36 mg of potassium iodide in 1.0 ml of DMF, and the mixture was stirred at room temperature for 15 min. Then 123 mg of 2-bromoethyl methyl sulphone were added and the mixture was stirred at room temperature overnight. Water was added, the mixture was extracted twice with ethyl acetate and the extracts were washed with saturated aqueous sodium chloride solution, filtered through a hydrophobic filter and concentrated. Purification of the residue by preparative HPLC gave 20 mg of the title compound.
UPLC (Method A2): Rₜ = 1.01 min;
MS (ESlpos): m/z = 471 (M+H)+
¹H NMR (400 MHz, DMSO-*d₆*): δ [ppm]= 1.63 (s, 6 H), 2.90 (s, 3 H), 3.85 (t, 2 H), 4.86 (t, 2 H), 5.97 (s, 1 H), 7.59 (s, 1 H), 8.13 - 8.19 (m, 1 H), 8.37 (s, 1 H), 8.41 - 8.48 (m, 2 H), 8.74 (s, 1 H), 12.37 (s, 1 H).

### Example 13

### 6-(Difluoromethyl)-N-[2-(3-hydroxy-3-methylbutyl)-6-(2-hydroxypropan-2-yl)-2H-indazol-5-yl]pyridine-2-carboxamide

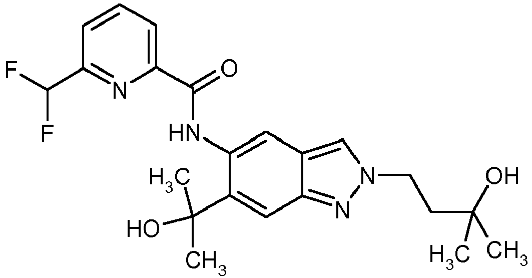

A mixture of 250 mg of 6-(difluoromethyl)-N-[6-(2-hydroxypropan-2-yl)-1H-indazol-5-yl]pyridine-2-carboxamide (crude product of Intermediate 5-2), 144 mg of potassium iodide and 239 mg of potassium carbonate in 2.5 ml of DMF was stirred at room temperature for 15 min. 145 mg (0.87 mmol) of 4-bromo-2-methylbutan-2-ol were added, the mixture was stirred at 110°C for 3 h, another 96 mg of 4-bromo-2-methylbutan-2-ol were added and the mixture was stirred at 110°C for 4 h. Water was added, the mixture was extracted twice with ethyl acetate and the extracts were washed with saturated aqueous sodium chloride solution, filtered through a hydrophobic filter and concentrated. Purification was effected by column chromatography on silica gel (hexane/ethyl acetate). This gave 61 mg of the title compound. UPLC-MS (Method A1): Rₜ = 1.00 min (UV detector: TIC), mass found 432.00.
¹H-NMR (300MHz, DMSO-d₆): δ [ppm]= 1.14 (s, 6H), 1.63 (s, 6H), 1.97 - 2.08 (m, 2H), 4.41 - 4.55 (m, 3H), 5.99 (s, 1H), 7.03 (t, 1H), 7.56 (s, 1H), 7.94 - 8.00 (m, 1H), 8.24 - 8.38 (m, 3H), 8.71 (s, 1H), 12.49 (s, 1H).

### Example 14

### 6-(Difluoromethyl)-N-{6-(2-hydroxypropan-2-yl)-2-[2-(methylsulphonyl)ethyl]-2H-indazol-5-yl}pyridine-2-carboxamide

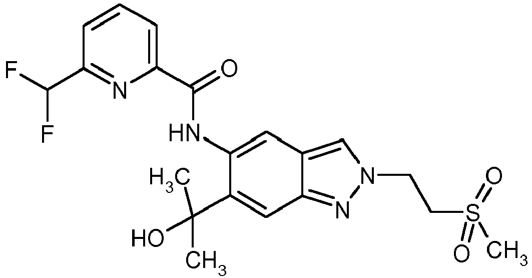

A mixture of 250 mg of 6-(difluoromethyl)-N-[6-(2-hydroxypropan-2-yl)-1H-indazol-5-yl]pyridine-2-carboxamide (crude product of Intermediate 5-2), 144 mg of potassium iodide and 239 mg of potassium carbonate in 2.5 ml of DMF was stirred at room temperature for 15 min. 162 mg of 2-bromoethyl methyl sulphone were added and the mixture was stirred at 110°C for 3 h. Water was added, the mixture was extracted twice with ethyl acetate and the extracts were washed with saturated aqueous sodium chloride solution, filtered through a hydrophobic filter and concentrated. The residue was purified by preparative HPLC and the product fractions were additionally purified by column chromatography purification on silica gel (hexane/ethyl acetate). This gave 40 mg of the title compound.
¹H-NMR (400MHz, DMSO-d₆): δ [ppm]= 1.65 (s, 6H), 2.90 (s, 3H), 3.85 (t, 2H), 4.85 (t, 2H), 6.03 (s, 1H), 7.04 (t, 1H), 7.59 (s, 1H), 7.98 (d, 1H), 8.25 - 8.36 (m, 2H), 8.43 (s, 1H), 8.75 (s, 1H), 12.52 (s, 1H).

### Example 15

### 6-(Difluoromethyl)-N-[6-(2-hydroxypropan-2-yl)-2-(3-hydroxypropyl)-2H-indazol-5-yl]pyridine-2-carboxamide

### Stage A:

### Preparation of N-[2-(3-{[tert-butyl(dimethyl)silyl]oxy}propyl)-6-(2-hydroxypropan-2-yl)-2H-indazol-5-yl]-6-(difluoromethyl)pyridine-2-carboxamide

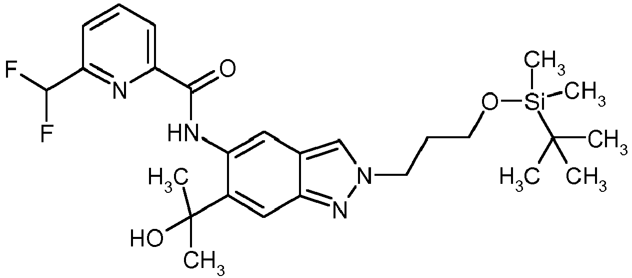

A mixture of 250 mg of 6-(difluoromethyl)-N-[6-(2-hydroxypropan-2-yl)-1H-indazol-5-yl]pyridine-2-carboxamide (Intermediate 5-2), 48 mg of potassium iodide and 239 mg of potassium carbonate in 2.5 ml of DMF was stirred at room temperature for 15 min. 219 mg (0.87 mmol, 1.5 equivalents) of (3-bromopropoxy)(tert-butyl)dimethylsilane were added and the mixture was stirred at 110°C for 3 h. 1 equivalent of (3-bromopropoxy)(tert-butyl)dimethylsilane was added and the mixture was stirred at 100°C for 4 h. Water was added, the mixture was extracted with ethyl acetate and the extract was washed with aqueous sodium chloride solution, filtered through a hydrophobic filter and concentrated. The residue was purified by column chromatography (hexane/ethyl acetate). This gave 92 mg of the title compound.

### Stage B:

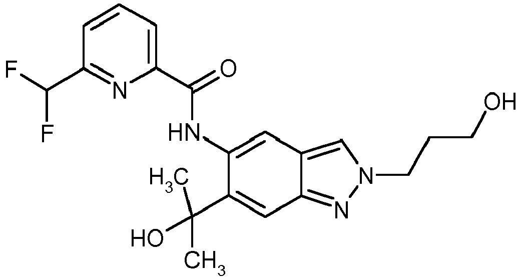

Analogously to the preparation of Example 6, Stage B, 92 mg of N-[2-(3-{[tert-butyl(dimethyl)silyl]oxy}propyl)-6-(2-hydroxypropan-2-yl)-2H-indazol-5-yl]-6-(difluoromethyl)pyridine-2-carboxamide were reacted with 0.53 ml of a 1 M solution of tetrabutylammonium fluoride in THF within 1 h. Aqueous workup as in Example 6 and purification by preparative HPLC gave 46 mg of the title compound. UPLC-MS (Method A1): Rₜ = 0.92 min (UV detector: TIC), mass found 404.00.
¹H-NMR (400MHz, DMSO-d₆): δ [ppm]= 1.64 (s, 6H), 2.05 (quin, 2H), 3.35 - 3.46 (m), 4.45 (t, 2H), 4.64 (t, 1H), 5.99 (s, 1H), 7.04 (t, 1H), 7.57 (s, 1H), 7.95 - 7.99 (m, 1H), 8.25 - 8.36 (m, 3H), 8.73 (s, 1H), 12.50 (s, 1H).

### Example 16

### N-[6-(2-Hydroxypropan-2-yl)-2-(4,4,4-trifluorobutyl)-2H-indazol-5-yl]-6-(trifluoromethyl)pyridine-2-carboxamide

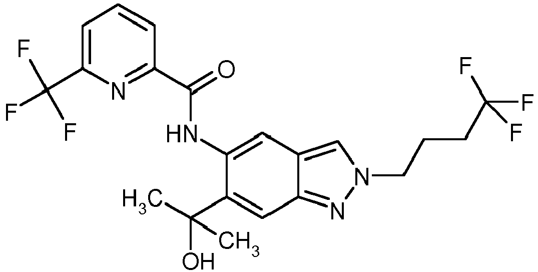

A mixture of 210 mg (0.58 mmol) of N-[6-(2-hydroxypropan-2-yl)-1H-indazol-5-yl]-6-(trifluoromethyl)pyridine-2-carboxamide (Intermediate 5-1) in 3 ml of DMF was admixed with 0.11 ml of 1,1,1-trifluoro-4-iodobutane and 239 mg of potassium carbonate, and the mixture was stirred at 80°C for 6 h. After addition of water, the mixture was extracted three times with ethyl acetate, and the combined organic phases were washed with saturated sodium chloride solution, filtered through a hydrophobic filter and concentrated. The crude product was purified by preparative HPLC. This gave 19 mg of the title compound.
UPLC-MS (Method A1): Rₜ = 1.27 min (UV detector: TIC), mass found 474.15.
¹H-NMR (400MHz, DMSO-d₆): δ [ppm]= 1.62 (s, 6H), 2.10 - 2.33 (m), 4.49 (t, 2H), 5.94 (s, 1H), 7.59 (s, 1H), 8.13 - 8.18 (m, 1H), 8.32 - 8.41 (m, 2H), 8.41 - 8.47 (m, 1H), 8.72 (s, 1H), 12.35 (s, 1H).

### Example 17

### N-{6-(2-Hydroxypropan-2-yl)-2-[3-(trifluoromethoxy)propyl]-2H-indazol-5-yl}-6-(trifluoromethyl)pyridine-2-carboxamide

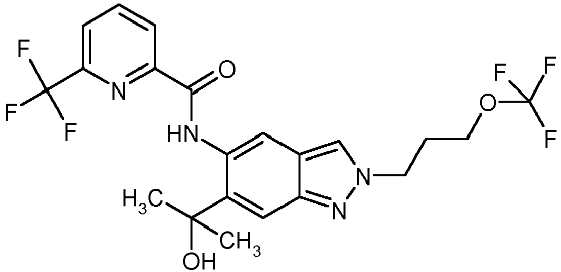

150 mg of N-[6-(2-hydroxypropan-2-yl)-1H-indazol-5-yl]-6-(trifluoromethyl)pyridine-2-carboxamide (Intermediate 5-1) were initially charged in 2 ml of THF. 58 mg of 3-(trifluoromethoxy)propan-1-ol, 131 mg of triphenylphosphine and 71 µl of diisopropyl azodicarboxylate (DIAD, CAS 2446-83-5) were added and the mixture was stirred at room temperature for 19 h. 0.83 ml of sodium hydroxide solution (2M) was added and the mixture was stirred at 40°C for 5 h. The mixture was diluted with water and extracted three times with ethyl acetate, and the combined organic phases were concentrated and purified by preparative HPLC. 16 mg of the title compound were obtained as a crude product.
UPLC-MS (Method A2): Rₜ = 1.26 min (UV detector: TIC), mass found 490.14.
¹H-NMR (400MHz, DMSO-d₆, selected signals): δ [ppm]= 1.61 (s, 6H), 1.84 (d, 1H), 2.32 (quint., 2H), 4.08 (t, 2H), 4.51 (t, 2H), 7.58 (s, 1H), 8.15 (d, 1H), 8.31 - 8.39 (m, 2H), 8.44 (d, 1H), 8.72 (s, 1H), 12.35 (s, 1H).

### Example 18

### N-{6-(2-Hydroxypropan-2-yl)-2-[3-(2,2,2-trifluoroethoxy)propyl]-2H-indazol-5-yl}-6-(trifluoromethyl)pyridine-2-carboxamide

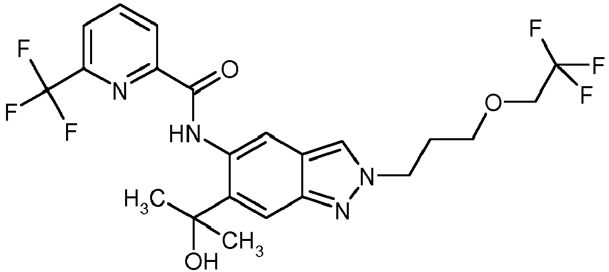

Analogously to the preparation of Example 11 (Preparation Method 1), 52 mg of methyl 2-[3-(2,2,2-trifluoroethoxy)propyl]-5-({[6-(trifluoromethyl)pyridin-2-yl]carbonyl}amino)-2H-indazole-6-carboxylate (Intermediate 4-10) in 3 ml of THF were reacted with 2 x 171 microlitres of 3M magnesium bromide solution in diethyl ether. Purification by preparative HPLC gave 12 mg of the title compound.
UPLC-MS (Method A1): Rₜ = 1.25 min (UV detector: TIC), mass found 504.16.
¹H-NMR (500 MHz, DMSO-d₆): δ [ppm]= 1.63 (s, 6H), 2.20(quin, 2H), 3.58(t, 2H),4.05(q, 2H), 4.47(t, 2H),5.94(s, 1H), 7.58 (s, 1H), 8.15 (dd, 1H), 8.32 (s, 1H), 8.36 (t, 1H), 8.45(d, 1H), 8.73 (s, 1H), 12.36 (s,1H).

### Example 19

### 5-Fluoro-N-[2-(3-hydroxy-3-methylbutyl)-6-(2-hydroxypropan-2-yl)-2H-indazol-5-yl]-6-methylpyridine-2-carboxamide

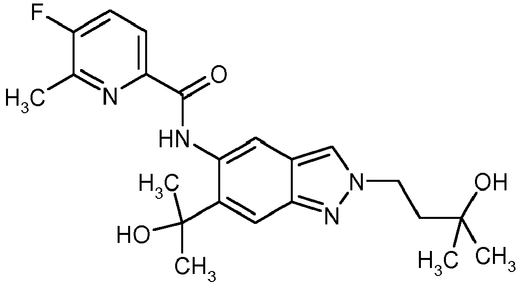

228 mg of methyl 5-{[(5-fluoro-6-methylpyridin-2-yl)carbonyl]amino}-2-(3-hydroxy-3-methylbutyl)-2H-indazole-6-carboxylate (Intermediate 4-8) were initially charged in 4.5 ml of THF and cooled with an ice cooling bath. 0.63 ml of 3M methylmagnesium bromide solution (in diethyl ether) was added and the mixture was left to stir while cooling with an ice bath for 2 h and at room temperature for 21 h. The reaction mixture was admixed with saturated aqueous ammonium chloride solution and extracted three times with ethyl acetate. The combined organic phases were concentrated. The residue was purified by preparative HPLC. This gave 82 mg of the title compound.
UPLC-MS (Method A2): Rₜ = 1.03 min (UV detector: TIC), mass found 414.21.
¹H-NMR (400MHz, DMSO-d₆): δ [ppm]= 1.13 (s, 6H), 1.63 (s, 6H), 1.99 - 2.05 (m, 2H), 2.55 - 2.59 (m, 3H), 4.42 - 4.50 (m, 3H), 5.95 (s, 1H), 7.54 (s, 1H), 7.83 (t, 1H), 8.05 (dd, 1H), 8.31 (s, 1H), 8.68 (s, 1H), 12.33 (s, 1H).

### Example 20

### N-[2-(3-Hydroxy-3-methylbutyl)-6-(2-hydroxypropan-2-yl)-2H-indazol-5-yl]-6-methylpyridine-2-carboxamide

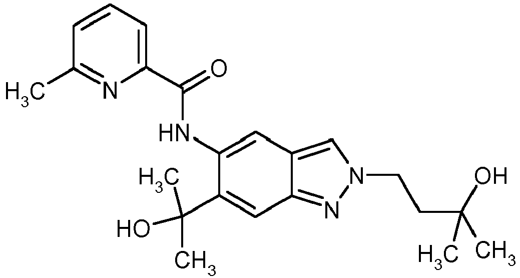

278 mg of methyl 2-(3-hydroxy-3-methylbutyl)-5-{[(6-methylpyridin-2-yl)carbonyl]amino}-2H-indazole-6-carboxylate (Intermediate 4-9) were initially charged in 5.0 ml of THF and cooled with an ice cooling bath. 0.97 ml of 3M methylmagnesium bromide solution (in diethyl ether) was added and the mixture was left to stir while cooling with an ice bath for 2 h and at room temperature for 20.5 h. Another 0.48 ml of 3M methylmagnesium bromide solution was added and the mixture was left to stir at room temperature for 67 h. The mixture was admixed with saturated aqueous ammonium chloride solution and extracted three times with ethyl acetate, and the extracts were washed with sodium chloride solution, filtered through a hydrophobic filter and concentrated. The residue was purified by preparative HPLC. This gave 111 mg of the title compound.
UPLC-MS (Method A2): Rₜ = 0.97 min (UV detector: TIC), mass found 396.22.
¹H-NMR (400MHz, DMSO-d₆): δ [ppm]= 1.15 (s, 6H), 1.64 (s, 6H), 2.00 - 2.08 (m, 2H), 2.61 (s, 3H), 4.41 - 4.59 (m, 3H), 5.92 (s, 1H), 7.50 (dd, 1H), 7.56 (s, 1H), 7.90 - 7.99 (m, 2H), 8.33 (s, 1H), 8.70 (s, 1H), 12.39 (s, 1H).

### Example 21

### 6-(2-Hydroxypropan-2-yl)-N-[6-(2-hydroxypropan-2-yl)-2-(4,4,4-trifluorobutyl)-2H-indazol-5-yl]pyridine-2-carboxamide

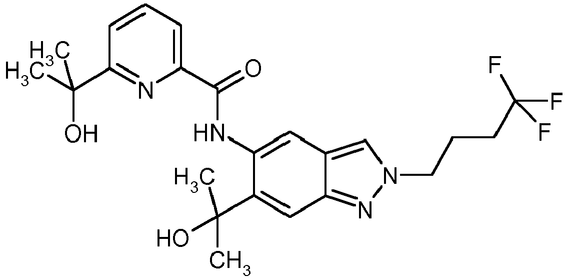

A solution of 72 mg (0.155 mmol) of methyl 5-({[6-(2-hydroxypropan-2-yl)pyridin-2-yl]carbonyl}amino)-2-(4,4,4-trifluorobutyl)-2H-indazole-6-carboxylate (Intermediate 4-7) in 10 ml of THF was cooled in an ice/water cooling bath. 0.26 ml of 3M methylmagnesium bromide solution in diethyl ether was added and the mixture was stirred for 2 h and then at room temperature for 20 h. Another 1 equivalent of the 3M methylmagnesium bromide solution was added and the mixture was stirred at room temperature for 24 h. Saturated aqueous ammonium chloride solution was added, the mixture was three times extracted with ethyl acetate and the extracts were washed with sodium chloride solution and concentrated. Preparative HPLC gave 22 mg (31% of theory) of the title compound.
UPLC-MS (Method A2): Rₜ = 1.15 min (UV detector: TIC), mass found 464.20.
¹H-NMR (400MHz, DMSO-d₆): δ [ppm]= 1.56 (s, 6H), 1.64 (s, 6H), 2.07 - 2.34 (m, 4H), 4.49 (t, 2H), 5.32 (s, 1H), 6.05 (s, 1H), 7.60 (s, 1H), 7.87 (dd, 1H), 7.99 - 8.05 (m, 2H), 8.35 (s, 1H), 8.79 (s, 1H), 12.45 (s, 1H).

Said component B may be in the form of a pharmaceutical formulation which is ready for use to be administered simultaneously, concurrently, separately or sequentially. The components may be administered independnently of one another by the oral, intravenous, topical, local installations, intraperitoneal or nasal route.

In accordance with another embodiment of the above-mentioned aspects of the present invention, said combinations are of :
component B : which is one or more IRAK4-inhibiting compounds of general formula (I) as defined herein, *supra,* which is selected from the list consisting of :
   1) N-[6-(2-hydroxypropan-2-yl)-2-(2-methoxyethyl)-2H-indazol-5-yl]-6-(trifluoromethyl)pyridine-2-carboxamide
   2) N-[6-(hydroxymethyl)-2-(2-methoxyethyl)-2H-indazol-5-yl]-6-(trifluoromethyl)pyridine-2-carboxamide
   3) N-[6-(2-hydroxypropan-2-yl)-2-(3-methoxypropyl)-2H-indazol-5-yl]-6-(trifluoromethyl)pyridine-2-carboxamide
   4) N-[6-(hydroxymethyl)-2-(3-methoxypropyl)-2H-indazol-5-yl]-6-(trifluoromethyl)pyridine-2-carboxamide
   5) N-[2-(2-hydroxyethyl)-6-(2-hydroxypropan-2-yl)-2H-indazol-5-yl]-6-(trifluoromethyl)pyridine-2-carboxamide
   6) N-[6-(2-hydroxypropan-2-yl)-2-(3-hydroxypropyl)-2H-indazol-5-yl]-6-(trifluoromethyl)pyridine-2-carboxamide
   7) N-[2-(2-hydroxyethyl)-6-(hydroxymethyl)-2H-indazol-5-yl]-6-(trifluoromethyl)pyridine-2-carboxamide
   8) N-[6-(2-hydroxypropan-2-yl)-2-(oxetan-3-ylmethyl)-2H-indazol-5-yl]-6-(trifluoromethyl)pyridine-2-carboxamide
   9) N-[6-(hydroxymethyl)-2-(oxetan-3-ylmethyl)-2H-indazol-5-yl]-6-(trifluoromethyl)pyridine-2-carboxamide
   10) N-{6-(2-hydroxypropan-2-yl)-2-[3-(methylsulphonyl)propyl]-2H-indazol-5-yl}-6-(trifluoromethyl)pyridine-2-carboxamide
   11) N-[2-(3-hydroxy-3-methylbutyl)-6-(2-hydroxypropan-2-yl)-2H-indazol-5-yl]-6-(trifluoromethyl)pyridine-2-carboxamide
   12) N-{6-(2-hydroxypropan-2-yl)-2-[2-(methylsulphonyl)ethyl]-2H-indazol-5-yl}-6-(trifluoromethyl)pyridine-2-carboxamide
   13) 6-(difluoromethyl)-N-[2-(3-hydroxy-3-methylbutyl)-6-(2-hydroxypropan-2-yl)-2H-indazol-5-yl]pyridine-2-carboxamide
   14) 6-(difluoromethyl)-N-{6-(2-hydroxypropan-2-yl)-2-[2-(methylsulphonyl)ethyl]-2H-indazol-5-yl}pyridine-2-carboxamide
   15) 6-(difluoromethyl)-N-[6-(2-hydroxypropan-2-yl)-2-(3-hydroxypropyl)-2H-indazol-5-yl]pyridine-2-carboxamide
   16) N-[6-(2-hydroxypropan-2-yl)-2-(4,4,4-trifluorobutyl)-2H-indazol-5-yl]-6-(trifluoromethyl)pyridine-2-carboxamide
   17) N-{6-(2-hydroxypropan-2-yl)-2-[3-(trifluoromethoxy)propyl]-2H-indazol-5-yl}-6-(trifluoromethyl)pyridine-2-carboxamide
   18) N-{6-(2-hydroxypropan-2-yl)-2-[3-(2,2,2-trifluoroethoxy)propyl]-2H-indazol-5-yl}-6-(trifluoromethyl)pyridine-2-carboxamide
   19) 5-fluoro-N-[2-(3-hydroxy-3-methylbutyl)-6-(2-hydroxypropan-2-yl)-2H-indazol-5-yl]-6-methylpyridine-2-carboxamide
   20) N-[2-(3-hydroxy-3-methylbutyl)-6-(2-hydroxypropan-2-yl)-2H-indazol-5-yl]-6-methylpyridine-2-carboxamide
   21) 6-(2-hydroxypropan-2-yl)-N-[6-(2-hydroxypropan-2-yl)-2-(4,4,4-trifluorobutyl)-2H-indazol-5-yl]pyridine-2-carboxamide.

In accordance with an embodiment of the above-mentioned aspects of the present invention, said combinations are of:
component A : 2-amino-N-[7-methoxy-8-(3-morpholin-4-ylpropoxy)-2,3-dihydroimidazo[1,2-c]quinazolin-5-yl]pyrimidine-5-carboxamide or 2-amino-N-[7-methoxy-8-(3-morpholin-4-ylpropoxy)-2,3-dihydroimid-azo[1,2-c]quinazolin-5-yl]pyrimidine-5-carboxamide dihydrochloride, (which is hereinafter referred to as "compound A" or "cpd. A")
   and
component B : N-[2-(3-Hydroxy-3-methylbutyl)-6-(2-hydroxypropan-2-yl)-2H-indazol-5-yl]-6-(trifluoromethyl)pyridine-2-carboxamide.

Said component B may be in the form of a pharmaceutical formulation which is ready for use to be administered simultaneously, concurrently, separately or sequentially. The components may be administered independnently of one another by the oral, intravenous, topical, local installations, intraperitoneal or nasal route.

In accordance with an embodiment, the present invention relates to a combination of any component A mentioned herein with any component B mentioned herein.

In a particular embodiment, the present invention relates to a combination of a component A with a component B, as mentioned in the Examples section herein.

### Useful forms of components A and B of the combinations of the present invention

As mentioned *supra,* either or both of components A and B of any of the combinations of the present invention may be in a useful form, such as pharmaceutically acceptable salts, co-precipitates, metabolites, hydrates, solvates and prodrugs of all the compounds of examples. The term "pharmaceutically acceptable salt" refers to a relatively non-toxic, inorganic or organic acid addition salt of a compound of the present invention. For example, see S. M. Berge, et al. "Pharmaceutical Salts," J. Pharm. Sci. 1977, 66, 1-19. Pharmaceutically acceptable salts include those obtained by reacting the main compound, functioning as a base, with an inorganic or organic acid to form a salt, for example, salts of hydrochloric acid, sulfuric acid, phosphoric acid, methane sulfonic acid, camphor sulfonic acid, oxalic acid, maleic acid, succinic acid and citric acid. Pharmaceutically acceptable salts also include those in which the main compound functions as an acid and is reacted with an appropriate base to form, e.g., sodium, potassium, calcium, magnesium, ammonium, and chorine salts. Those skilled in the art will further recognize that acid addition salts of the claimed compounds may be prepared by reaction of the compounds with the appropriate inorganic or organic acid via any of a number of known methods. Alternatively, alkali and alkaline earth metal salts of acidic compounds of the invention are prepared by reacting the compounds of the invention with the appropriate base via a variety of known methods.

Representative salts of the compounds of this invention include the conventional non-toxic salts and the quaternary ammonium salts which are formed, for example, from inorganic or organic acids or bases by means well known in the art. For example, such acid addition salts include acetate, adipate, alginate, ascorbate, aspartate, benzoate, benzenesulfonate, bisulfate, butyrate, citrate, camphorate, camphorsulfonate, cinnamate, cyclopentanepropionate, digluconate, dodecylsulfate, ethanesulfonate, fumarate, glucoheptanoate, glycerophosphate, hemisulfate, heptanoate, hexanoate, chloride, bromide, iodide, 2-hydroxyethanesulfonate, itaconate, lactate, maleate, mandelate, methanesulfonate, 2-naphthalenesulfonate, nicotinate, nitrate, oxalate, pamoate, pectinate, persulfate, 3-phenylpropionate, picrate, pivalate, propionate, succinate, sulfonate, sulfate, tartrate, thiocyanate, tosylate, and undecanoate.

Base salts include alkali metal salts such as potassium and sodium salts, alkaline earth metal salts such as calcium and magnesium salts, and ammonium salts with organic bases such as dicyclohexylamine and N-methyl-D-glucamine. Additionally, basic nitrogen containing groups may be quaternized with such agents as lower alkyl halides such as methyl, ethyl, propyl, or butyl chlorides, bromides and iodides; dialkyl sulfates like dimethyl, diethyl, dibutyl sulfate, or diamyl sulfates, long chain halides such as decyl, lauryl, myristyl and strearyl chlorides, bromides and iodides, aralkyl halides like benzyl and phenethyl bromides and others.

A solvate for the purpose of this invention is a complex of a solvent and a compound of the invention in the solid state. Exemplary solvates would include, but are not limited to, complexes of a compound of the invention with ethanol or methanol. Hydrates are a specific form of solvate wherein the solvent is water.

### Pharmaceutical formulations of components A and B of the combinations of the present invention

As mentioned *supra,* the components A or B may, independently from one another, be in the form of a pharmaceutical composition or formulation which is ready for use to be administered simultaneously, concurrently, separately or sequentially. The components may be administered independnently of one another by the oral, intravenous, topical, local installations, intraperitoneal or nasal route.

Said compositions can be utilized to achieve the desired pharmacological effect by administration to a patient in need thereof. A patient, for the purpose of this invention, is a mammal, including a human, in need of treatment for the particular condition or disease. Therefore, the present invention includes combinations in which components A and B, independently of one another, are pharmaceutical formulations compositions that are comprised of a pharmaceutically acceptable carrier and a pharmaceutically effective amount of a said component. A pharmaceutically acceptable carrier is preferably a carrier that is relatively non-toxic and innocuous to a patient at concentrations consistent with effective activity of the active ingredient so that any side effects ascribable to the carrier do not vitiate the beneficial effects of component, and/or combination. A pharmaceutically effective amount of a combination is preferably that amount which produces a result or exerts an influence on the particular condition being treated. The combinations of the present invention can be administered with pharmaceutically-acceptable carriers well known in the art using any effective conventional dosage unit forms, including immediate, slow and timed release preparations, orally, parenterally, topically, nasally, ophthalmically, optically, sublingually, rectally, vaginally, and the like.

It is possible for the compounds according to the invention to have systemic and/or local activity. For this purpose, they can be administered in a suitable manner, such as, for example, via the oral, parenteral, pulmonary, nasal, sublingual, lingual, buccal, rectal, vaginal, dermal, transdermal, conjunctival, otic route or as an implant or stent.
For these administration routes, it is possible for the compounds according to the invention to be administered in suitable administration forms.
For oral administration, it is possible to formulate the compounds according to the invention to dosage forms known in the art that deliver the compounds of the invention rapidly and/or in a modified manner, such as, for example, tablets (uncoated or coated tablets, for example with enteric or controlled release coatings that dissolve with a delay or are insoluble), orally-disintegrating tablets, films/wafers, films/lyophylisates, capsules (for example hard or soft gelatine capsules), sugar-coated tablets, granules, pellets, powders, emulsions, suspensions, aerosols or solutions. It is possible to incorporate the compounds according to the invention in crystalline and/or amorphised and/or dissolved form into said dosage forms.
Parenteral administration can be effected with avoidance of an absorption step (for example intravenous, intraarterial, intracardial, intraspinal or intralumbal) or with inclusion of absorption (for example intramuscular, subcutaneous, intracutaneous, percutaneous or intraperitoneal). Administration forms which are suitable for parenteral administration are, inter alia, preparations for injection and infusion in the form of solutions, suspensions, emulsions, lyophylisates or sterile powders.

Examples which are suitable for other administration routes are pharmaceutical forms for inhalation [inter alia powder inhalers, nebulizers], nasal drops, nasal solutions, nasal sprays; tablets/films/wafers/capsules for lingual, sublingual or buccal administration; suppositories; eye drops, eye ointments, eye baths, ocular inserts, ear drops, ear sprays, ear powders, ear-rinses, ear tampons; vaginal capsules, aqueous suspensions (lotions, mixturae agitandae), lipophilic suspensions, emulsions, ointments, creams, transdermal therapeutic systems (such as, for example, patches), milk, pastes, foams, dusting powders, implants or stents. The compounds according to the invention can be incorporated into the stated administration forms. This can be effected in a manner known per se by mixing with pharmaceutically suitable excipients. Pharmaceutically suitable excipients include, inter alia,
- fillers and carriers (for example cellulose, microcrystalline cellulose (such as, for example, Avicels^{®}), lactose, mannitol, starch, calcium phosphate (such as, for example, Di-Cafos^{®})),
- ointment bases (for example petroleum jelly, paraffins, triglycerides, waxes, wool wax, wool wax alcohols, lanolin, hydrophilic ointment, polyethylene glycols),
- bases for suppositories (for example polyethylene glycols, cacao butter, hard fat),
- solvents (for example water, ethanol, isopropanol, glycerol, propylene glycol, medium chain-length triglycerides fatty oils, liquid polyethylene glycols, paraffins),
- surfactants, emulsifiers, dispersants or wetters (for example sodium dodecyl sulfate), lecithin, phospholipids, fatty alcohols (such as, for example, Lanette'), sorbitan fatty acid esters (such as, for example, Span'), polyoxyethylene sorbitan fatty acid esters (such as, for example, Tween'), polyoxyethylene fatty acid glycerides (such as, for example, Cremophor'), polyoxethylene fatty acid esters, polyoxyethylene fatty alcohol ethers, glycerol fatty acid esters, poloxamers (such as, for example, Pluronic^{®}),
- buffers, acids and bases (for example phosphates, carbonates, citric acid, acetic acid, hydrochloric acid, sodium hydroxide solution, ammonium carbonate, trometamol, triethanolamine),
- isotonicity agents (for example glucose, sodium chloride),
- adsorbents (for example highly-disperse silicas),
- viscosity-increasing agents, gel formers, thickeners and/or binders (for example polyvinylpyrrolidone, methylcellulose, hydroxypropylmethylcellulose, hydroxypropylcellulose, carboxymethylcellulose-sodium, starch, carbomers, polyacrylic acids (such as, for example, Carbopol^{®}); alginates, gelatine),
- disintegrants (for example modified starch, carboxymethylcellulose-sodium, sodium starch glycolate (such as, for example, Explotab^{®}), cross- linked polyvinylpyrrolidone, croscarmellose-sodium (such as, for example, AcDiSol^{®})),
- flow regulators, lubricants, glidants and mould release agents (for example magnesium stearate, stearic acid, talc, highly-disperse silicas (such as, for example, Aerosil^{®})),
- coating materials (for example sugar, shellac) and film formers for films or diffusion membranes which dissolve rapidly or in a modified manner (for example polyvinylpyrrolidones (such as, for example, Kollidon^{®}), polyvinyl alcohol, hydroxypropylmethylcellulose, hydroxypropylcellulose, ethylcellulose, hydroxypropylmethylcellulose phthalate, cellulose acetate, cellulose acetate phthalate, polyacrylates, polymethacrylates such as, for example, Eudragit^{®})),
- capsule materials (for example gelatine, hydroxypropylmethylcellulose),
- synthetic polymers (for example polylactides, polyglycolides, polyacrylates, polymethacrylates (such as, for example, Eudragit'), polyvinylpyrrolidones (such as, for example, Kollidon^{®}), polyvinyl alcohols, polyvinyl acetates, polyethylene oxides, polyethylene glycols and their copolymers and blockcopolymers),
- plasticizers (for example polyethylene glycols, propylene glycol, glycerol, triacetine, triacetyl citrate, dibutyl phthalate),
- penetration enhancers,
- stabilisers (for example antioxidants such as, for example, ascorbic acid, ascorbyl palmitate, sodium ascorbate, butylhydroxyanisole, butylhydroxytoluene, propyl gallate),
- preservatives (for example parabens, sorbic acid, thiomersal, benzalkonium chloride, chlorhexidine acetate, sodium benzoate),
- colourants (for example inorganic pigments such as, for example, iron oxides, titanium dioxide),
- flavourings, sweeteners, flavour- and/or odour-masking agents.

The present invention furthermore relates to a pharmaceutical composition which comprise at least one compound according to the invention, conventionally together with one or more pharmaceutically suitable excipient(s), and to their use according to the present invention.

### Method of treating cancer

Within the context of the present invention, the term "cancer" includes, but is not limited to, cancers of the breast, lung, brain, reproductive organs, digestive tract, urinary tract, liver, eye, skin, head and neck, thyroid, parathyroid and their distant metastases. Those disorders also include multiple myeloma, lymphomas, sarcomas, and leukemias.

Examples of breast cancer include, but are not limited to invasive ductal carcinoma, invasive lobular carcinoma, ductal carcinoma in situ, and lobular carcinoma in situ.

Examples of cancers of the respiratory tract include, but are not limited to small-cell and non-small-cell lung carcinoma, as well as bronchial adenoma and pleuropulmonary blastoma.

Examples of brain cancers include, but are not limited to brain stem and hypophtalmic glioma, cerebellar and cerebral astrocytoma, medulloblastoma, ependymoma, as well as neuroectodermal and pineal tumor.

Tumors of the male reproductive organs include, but are not limited to prostate and testicular cancer. Tumors of the female reproductive organs include, but are not limited to endometrial, cervical, ovarian, vaginal, and vulvar cancer, as well as sarcoma of the uterus.

Tumors of the digestive tract include, but are not limited to anal, colon, colorectal, esophageal, gallbladder, gastric, pancreatic, rectal, small-intestine, and salivary gland cancers.

Tumors of the urinary tract include, but are not limited to bladder, penile, kidney, renal pelvis, ureter, urethral and human papillary renal cancers.

Eye cancers include, but are not limited to intraocular melanoma and retinoblastoma.

Examples of liver cancers include, but are not limited to hepatocellular carcinoma (liver cell carcinomas with or without fibrolamellar variant), cholangiocarcinoma (intrahepatic bile duct carcinoma), and mixed hepatocellular cholangiocarcinoma.

Skin cancers include, but are not limited to squamous cell carcinoma, Kaposi's sarcoma, malignant melanoma, Merkel cell skin cancer, and non-melanoma skin cancer.

Head-and-neck cancers include, but are not limited to laryngeal, hypopharyngeal, nasopharyngeal, oropharyngeal cancer, lip and oral cavity cancer and squamous cell.

Lymphomas include, but are not limited to AIDS-related lymphoma, non-Hodgkin's lymphoma, cutaneous T-cell lymphoma, Burkitt lymphoma, Hodgkin's disease, and lymphoma of the central nervous system.

Sarcomas include, but are not limited to sarcoma of the soft tissue, osteosarcoma, malignant fibrous histiocytoma, lymphosarcoma, and rhabdomyosarcoma.

Leukemias include, but are not limited to acute myeloid leukemia, acute lymphoblastic leukemia, chronic lymphocytic leukemia, chronic myelogenous leukemia, and hairy cell leukemia.

The present invention relates to a method for using the combinations of the present invention, in the treatment or prophylaxis of a cancer, particularly non-Hodgkin's lymphoma (hereinafter abbreviated to "NHL"), particularly 1st line, 2nd line, relapsed, refractory, indolent or aggressive non-Hodgkin's lymphoma (NHL), in particular follicular lymphoma (hereinafter abbreviated to "FL"), chronic lymphocytic leukaemia (hereinafter abbreviated to "CLL"), marginal zone lymphoma (hereinafter abbreviated to "MZL"), diffuse large B-cell lymphoma (hereinafter abbreviated to "DLBCL"), mantle cell lymphoma (MCL), transformed lymphoma (hereinafter abbreviated to "TL"), or peripheral T-cell lymphoma (hereinafter abbreviated to "PTCL"). Combinations can be utilized to inhibit, block, reduce, decrease, etc., cell proliferation and/or cell division, and/or produce apoptosis, in the treatment or prophylaxis of cancer, in particular non-Hodgkin's lymphoma (hereinafter abbreviated to "NHL"), particularly 1st line, 2nd line, relapsed, refractory, indolent or aggressive non-Hodgkin's lymphoma (NHL), in particular follicular lymphoma (hereinafter abbreviated to "FL"), chronic lymphocytic leukaemia (hereinafter abbreviated to "CLL"), marginal zone lymphoma (hereinafter abbreviated to "MZL"), diffuse large B-cell lymphoma (hereinafter abbreviated to "DLBCL"), mantle cell lymphoma (MCL), transformed lymphoma (hereinafter abbreviated to "TL"), or peripheral T-cell lymphoma (hereinafter abbreviated to "PTCL"). This method comprises administering to a mammal in need thereof, including a human, an amount of a combination of this invention, or a pharmaceutically acceptable salt, isomer, polymorph, metabolite, hydrate, solvate or ester thereof; etc. which is effective for the treatment or prophylaxis of cancer, in particular non-Hodgkin's lymphoma (hereinafter abbreviated to "NHL"), particularly 1st line, 2nd line, relapsed, refractory, indolent or aggressive non-Hodgkin's lymphoma (NHL), in particular follicular lymphoma (hereinafter abbreviated to "FL"), chronic lymphocytic leukaemia (hereinafter abbreviated to "CLL"), marginal zone lymphoma (hereinafter abbreviated to "MZL"), diffuse large B-cell lymphoma (hereinafter abbreviated to "DLBCL"), mantle cell lymphoma (MCL), transformed lymphoma (hereinafter abbreviated to "TL"), or peripheral T-cell lymphoma (hereinafter abbreviated to "PTCL").

The term "treating" or "treatment" as stated throughout this document is used conventionally, e.g., the management or care of a subject for the purpose of combating, alleviating, reducing, relieving, improving the condition of, etc., of a disease or disorder, such as a carcinoma.

### Dose and administration

Based upon standard laboratory techniques known to evaluate compounds useful for the treatment or prophylaxis of cancer, in particular non-Hodgkin's lymphoma (hereinafter abbreviated to "NHL"), particularly 1st line, 2nd line, relapsed, refractory, indolent or aggressive non-Hodgkin's lymphoma (NHL), in particular follicular lymphoma (hereinafter abbreviated to "FL"), chronic lymphocytic leukaemia (hereinafter abbreviated to "CLL"), marginal zone lymphoma (hereinafter abbreviated to "MZL"), diffuse large B-cell lymphoma (hereinafter abbreviated to "DLBCL"), mantle cell lymphoma (MCL), transformed lymphoma (hereinafter abbreviated to "TL"), or peripheral T-cell lymphoma (hereinafter abbreviated to "PTCL"), by standard toxicity tests and by standard pharmacological assays for the determination of treatment of the conditions identified above in mammals, and by comparison of these results with the results of known medicaments that are used to treat these conditions, the effective dosage of the combinations of this invention can readily be determined for treatment of the indication. The amount of the active ingredient to be administered in the treatment of the condition can vary widely according to such considerations as the particular combination and dosage unit employed, the mode of administration, the period of treatment, the age and sex of the patient treated, and the nature and extent of the condition treated.

The total amount of the active ingredient to be administered will generally range from about 0.001 mg/kg to about 200 mg/kg body weight per day, and preferably from about 0.01 mg/kg to about 20 mg/kg body weight per day. Clinically useful dosing schedules will range from one to three times a day dosing to once every four weeks dosing. In addition, "drug holidays" in which a patient is not dosed with a drug for a certain period of time, may be beneficial to the overall balance between pharmacological effect and tolerability. A unit dosage may contain from about 0.5 mg to about 1,500 mg of active ingredient, and can be administered one or more times per day or less than once a day. The average daily dosage for administration by injection, including intravenous, intramuscular, subcutaneous and parenteral injections, and use of infusion techniques will preferably be from 0.01 to 200 mg/kg of total body weight. The average daily rectal dosage regimen will preferably be from 0.01 to 200 mg/kg of total body weight. The average daily vaginal dosage regimen will preferably be from 0.01 to 200 mg/kg of total body weight. The average daily topical dosage regimen will preferably be from 0.1 to 200 mg administered between one to four times daily. The transdermal concentration will preferably be that required to maintain a daily dose of from 0.01 to 200 mg/kg. The average daily inhalation dosage regimen will preferably be from 0.01 to 100 mg/kg of total body weight.

Of course the specific initial and continuing dosage regimen for each patient will vary according to the nature and severity of the condition as determined by the attending diagnostician, the activity of the specific combination employed, the age and general condition of the patient, time of administration, route of administration, rate of excretion of the drug, drug combinations, and the like. The desired mode of treatment and number of doses of a combination of the present invention or a pharmaceutically acceptable salt or ester or composition thereof can be ascertained by those skilled in the art using conventional treatment tests.

### Therapies using combinations of component A as described supra, component B as described supra, and component C: one or more further pharmaceutical agents.

The combinations of component A and component B of this invention can be administered as the sole pharmaceutical agent or in combination with one or more further pharmaceutical agents where the resulting combination of components A, B and C causes no unacceptable adverse effects. For example, the combinations of components A and B of this invention can be combined with component C, *i.e.* one or more further pharmaceutical agents, such as known anti-angiogenesis, anti-hyper-proliferative, antiinflammatory, analgesic, immunoregulatory, diuretic, antiarrhytmic, anti-hypercholsterolemia, anti-dyslipidemia, anti-diabetic or antiviral agents, and the like, as well as with admixtures and combinations thereof.

Component C, can be one or more pharmaceutical agents such as 131l-chTNT, abarelix, abiraterone, aclarubicin, ado-trastuzumab emtansine, afatinib, aflibercept, aldesleukin, alemtuzumab, Alendronic acid, alitretinoin, altretamine, amifostine, aminoglutethimide, Hexyl aminolevulinate,amrubicin, amsacrine, anastrozole, ancestim, anethole dithiolethione, angiotensin II, antithrombin III, aprepitant, arcitumomab, arglabin, arsenic trioxide, asparaginase, axitinib, azacitidine, basiliximab, belotecan, bendamustine, belinostat, bevacizumab, bexarotene, bicalutamide, bisantrene, bleomycin, bortezomib, buserelin, bosutinib, brentuximab vedotin, busulfan, cabazitaxel, cabozantinib, calcium folinate, calcium levofolinate, capecitabine, capromab, carboplatin, carfilzomib, carmofur, carmustine, catumaxomab, celecoxib, celmoleukin, ceritinib, cetuximab, chlorambucil, chlormadinone, chlormethine, cidofovir, cinacalcet, cisplatin, cladribine, clodronic acid, clofarabine, crisantaspase, cyclophosphamide, cyproterone, cytarabine, dacarbazine, dactinomycin, darbepoetin alfa, dabrafenib, dasatinib, daunorubicin, decitabine, degarelix, denileukin diftitox, denosumab, depreotide, deslorelin, dexrazoxane, dibrospidium chloride, dianhydrogalactitol, diclofenac, docetaxel, dolasetron, doxifluridine, doxorubicin, doxorubicin + estrone, dronabinol, eculizumab, edrecolomab, elliptinium acetate, eltrombopag, endostatin, enocitabine, enzalutamide, epirubicin, epitiostanol, epoetin alfa, epoetin beta, epoetin zeta, eptaplatin, eribulin, erlotinib, esomeprazole, estradiol, estramustine, etoposide, everolimus, exemestane, fadrozole, fentanyl, filgrastim, fluoxymesterone, floxuridine, fludarabine, fluorouracil, flutamide, folinic acid, formestane, fosaprepitant, fotemustine, fulvestrant, gadobutrol, gadoteridol, gadoteric acid meglumine, gadoversetamide, gadoxetic acid, gallium nitrate, ganirelix, gefitinib, gemcitabine, gemtuzumab, Glucarpidase, glutoxim, GM-CSF, goserelin, granisetron, granulocyte colony stimulating factor, histamine dihydrochloride, histrelin, hydroxycarbamide, I-125 seeds, lansoprazole, ibandronic acid, ibritumomab tiuxetan, ibrutinib, idarubicin, ifosfamide, imatinib, imiquimod, improsulfan, indisetron, incadronic acid, ingenol mebutate, interferon alfa, interferon beta, interferon gamma, iobitridol, iobenguane (1231), iomeprol, ipilimumab, irinotecan, Itraconazole, ixabepilone, lanreotide, lapatinib, lasocholine, lenalidomide, lenograstim, lentinan, letrozole, leuprorelin, levamisole, levonorgestrel, levothyroxine sodium, lisuride, lobaplatin, lomustine, lonidamine, masoprocol, medroxyprogesterone, megestrol, melarsoprol, melphalan, mepitiostane, mercaptopurine, mesna, methadone, methotrexate, methoxsalen, methylaminolevulinate, methylprednisolone, methyltestosterone, metirosine, mifamurtide, miltefosine, miriplatin, mitobronitol, mitoguazone, mitolactol, mitomycin, mitotane, mitoxantrone, mogamulizumab, molgramostim, mopidamol, morphine hydrochloride, morphine sulfate, nabilone, nabiximols, nafarelin, naloxone + pentazocine, naltrexone, nartograstim, nedaplatin, nelarabine, neridronic acid, nivolumabpentetreotide, nilotinib, nilutamide, nimorazole, nimotuzumab, nimustine, nitracrine, nivolumab, obinutuzumab, octreotide, ofatumumab, omacetaxine mepesuccinate, omeprazole, ondansetron, oprelvekin, orgotein, orilotimod, oxaliplatin, oxycodone, oxymetholone, ozogamicine, p53 gene therapy, paclitaxel, palifermin, palladium-103 seed, palonosetron, pamidronic acid, panitumumab, pantoprazole, pazopanib, pegaspargase, PEG-epoetin beta (methoxy PEG-epoetin beta), pembrolizumab, pegfilgrastim, peginterferon alfa-2b, pemetrexed, pentazocine, pentostatin, peplomycin, Perflubutane, perfosfamide, Pertuzumab, picibanil, pilocarpine, pirarubicin, pixantrone, plerixafor, plicamycin, poliglusam, polyestradiol phosphate, polyvinylpyrrolidone + sodium hyaluronate, polysaccharide-K, pomalidomide, ponatinib, porfimer sodium, pralatrexate, prednimustine, prednisone, procarbazine, procodazole, propranolol, quinagolide, rabeprazole, racotumomab, radium-223 chloride, radotinib, raloxifene, raltitrexed, ramosetron, ramucirumab, ranimustine, rasburicase, razoxane, refametinib , regorafenib, risedronic acid, rhenium-186 etidronate, rituximab, romidepsin, romiplostim, romurtide, roniciclib , samarium (153Sm) lexidronam, sargramostim, satumomab, secretin, sipuleucel-T, sizofiran, sobuzoxane, sodium glycididazole, sorafenib, stanozolol, streptozocin, sunitinib, talaporfin, tamibarotene, tamoxifen, tapentadol, tasonermin, teceleukin, technetium (99mTc) nofetumomab merpentan, 99mTc-HYNIC-[Tyr3]-octreotide, tegafur, tegafur + gimeracil + oteracil, temoporfin, temozolomide, temsirolimus, teniposide, testosterone, tetrofosmin, thalidomide, thiotepa, thymalfasin, thyrotropin alfa, tioguanine, tocilizumab, topotecan, toremifene, tositumomab, trabectedin, tramadol, trastuzumab, trastuzumab emtansine, treosulfan, tretinoin, trifluridine + tipiracil, trilostane, triptorelin, trametinib, trofosfamide, thrombopoietin, tryptophan, ubenimex, valatinib , valrubicin, vandetanib, vapreotide, vemurafenib, vinblastine, vincristine, vindesine, vinflunine, vinorelbine, vismodegib, vorinostat, vorozole, yttrium-90 glass microspheres, zinostatin, zinostatin stimalamer, zoledronic acid, zorubicin.

Generally, the use of component C in combination with a combination of components A and B of the present invention will serve to:
(1) yield better efficacy in reducing the growth of a tumor or even eliminate the tumor as compared to administration of either agent alone,
(2) provide for the administration of lesser amounts of the administered chemotherapeutic agents,
(3) provide for a chemotherapeutic treatment that is well tolerated in the patient with fewer deleterious pharmacological complications than observed with single agent chemotherapies and certain other combined therapies,
(4) provide for treating a broader spectrum of different cancer types in mammals, especially humans,
(5) provide for a higher response rate among treated patients,
(6) provide for a longer survival time among treated patients compared to standard chemotherapy treatments,
(7) provide a longer time for tumor progression, and/or
(8) yield efficacy and tolerability results at least as good as those of the agents used alone, compared to known instances where other cancer agent combinations produce antagonistic effects.

The following Examples describe the feasability of the present invention, but not restricting the invention to these Examples only.

### EXAMPLES

The following abbreviations are used in the Examples:
"Compound A" (or "cpd. A") means 2-amino-N-[7-methoxy-8-(3-morpholin-4-ylpropoxy)-2,3-dihydroimid-azo[1,2-c]quinazolin-5-yl]pyrimidine-5-carboxamide dihydrochloride: it is published in international patent application PCT/EP2012/055600, published as WO 2012/136553 on October 11, 2012, (which is incorporated herein by reference in its entirety), as the compound of Examples 1 and 2 : 2-amino-N-[7-methoxy-8-(3-morpholin-4-ylpropoxy)-2,3-dihydroimidazo[1,2-c]quinazolin-5-yl]pyrimidine-5-carboxamide dinydrochloride: it may be synthesized according to the methods given in said Examples 1 and 2.
   2-amino-N-[7-methoxy-8-(3-morpholin-4-ylpropoxy)-2,3-dihydroimidazo[1,2-c]quinazolin-5-yl]pyrimidine-5-carboxamide dihydrochloride is a compound of structure : 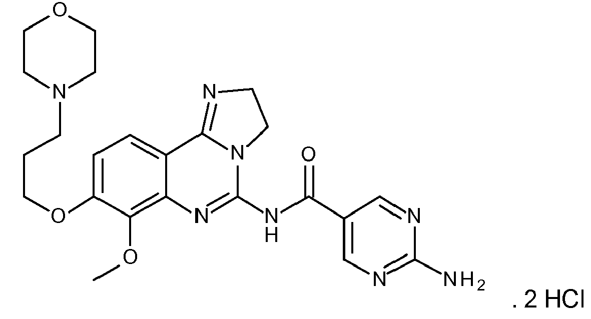 and is an example of component A as described and defined herein.
"Compound B" (or "cpd. B") means N-[2-(3-Hydroxy-3-methylbutyl)-6-(2-hydroxypropan-2-yl)-2H-indazol-5-yl]-6-(trifluoromethyl)pyridine-2-carboxamide, *i.e.* compound Example 11 as described herein, *i.e.* a compound of structure : 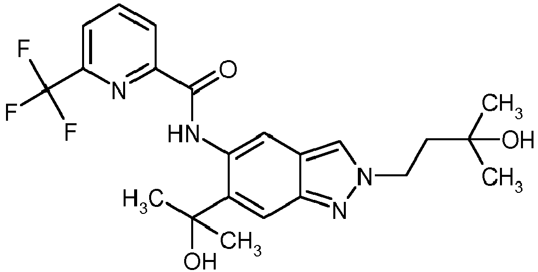 which is an example of component B as described and defined herein.

### EXAMPLE 1 : SYNERGISTIC COMBINATIONS OF COMPONENT A AND COMPONENT B OF THE PRESENT INVENTION IN A PANEL OF 4 DLBCL CELL LINES ASSESSED BY THE COMBINATION INDEX (CI) OF A 72-HOUR CELL PROLIFERATION ASSAY

The effect of combinations of the present invention was evaluated using combination index isobologram analysis for in vitro assessment. The efficacy parameter was the effect in a 72-hour cell proliferation assay. Briefly, cells were plated in 384-well plate with 30 µL medium. After 24 hours, compounds were added to the cells by means of an HP D300 digital dispenser in a 7-step 3-fold dilution series:
- Component A, respectively Compound A alone,
- Component B, respectively Compound B alone, and
- the combination of Component A and component B, respectively Compound A plus Component B, respectively Compound B.

Different ratios (1:0; 0.85:0.15; 0.7:0.3; 0.5:0.5; 0.3:0.7; 0.15:0.85; 0:1) were used to make serial three-fold dilutions to generate response curves at 7 concentrations. Experiments were conducted in triplicates. The mapping IC50 values were calculated. The corresponding component concentrations of A and B were calculated and used for plotting isobolograms. Effects were analyzed as described by Chou (Pharmacology Reviews 2006) and the combination index was calculated using the formula: $Combination Index = \left[Ax\right] / A ′ + \left[Bx\right] / B ′$ [Ax] and [Bx] refer to component A and component B.

A' and B' refer to the IC50 values of A and B, respectively, as a single agent. Combination indices (CIs) of 0-0.3, 0.3-0.6, and 0.6-0.9 were defined to indicate very strong synergy, strong synergy and synergy, respectively. CIs of 0.9-1.1 were defined as additive effect. CIs greater than 1.1 were defined as antagonistic effects.

Table A shows the synergistic combination of component A, *i.e.* the PI3K inhibitor 2-amino-N-[7-methoxy-8-(3-morpholin-4-ylpropoxy)-2,3-dihydroimidazo[1,2-c]quinazolin-5-yl]pyrimidine-5-carboxamide dihydrochloride (compound A (cpdA)) and component B, *i.e.* the IRAK4 inhibitor N-[2-(3-Hydroxy-3-methylbutyl)-6-(2-hydroxypropan-2-yl)-2H-indazol-5-yl]-6-(trifluoromethyl)pyridine-2-carboxamide (compound B (cpd B)) in a panel of 4 DLBCL cell lines assessed by the combination index (CI) of a 72-hour cell proliferation assay.

**TABLE A**

| **Subtype DLBCL** | **cell line** | **compound** | **CI min-max (IC50)** | **Effect** |
|---|---|---|---|---|
| ABC-DLBCL | TMD-8 | cpd. A + cpd. B | 0.60 - 0.78 | S |
| ABC-DLBCL | HBL-1 | cpd. A + cpd. B | 0.71 - 1.04 | SS/ Ad |
| ABC-DLBCL | OCI-Ly3 | cpd. A + cpd. B | 0.58 - 0.92 | S/ Ad |
| ABC-DLBCL | OCI-Ly10 | cpd. A + cpd. B | 0.54 - 0.70 | S |

The abbreviations within the table have the following meaning:
- CI: = Combination Index
- VSS: = Very strong synergy, CI: 0-0.3
- SS: = Strong synergy, CI: 0.3-0.6
- S: = Synergy, CI: 0.6-0.9
- Ad: = Additive effect, CI 0.9-1.2
- Ant: = Antagonistic effect, CI: >1.2

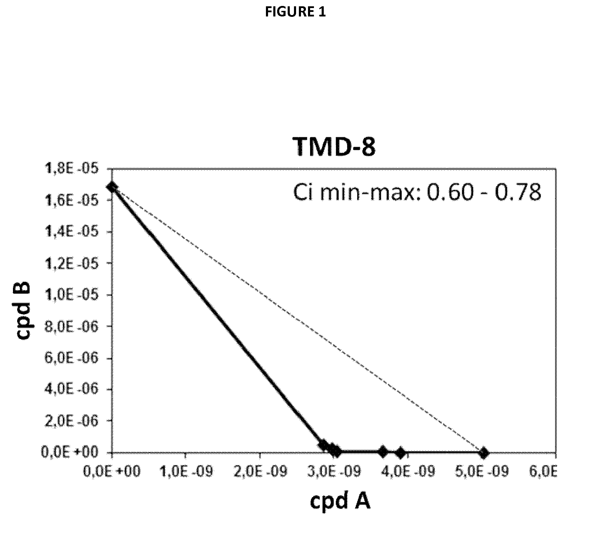

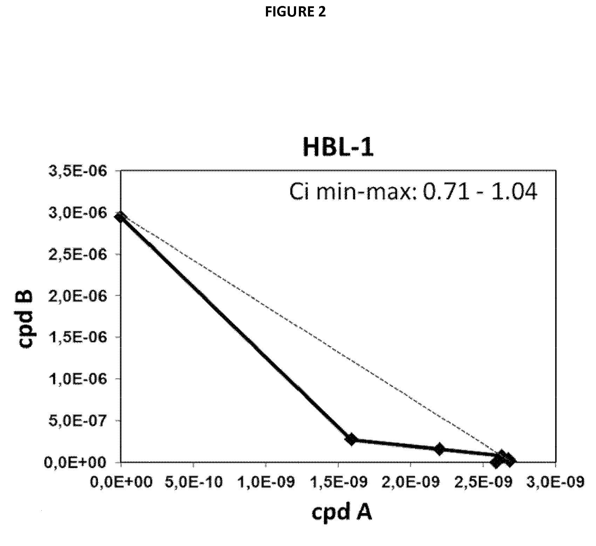

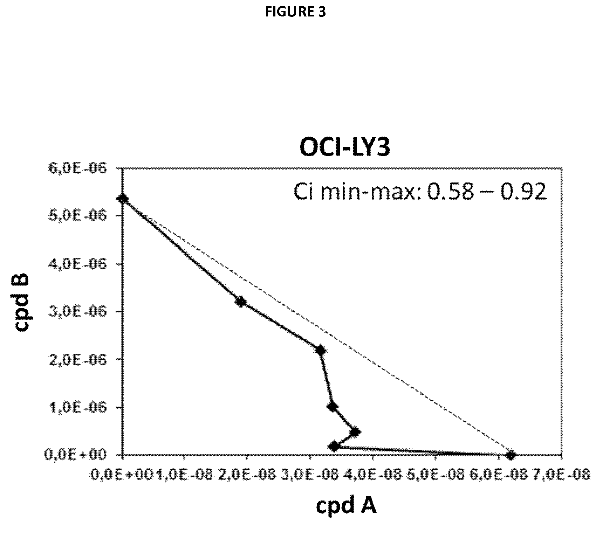

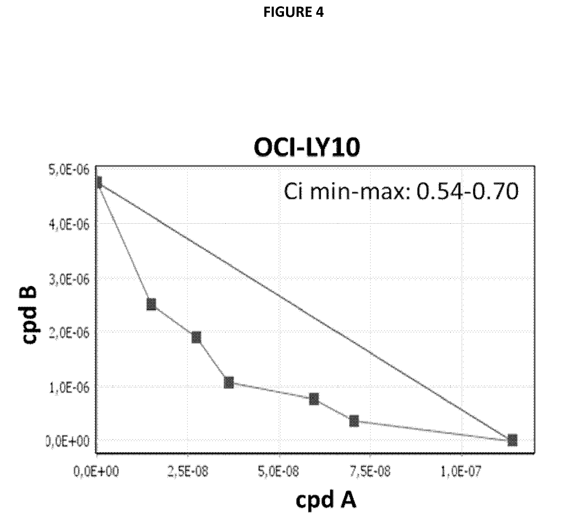

Figures 1 to 4 show the respective isobologramms of the combinations listed in Table A.

### EXAMPLE 2 : SYNERGISTIC COMBINATIONS OF COMPONENT A AND COMPONENT B OF THE PRESENT INVENTION IN A 2 DLBCL CELL LINES ASSESSED BY THE COMBINATION INDEX (CI) OF A 6-HOUR NFKB-PATHWAY REPORTER LUCIFERASE ASSAY

The effect of combinations of the present invention was evaluated using combination index isobologram analysis for in vitro assessment. The efficacy parameter was the effect in a 6-hour NFKB-pathway reporter luciferase assay. Briefly, cells were plated in 384-well plate with 30 µL medium. After 24 hours, compounds were added to the cells by means of an HP D300 digital dispenser in a 7-step 3-fold dilution series:
- Component A, respectively Compound A alone,
- Component B, respectively Compound B alone, and
- the combination of Component A and component B, respectively Compound A plus Component B, respectively Compound B.

Different ratios (1:0; 0.85:0.15; 0.7:0.3; 0.5:0.5; 0.3:0.7; 0.15:0.85; 0:1) were used to make serial three-fold dilutions to generate response curves at 7 concentrations. Experiments were conducted in triplicates. The mapping IC50 values were calculated. The corresponding component concentrations of A and B were calculated and used for plotting isobolograms. Effects were analyzed as described by Chou (Pharmacology Reviews 2006) and the combination index was calculated using the formula: $Combination Index = \left[Ax\right] / A ′ + \left[Bx\right] / B ′$ [Ax] and [Bx] refer to component A and component B.

A' and B' refer to the IC50 values of A and B, respectively, as a single agent. Combination indices (CIs) of 0-0.3, 0.3-0.6, and 0.6-0.9 were defined to indicate very strong synergy, strong synergy and synergy, respectively. CIs of 0.9-1.1 were defined as additive effect. CIs greater than 1.1 were defined as antagonistic effects

Table B shows the synergistic combination of the PI3K inhibitor 2-amino-N-[7-methoxy-8-(3-morpholin-4-ylpropoxy)-2,3-dihydroimidazo[1,2-c]quinazolin-5-yl]pyrimidine-5-carboxamide dihydrochloride (compound A (CpdA)) and the IRAK4 inhibitor (compound B (cpd. B)) in 2 DLBCL cell lines assessed by the combination index (CI) of a 6-hour NFKB-pathway reporter luciferase assay.

**TABLE B**

| **Subtype DLBCL** | **cell line** | **Compound** | **CI min-max (IC50)** | **Effect** |
|---|---|---|---|---|
| ABC-DLBCL | TMD-8-NF-KB-luc | cpd. A + cpd. B | 0.26 - 0.43 | VSS/SS |
| ABC-DLBCL | HBL-1-NF-KB-luc | cpd. A + cpd. B | 0.25 - 0.39 | VSS/SS |

The abbreviations within the table have the following meaning:
- CI: = Combination Index
- VSS: = Very strong synergy, CI: 0-0.3
- SS: = Strong synergy, CI: 0.3-0.6
- S: = Synergy, CI: 0.6-0.9
- Ad: = Additive effect, CI 0.9-1.2
- Ant: = Antagonistic effect, CI: >1.2

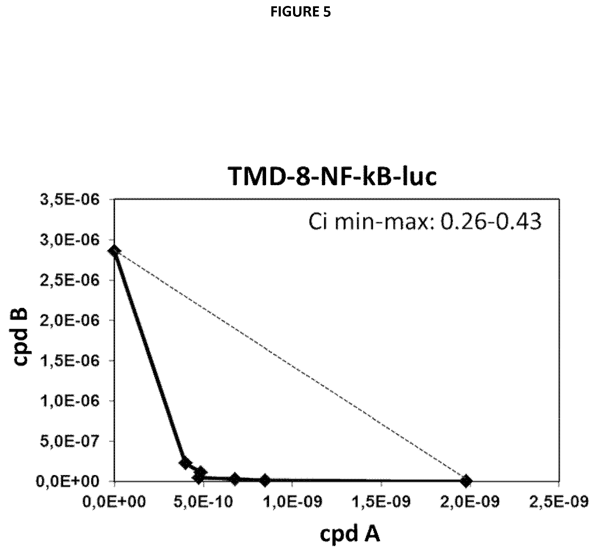

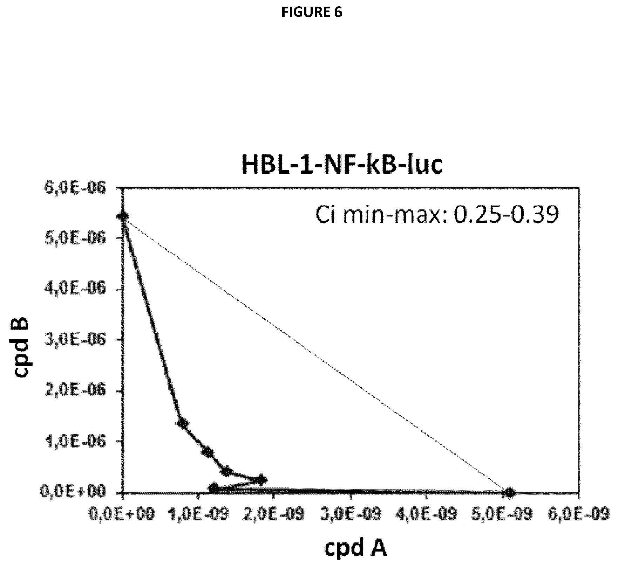

Figures 5 and 6 show the respective isobologramms of the combinations listed in Table B.

### EXAMPLE 3 : IN VIVO B CELL LYMPHOMA XENOGRAFT MODEL

Anti-tumor activity of cpd. B has been analyzed in murine xenograft models. For that purpose tumor fragments from stock mice inoculated with primary human lymphoma tissue were harvested and used for inoculation into female NOD/SCID mice. Each mouse was inoculated subcutaneously at the right flank with primary (PDX) human lymphoma model LY2298 fragment (P7, 2-4 mm in diameter) for tumor development. The treatment was started when the average tumor size reached about 158 mm³. Mice were allocated randomly into experimental groups according to their tumor sizes, 10 mice per group. The day was denoted as day 0. Treatments were administrated to tumor-bearing mice from day 1 through day 34. Treatment groups were: vehicle control, cpd. B monotherapy, **cpd. A** monotherapy and combination of cpd. B and **cpd A** (Table C). Body weights were determined twice per week. Changes in body weight were depicted as treatment related toxicities (>10% stop treatment, >20% termination). Tumor sizes were measured twice weekly in two dimensions using a caliper, and the volume is expressed in mm³ using the formula: V = 0.5 a x b², where a and b are the long and short diameters of the tumor, respectively. The T/C value is an indicator of tumor response to treatment and commonly used as anti-tumor activity endpoint. T and C are the mean tumor volume of the treated and control groups, respectively. Statistical analysis of difference in tumor volume among the groups was evaluated using one-way ANOVA followed by pairweise comparison procedures (Dunett method). P < 0.05 was considered to be statistically significant.

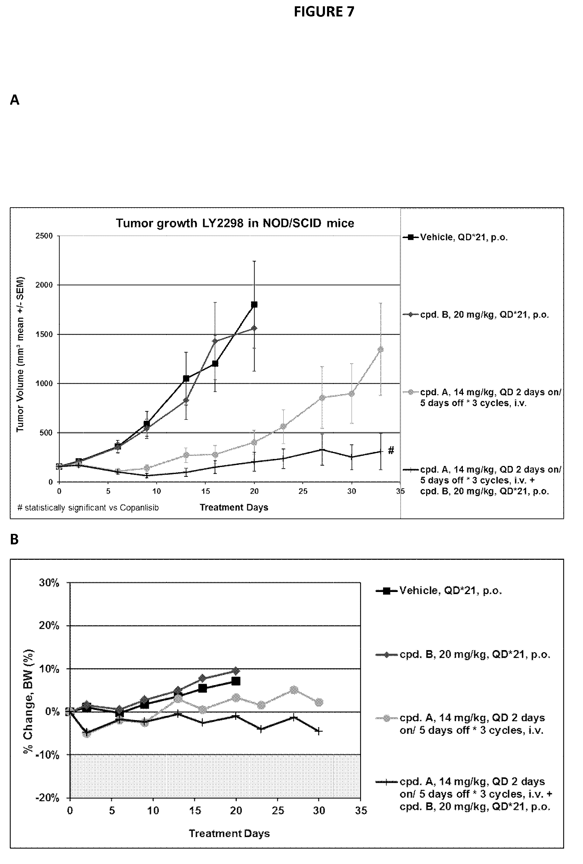

Figure 7 shows activity of cpd. B in monotherapy and combination therapy with cpd. A during treatment of human LY2298 ABC-DLBCL tumors. Cpd. B has been applied orally upon a dose of 20 mg/kg once daily. **Cpd. A** was applied intraveneously for 2 days followd by a 5 day treatment break upon a dose of 14 mg/kg.
Cpd. B alone alone does not exhibit any anti-tumor activity in vivo in LY2298 tumors. **Cpd. A** shows initially a strong anti-tumor activity but after 20 treatment days a clear tumor relapse can be observed despite ongoing therapy. Combination of cpd. B with **cpd. A** can strongly inhibit tumor relapse of **cpd. A** monotherapy indicating synergistic activity of cpd. B and **cpd. A** as demonstrated by statistically significant improvement of **cpd. A** monotherapy by combination with cpd. B. Treatments were well tolerated without critical body weight loss.
In summary this study demonstrates that combination of cpd. B with PI3K inhibitor **cpd.** A can significantly improve the respective monotherapy efficacies in a model of ABC-DLBCL.

**TABLE C: Anti-Tumor activity of cpd. B in monotherapy and combination with cpd. A in LY2298 PDX model in NOD/SCID mice.**

| | **LY2298: human PDX ABC-DLBCL Xenograft Model** | | | |
|---|---|---|---|---|
| **Study-No.** | **E0131-1516 (CrownBio)** | | | |
| **Compound** | **Dose** | **T/C^{a} Volume (at day 20)** | **Max. body weight loss^{b} (%)** | **Response^{c} (at termination)** |
| **Vehicle** | 10 ml/kg QD p.o. + 5 ml/kg QD 2on/5off i.v. | 1.00 | - | 10x PD |
| **cpd. B** | 20 mg/kg QD p.o. | 0.86 | - | 10x PD |
| **cpd. A** | 14 mg/kg QD 2on/5off i.v. | 0.22* | -5 | 7x PD, 1x SD, 1x PR, 1x CR |
| **cpd. B + cpd A** | 20 mg/kg QD p.o. + 14 mg/kg QD 2on/5off i.v. | 0.11* | -5 | 6x CR, 1x PR 3x PD |

| | | | | |
|---|---|---|---|---|
| * P<0.05 (statistically significant vs. Vehicle) a) T/C= Treatment/ Control ratio, Calculated from mean tumor volume at termination of control. b) Body Weight Loss: the maximum mean body weight loss expressed as a percent of the starting weight of the animal. Weight loss greater than 20% is considered toxic. c) Response: PD = progressive disease, the number of tumors exhibiting >20% tumor increase; SD = stable disease, the number of tumors exhibiting <30% tumor shrinkage and <20% tumor increase; PR = partial response, the number of tumors exhibiting >30% tumor shrinkage; CR = complete response, the number of not measureable tumors. | | | | |

Figure 7 shows anti-tumor activity of cpd. B in monotherapy and combination with **cpd. A** in LY2298 PDX model in NOD/SCID mice. (A) Tumor growth curve. (B) Body weight change.

## Claims

**1.** A combination of component A: one or more 2,3-dihydroimidazo[1,2-c]quinazoline compounds of general formula (A1) : 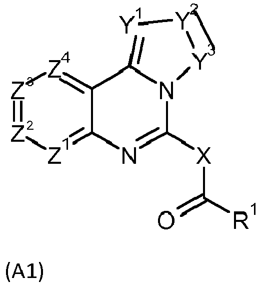 wherein
X represents CR⁵R⁶or NH;
Y¹ represents CR³ or N;
Chemical bond between Y²------Y³ represents a single bond or double bond,
with the proviso that when the Y²------Y³ represents a double bond,
Y² and Y³ independently represent CR⁴ or N, and
when Y²Y³ represents a single bond, Y² and Y³ independently represent CR³R⁴ or NR⁴;
Z¹, Z², Z³ and Z⁴ independently represent CH , CR² or N;
R¹ represents aryl optionally having 1 to 3 substituents selected from R¹¹, C₃₋₈ cycloalkyl optionally having 1 to 3 substituents selected from R¹¹,
C₁₋₆ alkyl optionally substituted by
aryl, heteroaryl, C₁₋₆ alkoxyaryl, aryloxy, heteroaryloxy or one or more halogen,
C₁₋₆ alkoxy optionally substituted by
carboxy, aryl, heteroaryl, C₁₋₆ alkoxyaryl, aryloxy, heteroaryloxy or one or more halogen, or
a 3 to 15 membered mono- or bi-cyclic heterocyclic ring that is saturated or unsaturated, and contains 1 to 3 heteroatoms selected from the group consisting of N, O and S, and optionally having 1 to 3 substituents selected from R¹¹
wherein
R¹¹ represents
halogen, nitro, hydroxy, cyano, carboxy, amino, N-(C₁₋₆alkyl)amino, N-(hydroxyC₁₋₆alkyl)amino, N,N-di(C₁₋₆alkyl)amino, N-(C₁₋₆acyl)amino, N-(formyl)-N-(C₁₋₆alkyl)amino, N-(C₁₋₆alkanesulfonyl) amino, N-(carboxyC₁₋₆alkyl)-N-(C₁₋₆alkyl)amino, N-(C₁₋₆alkoxycabonyl)amino, N-[N,N-di(C₁₋₆alkyl)amino methylene]amino, N-[N,N-di(C₁₋₆alkyl)amino (C₁₋₆ alkyl)methylene]amino, N-[N,N-di(C₁₋₆alkyl)amino C₂₋₆alkenyl]amino, aminocarbonyl, N-(C₁₋₆alkyl)aminocarbonyl, N,N-di(C₁₋₆alkyl)aminocarbonyl, C₃₋₈cycloalkyl, C₁₋₆ alkylthio, C₁₋₆alkanesulfonyl, sulfamoyl, C₁₋₆alkoxycarbonyl,
N-arylamino wherein said aryl moiety is optionally having 1 to 3 substituents selected from R¹⁰¹, N-(aryl C₁₋₆alkyl)amino wherein said aryl moiety is optionally having 1 to 3 substituents selected from R¹⁰¹, aryl C₁₋₆alkoxycarbonyl wherein said aryl moiety is optionally having 1 to 3 substituents selected from R¹⁰¹,
C₁₋₆alkyl optionally substituted by
mono-, di- or tri- halogen, amino, N-(C₁₋₆alkyl)amino or N,N-di(C₁₋₆alkyl)amino, C₁₋₆alkoxy optionally substituted by
mono-, di- or tri- halogen, N-(C₁₋₆alkyl)sulfonamide, or N-(aryl)sulfonamide,
or
a 5 to 7 membered saturated or unsaturated ring having 1 to 3 heteroatoms selected from the group consisting of O, S and N, and optionally having 1 to 3 substituents selected from R¹⁰¹
wherein
R¹⁰¹ represents
halogen, carboxy, amino, N-(C₁₋₆ alkyl)amino, N,N-di(C₁₋₆alkyl)amino, aminocarbonyl, N-(C₁₋₆alkyl)aminocarbonyl, N,N-di(C₁₋₆alkyl)aminocarbonyl, pyridyl,
C₁₋₆ alkyl optionally substituted by cyano or mono- di- or tri- halogen,
or
C₁₋₆alkoxy optionally substituted by cyano, carboxy, amino, N-(C₁₋₆ alkyl)amino, N,N-di(C₁₋₆alkyl)amino, aminocarbonyl, N-(C₁₋₆alkyl)aminocarbonyl, N,N-di(C₁₋₆alkyl)aminocarbonyl or mono-, di- or tri- halogen;
R² represents hydroxy, halogen, nitro, cyano, amino, N-(C₁₋₆alkyl)amino, N,N-di(C₁₋₆alkyl)amino, N-(hydroxyC₁₋₆alkyl)amino, N-(hydroxyC₁₋₆alkyl)-N-(C₁₋₆alkyl)amino, C₁₋₆acyloxy, aminoC₁₋₆acyloxy, C₂₋₆alkenyl, aryl,
a 5-7 membered saturated or unsaturated heterocyclic ring having 1 to 3 heteroatoms selected from the group consisting O, S and N, and optionally substituted by hydroxy, C₁₋₆ alkyl, C₁₋₆ alkoxy, oxo, amino, amino C₁₋₆alkyl, N-(C₁₋₆alkyl)amino, N,N-di(C₁₋₆alkyl)amino, N-(C₁₋₆ acyl)amino, N-(C₁₋₆alkyl)carbonylamino, phenyl, phenyl C₁₋₆ alkyl, carboxy, C₁₋₆alkoxycarbonyl, aminocarbonyl, N-(C₁₋₆alkyl)aminocarbonyl, or N,N-di(C₁₋₆alkyl)amino,
-C(O)- R²⁰
wherein
R²⁰ represents C₁₋₆ alkyl, C₁₋₆ alkoxy, amino, N-(C₁₋₆alkyl)amino, N,N-di(C₁₋₆alkyl)amino, N-(C₁₋₆ acyl)amino, or a 5-7 membered saturated or unsaturated heterocyclic ring having 1 to 3 heteroatoms selected from the group consisting O, S and N, and optionally substituted by C₁₋₆ alkyl, C₁₋₆ alkoxy, oxo, amino, N-(C₁₋₆alkyl)amino, N,N-di(C₁₋₆alkyl)amino, N-(C₁₋₆ acyl)amino, phenyl, or benzyl,
C₁₋₆ alkyl optionally substituted by R²¹ or
C₁₋₆ alkoxy optionally substituted by R²¹
wherein
R²¹ represents cyano, mono-, di or tri- halogen, hydroxy, amino, N-(C₁₋₆alkyl)amino, N,N-di(C₁₋₆alkyl)amino, N- (hydroxyC₁₋₆ alkyl) amino, N- (halophenylC₁₋₆ alkyl) amino, amino C₂₋₆ alkylenyl, C₁₋₆ alkoxy, hydroxyC₁₋₆ alkoxy, -C(O)- R²⁰¹,-NHC(O)- R²⁰¹, C₃₋₈cycloalkyl, isoindolino, phthalimidyl, 2-oxo-1,3-oxazolidinyl, aryl or a 5 or 6 membered saturated or unsaturated heterocyclic ring having 1 to 4 heteroatoms selected from the group consisting O, S and N optionally substituted by
hydroxy, C₁₋₆ alkyl, C₁₋₆ alkoxy, C₁₋₆ alkoxycarbonyl, hydroxyC₁₋₆ alkoxy, oxo, amino, aminoC₁₋₆alkyl, N-(C₁₋₆alkyl)amino, N,N-di(C₁₋₆alkyl)amino, N-(C₁₋₆ acyl)amino, or benzyl,
wherein
R²⁰¹ represents hydroxy, amino, N-(C₁₋₆alkyl)amino, N,N-di(C₁₋₆alkyl)amino, N-(halophenylC₁₋₆ alkyl) amino, C₁₋₆alkyl, aminoC₁₋₆ alkyl, aminoC₂₋₆ alkylenyl, C₁₋₆ alkoxy, a 5 or 6 membered saturated or unsaturated heterocyclic ring having 1 to 4 heteroatoms selected from the group consisting O, S and N optionally substituted by
hydroxy, C₁₋₆ alkyl, C₁₋₆ alkoxy, C₁₋₆ alkoxycarbonyl, hydroxyC₁₋₆ alkoxy, oxo, amino, N-(C₁₋₆alkyl)amino, N,N-di(C₁₋₆alkyl)amino, N-(C₁₋₆ acyl)amino or benzyl;
R³ represents hydrogen, halogen, aminocarbonyl, or C₁₋₆ alkyl optionally substituted by aryl C₁₋₆ alkoxy or mono-, di- or tri- halogen;
R⁴ represents hydrogen or C₁₋₆ alkyl;
R⁵ represents hydrogen or C₁₋₆ alkyl; and
R⁶ represents halogen, hydrogen or C₁₋₆ alkyl ;
or a physiologically acceptable salt, solvate, hydrate or stereoisomer thereof;
optionally in the form of a pharmaceutical formulation which is ready for use to be administered simultaneously, concurrently, separately or sequentially ;
and
component B : which is one or more IRAK4-inhibiting compounds of general formula (I) : 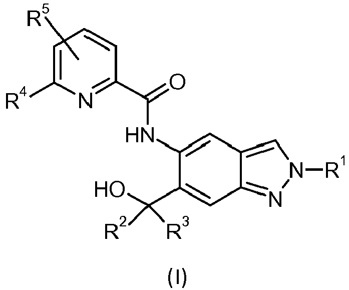 in which:
R¹ is C₁-C₆-alkyl, where the C₁-C₆-alkyl radical is unsubstituted or mono- or polysubstituted identically or differently by
halogen, hydroxyl, an unsubstituted or mono- or poly-halogen-substituted C₃-C₆-cycloalkyl, or an R⁶, R⁷SO₂, R⁷SO or R⁸O radical,
or a group selected from: 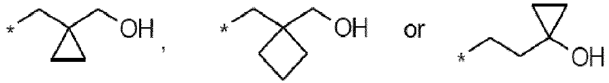 where * represents the bonding site of the group to the rest of the molecule;
R² and R³ always have the same definition and are both either hydrogen or C₁-C₆-alkyl;
R⁴ is halogen, cyano, an unsubstituted or a singly or multiply, identically or differently substituted C₁-C₆-alkyl or an unsubstituted or a singly or multiply, identically or differently substituted C₃-C₆-cycloalkyl, and the substituents are selected from the group of halogen and hydroxyl;
R⁵ is hydrogen, halogen or an unsubstituted or poly-halogen-substituted C₁-C₆-alkyl;
R⁶ is an unsubstituted or mono- or di-methyl-substituted monocyclic saturated heterocycle having 4 to 6 ring atoms, which contains a heteroatom or a heterogroup from the group of O, S, SO and SO₂;
R⁷ is C₁-C₆-alkyl, where the C₁-C₆-alkyl radical is unsubstituted or mono- or polysubstituted identically or differently by halogen, hydroxyl or C₃-C₆-cycloalkyl; or R⁷ is C₃-C₆-cycloalkyl,
R⁸ is C₁-C₆-alkyl, where the C₁-C₆-alkyl radical is unsubstituted or mono- or polysubstituted identically or differently by halogen;
or a diastereomer, an enantiomer, a metabolite, a salt, a solvate or a solvates of a salt thereof.

**2.** The combination according to claim 1, wherein :
said component A is one or more 2,3-dihydroimidazo[1,2-c]quinazoline compounds of general formula (A2) : 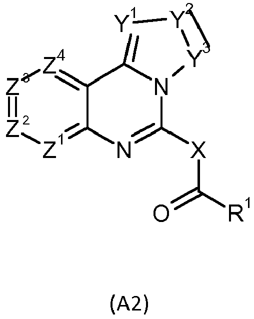 in which :
X represents CR⁵R⁶or NH;
Y¹ represents CR³ or N;
the chemical bond between Y²------Y³ represents a single bond or double bond,
with the proviso that when the Y²------Y³ represents a double bond, Y² and Y³ independently represent CR⁴ or N, and
when Y²Y³ represents a single bond, Y² and Y³ independently represent CR³R⁴ or NR⁴;
Z¹**,** Z², Z³ and Z⁴ independently represent CH , CR² or N;
R¹ represents aryl optionally having 1 to 3 substituents selected from R¹¹, C₃₋₈ cycloalkyl optionally having 1 to 3 substituents selected from R¹¹,
C₁₋₆ alkyl optionally substituted by aryl, heteroaryl, C₁₋₆ alkoxyaryl, aryloxy, heteroaryloxy or one or more halogen,
C₁₋₆ alkoxy optionally substituted by carboxy, aryl, heteroaryl, C₁₋₆ alkoxyaryl, aryloxy, heteroaryloxy or one or more halogen,
or
a 3 to 15 membered mono- or bi-cyclic heterocyclic ring that is saturated or unsaturated, optionally having 1 to 3 substituents selected from R¹¹, and contains 1 to 3 heteroatoms selected from the group consisting of N, O and S,
wherein
R¹¹ represents halogen, nitro, hydroxy, cyano, carboxy, amino, N-(C₁₋₆alkyl)amino, N-(hydroxyC₁₋₆alkyl)amino, N,N-di(C₁₋₆alkyl)amino, N-(C₁₋₆acyl)amino, N-(formyl)-N-(C₁₋₆alkyl)amino, N-(C₁₋₆alkanesulfonyl) amino, N-(carboxyC₁₋₆alkyl)-N-(C₁₋₆alkyl)amino, N-(C₁₋₆alkoxycabonyl)amino, N-[N,N-di(C₁₋₆alkyl)amino methylene]amino, N-[N,N-di(C₁₋₆alkyl)amino (C₁₋₆alkyl)methylene]amino, N-[N,N-di(C₁₋₆alkyl)amino C₂₋₆alkenyl]amino, aminocarbonyl, N-(C₁₋₆alkyl)aminocarbonyl, N,N-di(C₁₋₆alkyl)aminocarbonyl, C₃₋₈cycloalkyl, C₁₋₆ alkylthio, C₁₋₆alkanesulfonyl, sulfamoyl, C₁₋₆alkoxycarbonyl, N-arylamino wherein said aryl moiety is optionally having 1 to 3 substituents selected from R¹⁰¹, N-(aryl C₁₋₆alkyl)amino wherein said aryl moiety is optionally having 1 to 3 substituents selected from R¹⁰¹, aryl C₁₋₆alkoxycarbonyl wherein said aryl moiety is optionally having 1 to 3 substituents selected from R¹⁰¹,
C₁₋₆alkyl optionally substituted by mono-, di- or tri- halogen, amino, N-(C₁₋₆alkyl)amino or N,N-di(C₁₋₆alkyl)amino,
C₁₋₆alkoxy optionally substituted by mono-, di- or tri- halogen, N-(C₁₋₆alkyl)sulfonamide, or N-(aryl)sulfonamide,
or
a 5 to 7 membered saturated or unsaturated ring having 1 to 3 heteroatoms selected from the group consisting of O, S and N, and optionally having 1 to 3 substituents selected from R¹⁰¹
wherein
R¹⁰¹ represents halogen, carboxy, amino, N-(C₁₋₆ alkyl)amino, N,N-di(C₁₋₆alkyl)amino, aminocarbonyl, N-(C₁₋₆alkyl)aminocarbonyl, N,N-di(C₁₋₆alkyl)aminocarbonyl, pyridyl,
C₁₋₆ alkyl optionally substituted by cyano or mono- di- or tri- halogen, and
C₁₋₆alkoxy optionally substituted by cyano, carboxy, amino, N-(C₁₋₆ alkyl)amino, N,N-di(C₁₋₆alkyl)amino, aminocarbonyl, N-(C₁₋₆alkyl)aminocarbonyl, N,N-di(C₁₋₆alkyl)aminocarbonyl or mono-, di- or tri- halogen;
R² represents hydroxy, halogen, nitro, cyano, amino, N-(C₁₋₆alkyl)amino, N,N-di(C₁₋₆alkyl)amino, N-(hydroxyC₁₋₆alkyl)amino, N-(hydroxyC₁₋₆alkyl)-N-(C₁₋₆alkyl)amino, C₁₋₆acyloxy, aminoC₁₋₆acyloxy, C₂₋₆alkenyl, aryl,
a 5-7 membered saturated or unsaturated heterocyclic ring having 1 to 3 heteroatoms selected from the group consisting O, S and N, and optionally substituted by
hydroxy, C₁₋₆ alkyl, C₁₋₆ alkoxy, oxo, amino, amino C₁₋₆alkyl, N-(C₁₋₆alkyl)amino, N,N-di(C₁₋₆alkyl)amino, N-(C₁₋₆ acyl)amino, N-(C₁₋₆alkyl)carbonylamino, phenyl, phenyl C₁₋₆ alkyl, carboxy, C₁₋₆alkoxycarbonyl, aminocarbonyl, N-(C₁₋₆alkyl)aminocarbonyl, or N,N-di(C₁₋₆alkyl)amino, -C(O)- R²⁰ wherein
R²⁰ represents C₁₋₆ alkyl, C₁₋₆ alkoxy, amino, N-(C₁₋₆alkyl)amino, N,N-di(C₁₋₆alkyl)amino, N-(C₁₋₆ acyl)amino, or a 5-7 membered saturated or unsaturated heterocyclic ring having 1 to 3 heteroatoms selected from the group consisting O, S and N, and optionally substituted by C₁₋₆ alkyl, C₁₋₆ alkoxy, oxo, amino, N-(C₁₋₆alkyl)amino, N,N-di(C₁₋₆alkyl)amino, N-(C₁₋₆acyl)amino, phenyl, or benzyl,
C₁₋₆ alkyl optionally substituted by R²¹, or
C₁₋₆ alkoxy optionally substituted by R²¹, wherein
R²¹ represents cyano, mono-, di or tri- halogen, hydroxy, amino, N-(C₁₋₆alkyl)amino, N,N-di(C₁₋₆alkyl)amino, N- (hydroxyC₁₋₆ alkyl) amino, N- (halophenylC₁₋₆ alkyl) amino, amino C₂₋₆ alkylenyl, C₁₋₆ alkoxy, hydroxyC₁₋₆ alkoxy, -C(O)- R²⁰¹, -NHC(O)- R²⁰¹, C₃₋₈cycloalkyl, isoindolino, phthalimidyl, 2-oxo-1,3-oxazolidinyl, aryl or a 5 or 6 membered saturated or unsaturated heterocyclic ring having 1 to 4 heteroatoms selected from the group consisting O, S and N , and optionally substituted by hydroxy, C₁₋₆ alkyl, C₁₋₆ alkoxy, C₁₋₆ alkoxycarbonyl, hydroxyC₁₋₆ alkoxy, oxo, amino, aminoC₁₋₆alkyl, N-(C₁₋₆alkyl)amino, N,N-di(C₁₋₆alkyl)amino, N-(C₁₋₆ acyl)amino, or benzyl,
wherein
R²⁰¹ represents hydroxy, amino, N-(C₁₋₆alkyl)amino, N,N-di(C₁₋₆alkyl)amino, N- (halophenylC₁₋₆ alkyl) amino, C₁₋₆alkyl, aminoC₁₋₆ alkyl, aminoC₂₋₆ alkylenyl, C₁₋₆ alkoxy, a 5 or 6 membered saturated or unsaturated heterocyclic ring having 1 to 4 heteroatoms selected from the group consisting O, S and N, and optionally substituted by hydroxy, C₁₋₆ alkyl, C₁₋₆ alkoxy, C₁₋₆ alkoxycarbonyl, hydroxyC₁₋₆ alkoxy, oxo, amino, N-(C₁₋₆alkyl)amino, N,N-di(C₁₋₆alkyl)amino, N-(C₁₋₆ acyl)amino or benzyl;
R³ represents hydrogen, halogen, aminocarbonyl, or C₁₋₆ alkyl optionally substituted by aryl C₁₋₆ alkoxy or mono-, di- or tri- halogen;
R⁴ represents hydrogen or C₁₋₆ alkyl;
R⁵ represents hydrogen or C₁₋₆ alkyl; and
R⁶ represents halogen, hydrogen or C₁₋₆ alkyl ;
or a physiologically acceptable salt, solvate, hydrate or stereoisomer thereof;
optionally in the form of a pharmaceutical formulation which is ready for use to be administered simultaneously, concurrently, separately or sequentially.

**3.** The combination according to claim 1, wherein :
said component A is one or more 2,3-dihydroimidazo[1,2-c]quinazoline compounds of general formula (A1) according to claim 1, which is selected from the list consisting of specific compound Examples 1-1 to 1-210 on pp. 47 to 106, specific compound Examples 2-1 to 2-368 on pp. 107 to 204, specific compound Examples 3-1 to 3-2 on pp. 205 to 207, specific compound Examples 4-1 to 4-2 on pp. 208 to 210, of in International patent application PCT/EP2003/010377, published as WO 04/029055 A1 on April 08, 2004 and 2-amino-N-[7-methoxy-8-(3-morpholin-4-ylpropoxy)-2,3-dihydroimid-azo[1,2-c]quinazolin-5-yl]pyrimidine-5-carboxamide dihydrochloride, published in international patent application PCT/EP2012/055600, published as WO 2012/136553 ;
or a physiologically acceptable salt, solvate, hydrate or stereoisomer thereof;
optionally in the form of a pharmaceutical formulation which is ready for use to be administered simultaneously, concurrently, separately or sequentially.

**4.** The combination according to claim 2, wherein :
said component A is one or more 2,3-dihydroimidazo[1,2-c]quinazoline compounds of general formula (A2) according to claim 2, which is selected from the list consisting of :
Example 1 : N-[7-methoxy-8-(3-morpholin-4-ylpropoxy)-2,3-dihydroimidazo[1,2-c]quinazolin-5-yl]pyrimidine-5-carboxamide
Example 2 : N-(8-{3-[(2R,6S)-2,6-dimethylmorpholin-4-yl]propoxy}-7-methoxy-2,3-dihydroimidazo[1,2-c]quinazolin-5-yl)nicotinamide
Example 3 : N-(8-{3-[(2R,6S)-2,6-dimethylmorpholin-4-yl]propoxy}-7-methoxy-2,3-dihydroimidazo[1,2-c]quinazolin-5-yl)-2,4-dimethyl-1,3-thiazole-5-carboxamide
Example 4 : 2-amino-N-[7-methoxy-8-(3-morpholin-4-ylpropoxy)-2,3-dihydroimidazo[1,2-c]quinazolin-5-yl]-1,3-thiazole-5-carboxamide.
Example 5 : 2-amino-N-[7-methoxy-8-(3-morpholin-4-ylpropoxy)-2,3-dihydroimidazo[1,2-c]quinazolin-5-yl]isonicotinamide
Example 6 : 2-amino-N-[7-methoxy-8-(3-morpholin-4-ylpropoxy)-2,3-dihydroimidazo[1,2-c]quinazolin-5-yl]-4-methyl-1,3-thiazole-5-carboxamide
Example 7 : 2-amino-N-[7-methoxy-8-(3-morpholin-4-ylpropoxy)-2,3-dihydroimidazo[1,2-c]quinazolin-5-yl]-4-propylpyrimidine-5-carboxamide
Example 8 : N-{8-[2-(4-ethylmorpholin-2-yl)ethoxy]-7-methoxy-2,3-dihydroimidazo[1,2-c]quinazolin-5-yl}nicotinamide
Example 9 : N-{8-[2-(dimethylamino)ethoxy]-7-methoxy-2,3-dihydroimidazo[1,2-c]quinazolin-5-yl}pyrimidine-5-carboxamide
Example 10 : N-(8-{3-[2-(hydroxymethyl)morpholin-4-yl]propoxy}-7-methoxy-2,3-dihydroimidazo[1,2-c]quinazolin-5-yl)nicotinamide
Example 11 : N-(8-{3-[2-(hydroxymethyl)morpholin-4-yl]propoxy}-7-methoxy-2,3-dihydroimidazo[1,2-c]quinazolin-5-yl)nicotinamide
Example 12 : N-{8-[3-(dimethylamino)propoxy]-7-methoxy-2,3-dihydroimidazo[1,2-c]quinazolin-5-yl}nicotinamide 1-oxide
Example 13 : 2-amino-N-[7-methoxy-8-(3-morpholin-4-ylpropoxy)-2,3-dihydroimidazo[1,2-c]quinazolin-5-yl]pyrimidine-5-carboxamide.
Example 14 : N-[7-methoxy-8-(3-morpholin-4-ylpropoxy)-2,3-dihydroimidazo[1,2-c]quinazolin-5-yl]-6-(2-pyrrolidin-1-ylethyl)nicotinamide.
Example 15 : 6-(cyclopentylamino)-N-[7-methoxy-8-(3-morpholin-4-ylpropoxy)-2,3-dihydroimidazo[1,2-c]quinazolin-5-yl]nicotinamide
| Example | Structure |
|---|---|
| 16 | 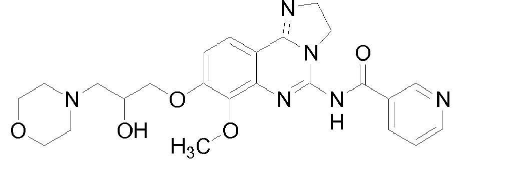 |
| 17 | 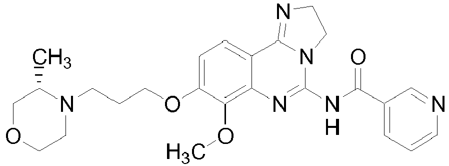 |
| 18 | 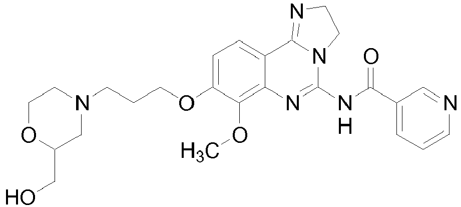 |
| 19 | 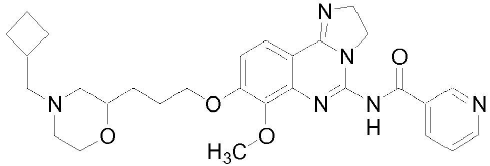 |
| 20 | 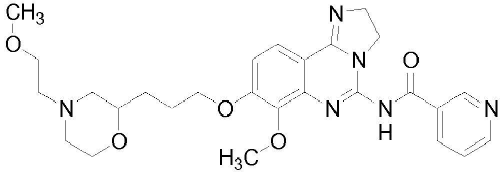 |
| 21 | 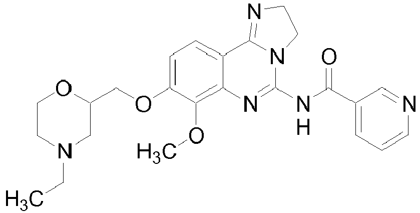 |
| 22 | 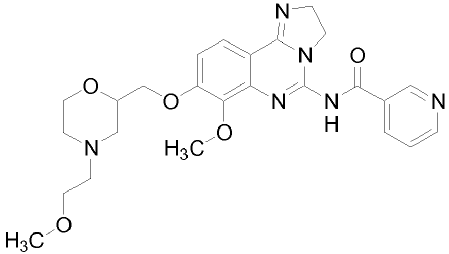 |
| 23 | 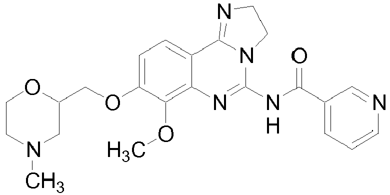 |
| 24 | 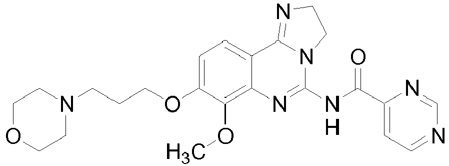 |
| 25 | 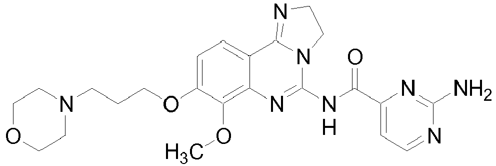 |
| 26 | 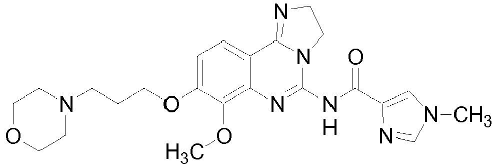 |
| 27 | 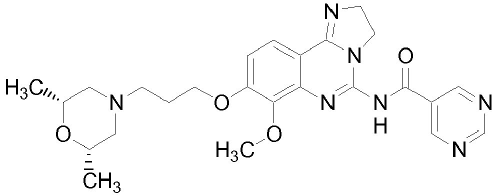 |
| 28 | 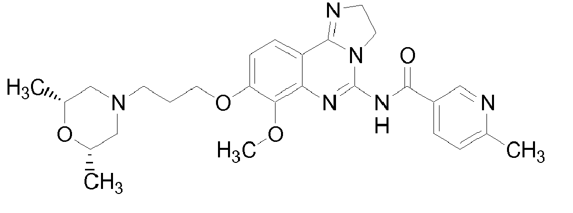 |
| 29 | 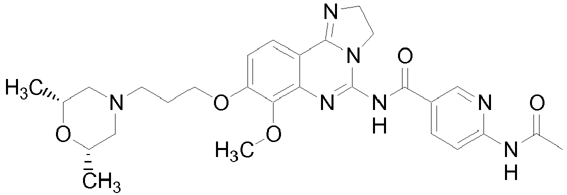 |
| 30 | 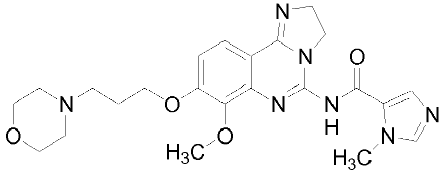 |
| 31 | 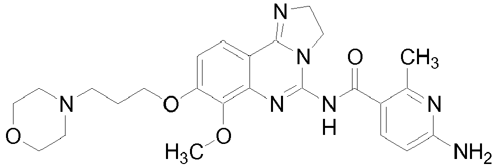 |
| 32 | 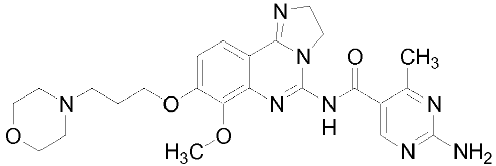 |
| 33 | 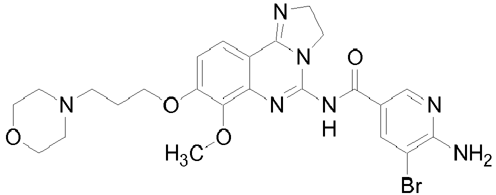 |
| 34 | 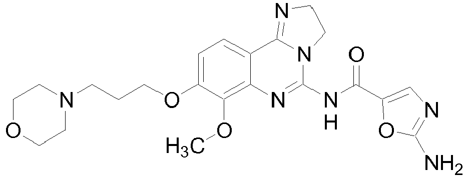 |
| 35 | 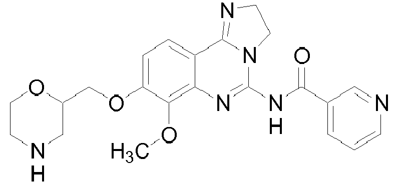 |
| 36 | 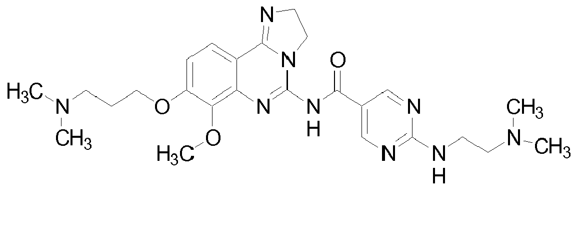 |
| 37 | 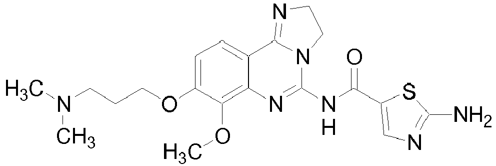 |
| 38 | 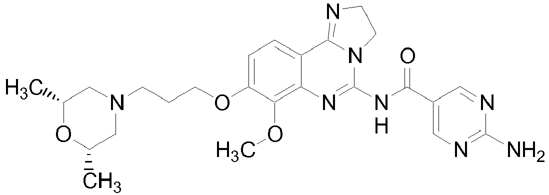 |
| 39 | 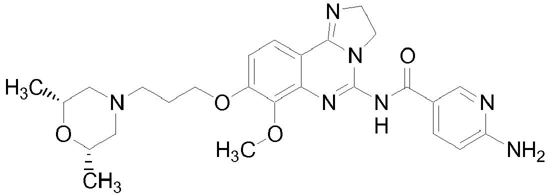 |
| 40 | 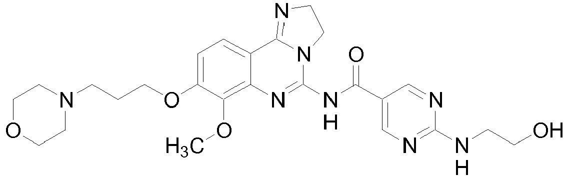 |
| 41 | 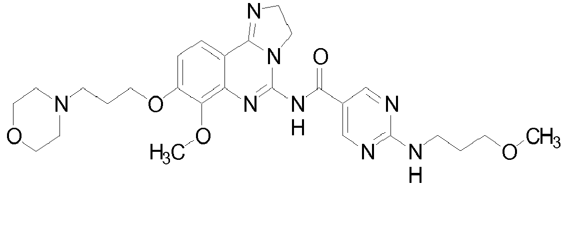 |
| 42 | 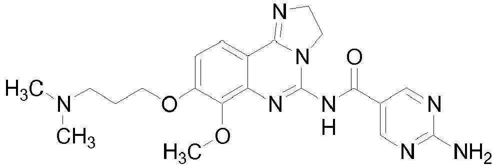 |
| 43 | 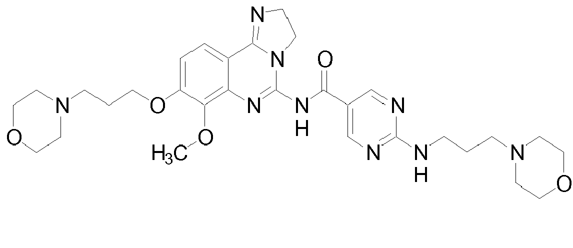 |
| 44 | 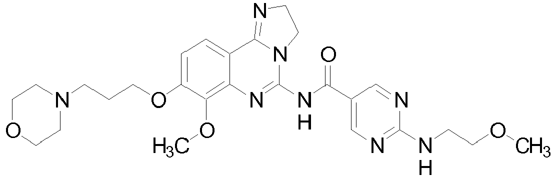 |
| 45 | 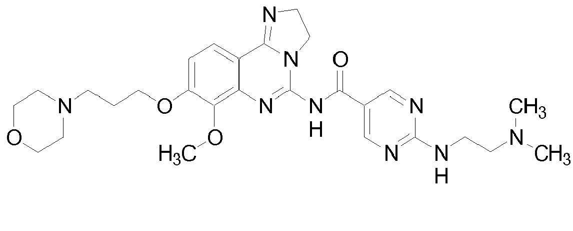 |
| 46 | 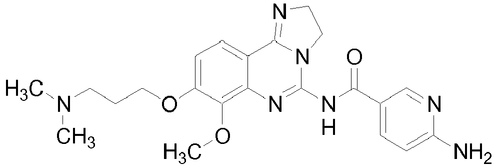 |
| 47 | 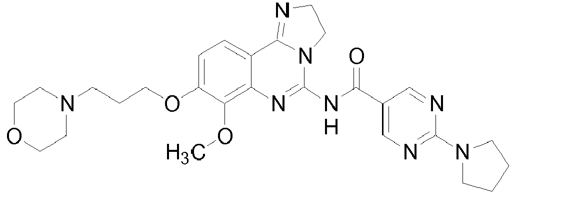 |
| 48 | 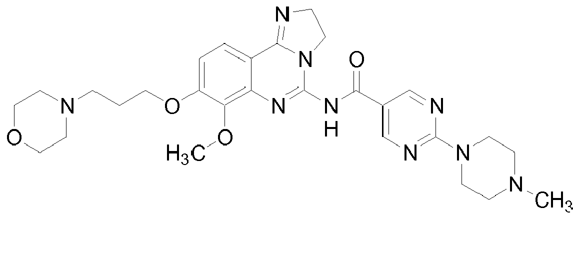 |
| 49 | 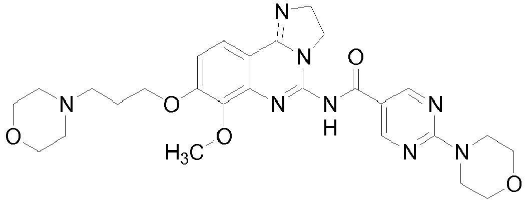 |
| 50 | 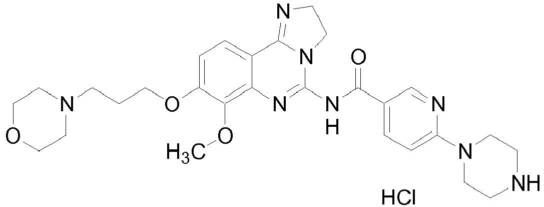 |
| 51 | 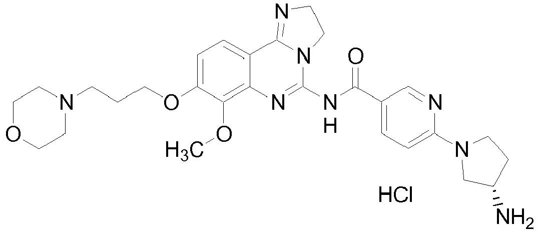 |
| 52 | 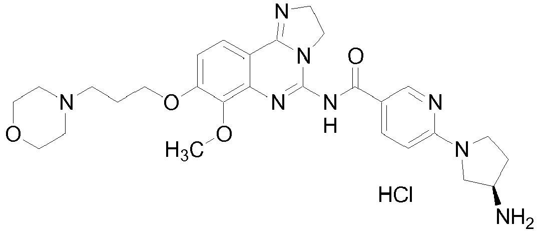 |
| 53 | 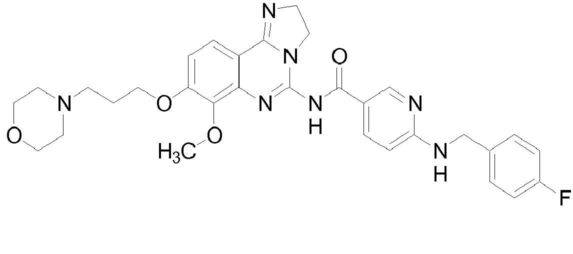 |
| 54 | 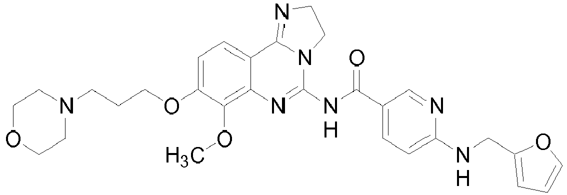 |
| 55 | 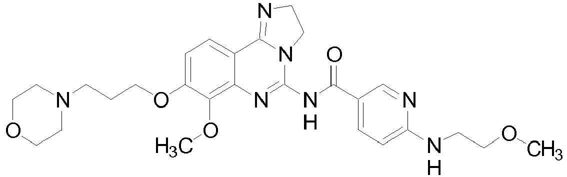 |
| 56 | 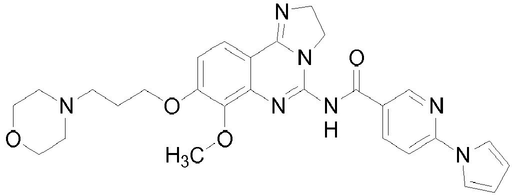 |
| 57 | 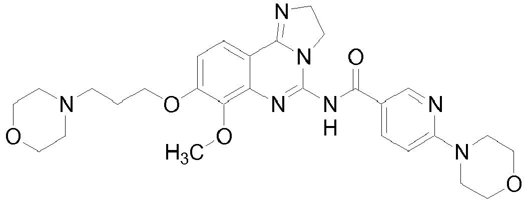 |
| 58 | 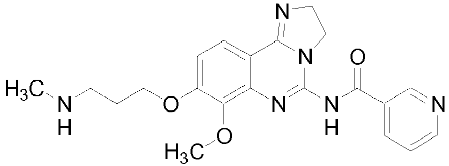 |
| 59 | 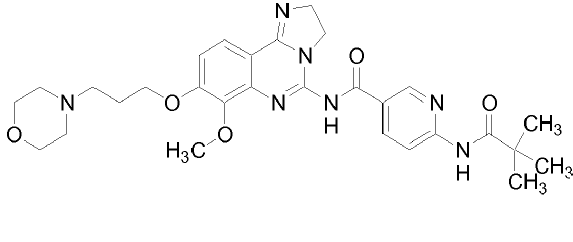 |
| 60 | 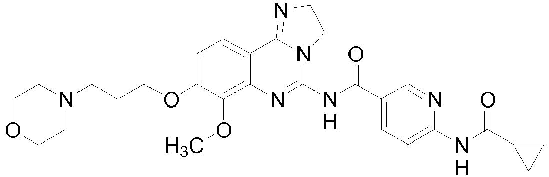 |
| 61 | 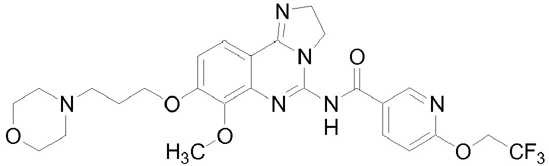 |
| 62 | 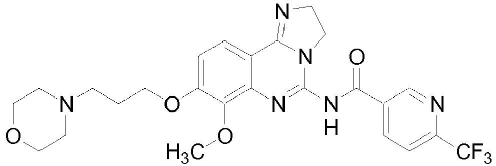 |
| 63 | 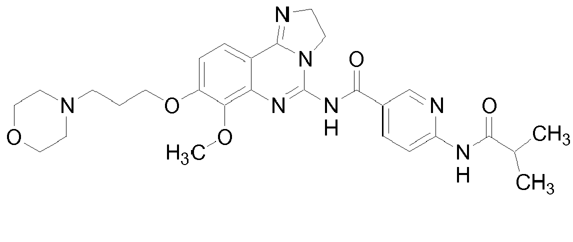 |
| 64 | 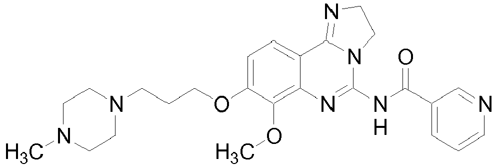 |
| 65 | 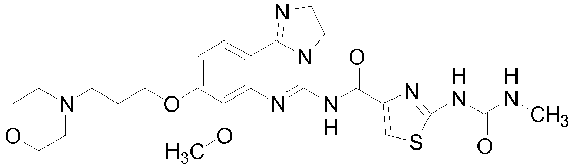 |
| 66 | 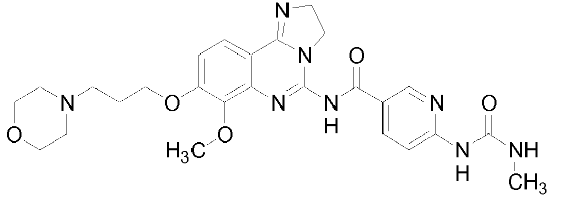 |
| 67 | 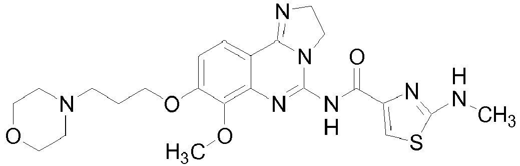 |
| 68 | 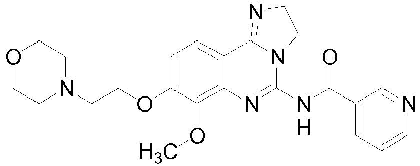 |
| 69 | 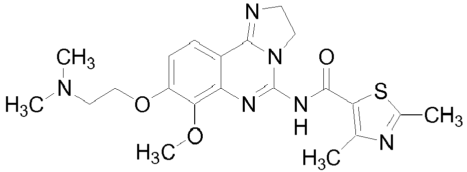 |
| 70 | 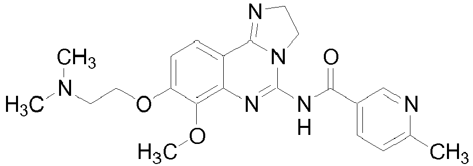 |
| 71 | 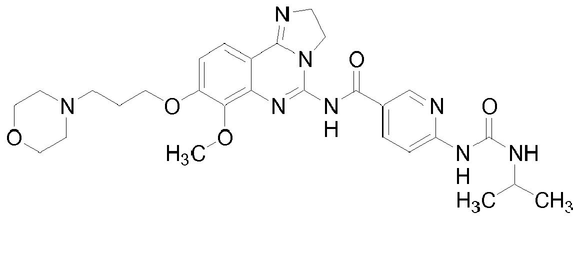 |
| 72 | 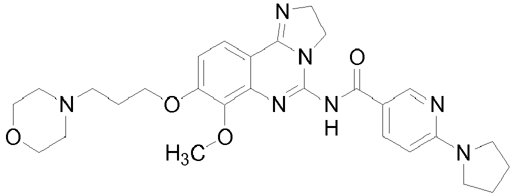 |
| 73 | 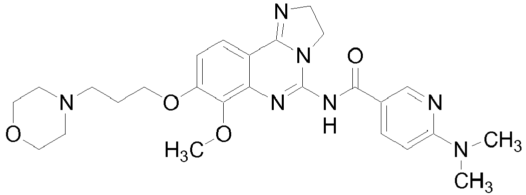 |
| 74 | 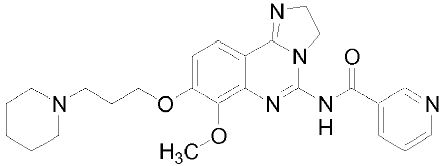 |
| 75 | 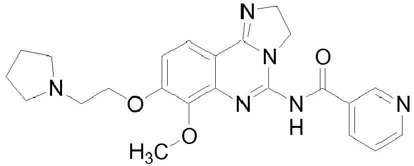 |
| 76 | 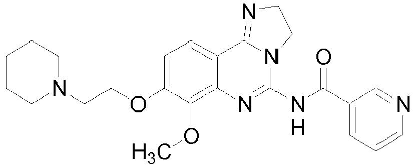 |
| 77 | 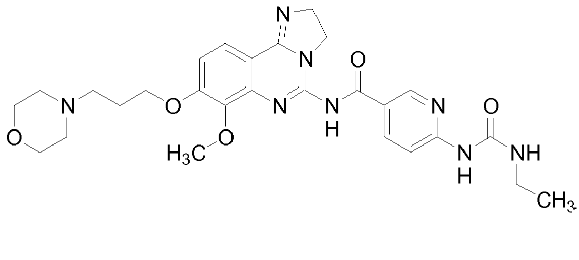 |
| 78 | 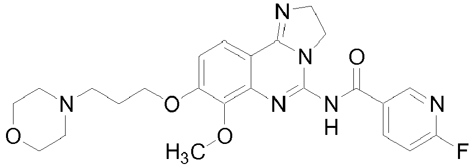 |
| 79 | 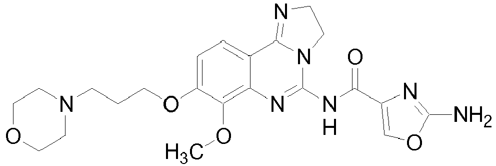 |
| 80 | 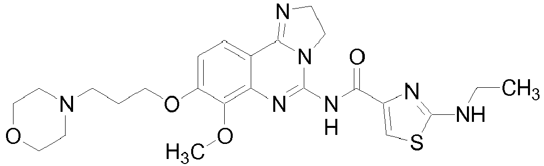 |
| 81 | 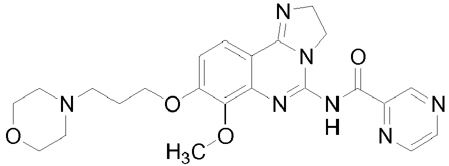 |
| 82 | 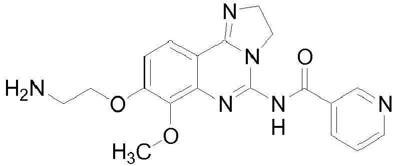 |
| 83 | 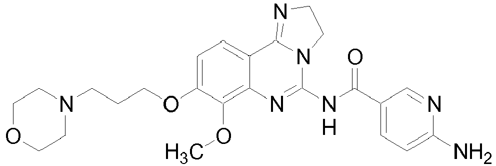 |
| 84 | 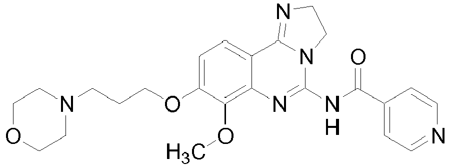 |
| 85 | 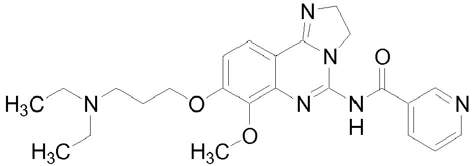 |
| 86 | 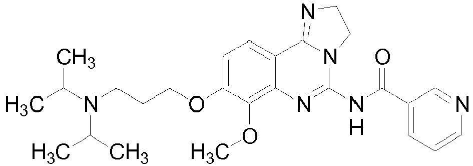 |
| 87 | 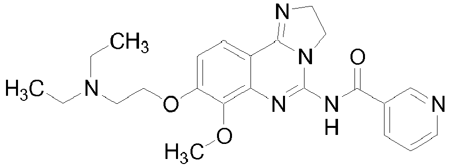 |
| 88 | 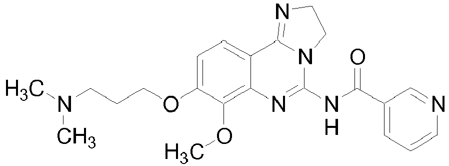 |
| 89 | 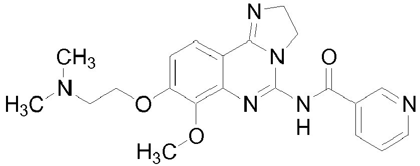 |
| 90 | 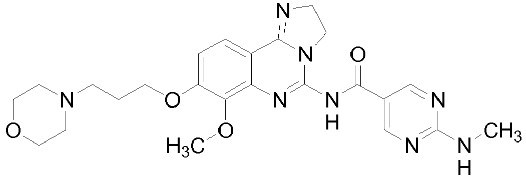 |
| 91 | 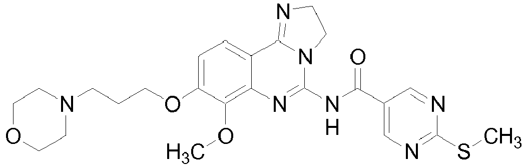 |
| 92 | 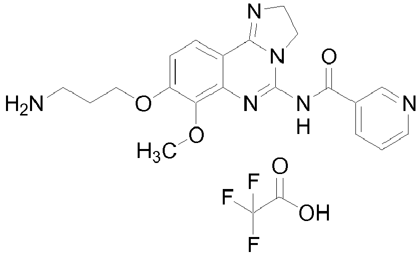 |
| 93 | 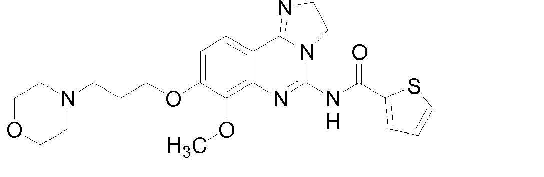 |
| 94 | 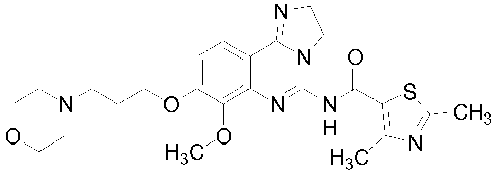 |
| 95 | 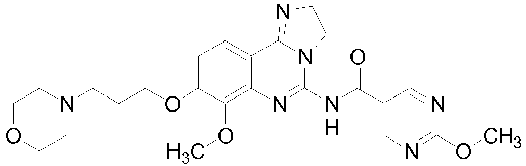 |
| 96 | 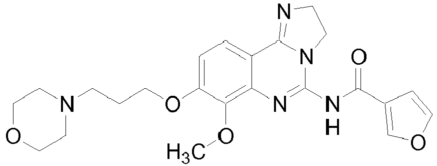 |
| 97 | 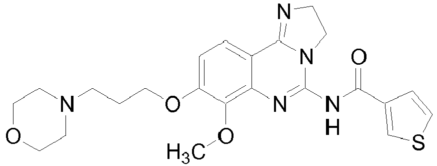 |
| 98 | 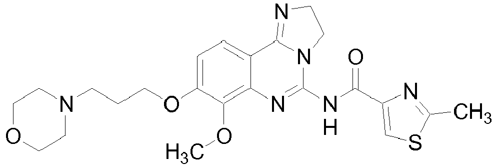 |
| 99 | 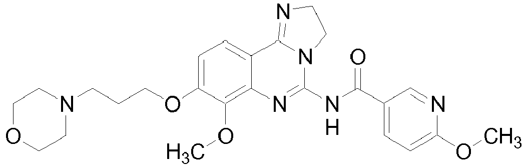 |
| 100 | 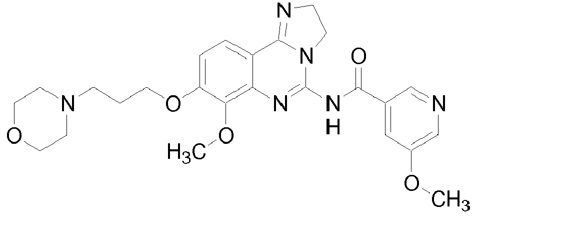 |
| 101 | 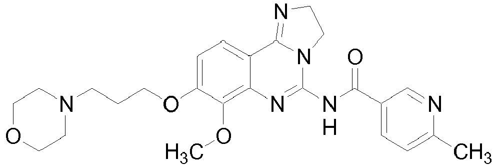 |
| 102 | 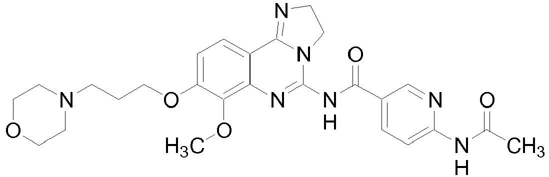 |
| 103 | 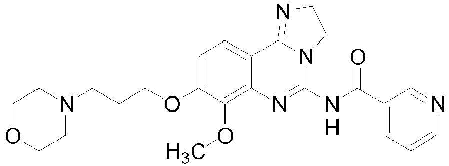 |
2-amino-N-[7-methoxy-8-(3-morpholin-4-ylpropoxy)-2,3-dihydroimid-azo[1,2-c]quinazolin-5-yl]pyrimidine-5-carboxamide dihydrochloride;
or a physiologically acceptable salt, solvate, hydrate or stereoisomer thereof;
optionally in the form of a pharmaceutical formulation which is ready for use to be administered simultaneously, concurrently, separately or sequentially.

**4.** The combination according to any one of claims 1 to 3, wherein :
said component B is one or more IRAK4-inhibiting compounds of general formula (I) as defined herein according to claim 1, which is selected from the list consisting of :
N-[6-(2-hydroxypropan-2-yl)-2-(2-methoxyethyl)-2H-indazol-5-yl]-6-(trifluoromethyl)pyridine-2-carboxamide;
N-[6-(hydroxymethyl)-2-(2-methoxyethyl)-2H-indazol-5-yl]-6-(trifluoromethyl)pyridine-2-carboxamide;
N-[6-(2-hydroxypropan-2-yl)-2-(3-methoxypropyl)-2H-indazol-5-yl]-6-(trifluoromethyl)pyridine-2-carboxamide;
N-[6-(hydroxymethyl)-2-(3-methoxypropyl)-2H-indazol-5-yl]-6-(trifluoromethyl)pyridine-2-carboxamide;
N-[2-(2-hydroxyethyl)-6-(2-hydroxypropan-2-yl)-2H-indazol-5-yl]-6-(trifluoromethyl)pyridine-2-carboxamide;
N-[6-(2-hydroxypropan-2-yl)-2-(3-hydroxypropyl)-2H-indazol-5-yl]-6-(trifluoromethyl)pyridine-2-carboxamide;
N-[2-(2-hydroxyethyl)-6-(hydroxymethyl)-2H-indazol-5-yl]-6-(trifluoromethyl)pyridine-2-carboxamide;
N-[6-(2-hydroxypropan-2-yl)-2-(oxetan-3-ylmethyl)-2H-indazol-5-yl]-6-(trifluoromethyl)pyridine-2-carboxamide;
N-[6-(hydroxymethyl)-2-(oxetan-3-ylmethyl)-2H-indazol-5-yl]-6-(trifluoromethyl)pyridine-2-carboxamide;
N-{6-(2-hydroxypropan-2-yl)-2-[3-(methylsulphonyl)propyl]-2H-indazol-5-yl}-6-(trifluoromethyl)pyridine-2-carboxamide;
N-[2-(3-hydroxy-3-methylbutyl)-6-(2-hydroxypropan-2-yl)-2H-indazol-5-yl]-6-(trifluoromethyl)pyridine-2-carboxamide;
N-{6-(2-hydroxypropan-2-yl)-2-[2-(methylsulphonyl)ethyl]-2H-indazol-5-yl}-6-(trifluoromethyl)pyridine-2-carboxamide;
6-(difluoromethyl)-N-[2-(3-hydroxy-3-methylbutyl)-6-(2-hydroxypropan-2-yl)-2H-indazol-5-yl]pyridine-2-carboxamide;
6-(difluoromethyl)-N-{6-(2-hydroxypropan-2-yl)-2-[2-(methylsulphonyl)ethyl]-2H-indazol-5-yl}pyridine-2-carboxamide;
6-(difluoromethyl)-N-[6-(2-hydroxypropan-2-yl)-2-(3-hydroxypropyl)-2H-indazol-5-yl]pyridine-2-carboxamide;
N-[6-(2-hydroxypropan-2-yl)-2-(4,4,4-trifluorobutyl)-2H-indazol-5-yl]-6-(trifluoromethyl)pyridine-2-carboxamide;
N-{6-(2-hydroxypropan-2-yl)-2-[3-(trifluoromethoxy)propyl]-2H-indazol-5-yl}-6-(trifluoromethyl)pyridine-2-carboxamide;
N-{6-(2-hydroxypropan-2-yl)-2-[3-(2,2,2-trifluoroethoxy)propyl]-2H-indazol-5-yl}-6-(trifluoromethyl)pyridine-2-carboxamide;
5-fluoro-N-[2-(3-hydroxy-3-methylbutyl)-6-(2-hydroxypropan-2-yl)-2H-indazol-5-yl]-6-methylpyridine-2-carboxamide;
N-[2-(3-hydroxy-3-methylbutyl)-6-(2-hydroxypropan-2-yl)-2H-indazol-5-yl]-6-methylpyridine-2-carboxamide; and
6-(2-hydroxypropan-2-yl)-N-[6-(2-hydroxypropan-2-yl)-2-(4,4,4-trifluorobutyl)-2H-indazol-5-yl]pyridine-2-carboxamide;
or a physiologically acceptable salt, solvate, hydrate or stereoisomer thereof;
optionally in the form of a pharmaceutical formulation which is ready for use to be administered simultaneously, concurrently, separately or sequentially.

**5.** The combination according to any one of claims 1 to 4, wherein said component A is 2-amino-N-[7-methoxy-8-(3-morpholin-4-ylpropoxy)-2,3-dihydroimidazo[1,2-c]quinazolin-5-yl]pyrimidine-5-carboxamide or 2-amino-N-[7-methoxy-8-(3-morpholin-4-ylpropoxy)-2,3-dihydroimid-azo[1,2-c]quinazolin-5-yl]pyrimidine-5-carboxamide dihydrochloride.

**6.** A combination of a component A and a component B, wherein:
said component A is 2-amino-N-[7-methoxy-8-(3-morpholin-4-ylpropoxy)-2,3-dihydroimidazo[1,2-c]quinazolin-5-yl]pyrimidine-5-carboxamide, or 2-amino-N-[7-methoxy-8-(3-morpholin-4-ylpropoxy)-2,3-dihydroimid-azo[1,2-c]quinazolin-5-yl]pyrimidine-5-carboxamide dihydrochloride;
or a physiologically acceptable salt, solvate, hydrate or stereoisomer thereof;
optionally in the form of a pharmaceutical formulation which is ready for use to be administered simultaneously, concurrently, separately or sequentially ;
and wherein:
said component B is N-[2-(3-Hydroxy-3-methylbutyl)-6-(2-hydroxypropan-2-yl)-2H-indazol-5-yl]-6-(trifluoromethyl)pyridine-2-carboxamide;
or a physiologically acceptable salt, solvate, hydrate or stereoisomer thereof;
optionally in the form of a pharmaceutical formulation which is ready for use to be administered simultaneously, concurrently, separately or sequentially.

**7.** The combination according to claim 6, wherein:
said component A is 2-amino-N-[7-methoxy-8-(3-morpholin-4-ylpropoxy)-2,3-dihydroimidazo[1,2-c]quinazolin-5-yl]pyrimidine-5-carboxamide,
and wherein:
said component B is N-[2-(3-Hydroxy-3-methylbutyl)-6-(2-hydroxypropan-2-yl)-2H-indazol-5-yl]-6-(trifluoromethyl)pyridine-2-carboxamide.

**8.** The combination according to claim 6, wherein:
said component A is 2-amino-N-[7-methoxy-8-(3-morpholin-4-ylpropoxy)-2,3-dihydroimidazo[1,2-c]quinazolin-5-yl]pyrimidine-5-carboxamide dihydrochloride,
and wherein:
said component B is N-[2-(3-Hydroxy-3-methylbutyl)-6-(2-hydroxypropan-2-yl)-2H-indazol-5-yl]-6-(trifluoromethyl)pyridine-2-carboxamide.

**9.** Use of a combination according to any one of claims 1 to 8 for the preparation of a medicament for the treatment or prophylaxis of a cancer, particularly non-Hodgkin's lymphoma (hereinafter abbreviated to "NHL"), particularly 1st line, 2nd line, relapsed, refractory, indolent or aggressive non-Hodgkin's lymphoma (NHL), in particular follicular lymphoma (hereinafter abbreviated to "FL"), chronic lymphocytic leukaemia (hereinafter abbreviated to "CLL"), marginal zone lymphoma (hereinafter abbreviated to "MZL"), diffuse large B-cell lymphoma (hereinafter abbreviated to "DLBCL"), mantle cell lymphoma (MCL), transformed lymphoma (hereinafter abbreviated to "TL"), or peripheral T-cell lymphoma (hereinafter abbreviated to "PTCL").

**10.** Use according to claim 9, wherein said cancer is non-Hodgkin's lymphoma.

**11.** Use according to claim 9 or 10, wherein said cancer is diffuse large B-cell lymphoma.

**12.** A method of treatment or prophylaxis of a cancer, particularly lung cancer, in particular non-Hodgkin's lymphoma (hereinafter abbreviated to "NHL"), particularly 1st line, 2nd line, relapsed, refractory, indolent or aggressive non-Hodgkin's lymphoma (NHL), in particular follicular lymphoma (hereinafter abbreviated to "FL"), chronic lymphocytic leukaemia (hereinafter abbreviated to "CLL"), marginal zone lymphoma (hereinafter abbreviated to "MZL"), diffuse large B-cell lymphoma (hereinafter abbreviated to "DLBCL"), mantle cell lymphoma (MCL), transformed lymphoma (hereinafter abbreviated to "TL"), or peripheral T-cell lymphoma (hereinafter abbreviated to "PTCL"), in subject, comprising administering to said subject a therapeutically effective amount of a combination accoring to any one of claims 1 to 8.

**13.** A kit comprising a combination of:
component A : one or more 2,3-dihydroimidazo[1,2-c]quinazoline compounds of general formula (A1) or (A2) as defined herein, or a physiologically acceptable salt, solvate, hydrate or stereoisomer thereof, according to any one of claims 1 to 8 ;
component B : one or more IRAK4-inhibiting compounds of general formula (I) as defined herein, or a physiologically acceptable salt, solvate, hydrate or stereoisomer thereof, according to any one of claims 1 to 8 ;
in which optionally both or either of said components A) and B) are in the form of a pharmaceutical formulation which is ready for use to be administered simultaneously, concurrently, separately or sequentially.

**14.** The kit according to claim 13, wherein:
said component A is 2-amino-N-[7-methoxy-8-(3-morpholin-4-ylpropoxy)-2,3-dihydroimidazo[1,2-c]quinazolin-5-yl]pyrimidine-5-carboxamide, or 2-amino-N-[7-methoxy-8-(3-morpholin-4-ylpropoxy)-2,3-dihydroimidazo[1,2-c]quinazolin-5-yl]pyrimidine-5-carboxamide dihydrochloride,
and
said component B is a compound selected from the group consisting of :
N-[6-(2-hydroxypropan-2-yl)-2-(2-methoxyethyl)-2H-indazol-5-yl]-6-(trifluoromethyl)pyridine-2-carboxamide,
N-[6-(hydroxymethyl)-2-(2-methoxyethyl)-2H-indazol-5-yl]-6-(trifluoromethyl)pyridine-2-carboxamide,
N-[6-(2-hydroxypropan-2-yl)-2-(3-methoxypropyl)-2H-indazol-5-yl]-6-(trifluoromethyl)pyridine-2-carboxamide,
N-[6-(hydroxymethyl)-2-(3-methoxypropyl)-2H-indazol-5-yl]-6-(trifluoromethyl)pyridine-2-carboxamide,
N-[2-(2-hydroxyethyl)-6-(2-hydroxypropan-2-yl)-2H-indazol-5-yl]-6-(trifluoromethyl)pyridine-2-carboxamide,
N-[6-(2-hydroxypropan-2-yl)-2-(3-hydroxypropyl)-2H-indazol-5-yl]-6-(trifluoromethyl)pyridine-2-carboxamide,
N-[2-(2-hydroxyethyl)-6-(hydroxymethyl)-2H-indazol-5-yl]-6-(trifluoromethyl)pyridine-2-carboxamide,
N-[6-(2-hydroxypropan-2-yl)-2-(oxetan-3-ylmethyl)-2H-indazol-5-yl]-6-(trifluoromethyl)pyridine-2-carboxamide,
N-[6-(hydroxymethyl)-2-(oxetan-3-ylmethyl)-2H-indazol-5-yl]-6-(trifluoromethyl)pyridine-2-carboxamide,
N-{6-(2-hydroxypropan-2-yl)-2-[3-(methylsulphonyl)propyl]-2H-indazol-5-yl}-6-(trifluoromethyl)pyridine-2-carboxamide,
N-[2-(3-hydroxy-3-methylbutyl)-6-(2-hydroxypropan-2-yl)-2H-indazol-5-yl]-6-(trifluoromethyl)pyridine-2-carboxamide,
N-{6-(2-hydroxypropan-2-yl)-2-[2-(methylsulphonyl)ethyl]-2H-indazol-5-yl}-6-(trifluoromethyl)pyridine-2-carboxamide,
6-(difluoromethyl)-N-[2-(3-hydroxy-3-methylbutyl)-6-(2-hydroxypropan-2-yl)-2H-indazol-5-yl]pyridine-2-carboxamide,
6-(difluoromethyl)-N-{6-(2-hydroxypropan-2-yl)-2-[2-(methylsulphonyl)ethyl]-2H-indazol-5-yl}pyridine-2-carboxamide,
6-(difluoromethyl)-N-[6-(2-hydroxypropan-2-yl)-2-(3-hydroxypropyl)-2H-indazol-5-yl]pyridine-2-carboxamide,
N-[6-(2-hydroxypropan-2-yl)-2-(4,4,4-trifluorobutyl)-2H-indazol-5-yl]-6-(trifluoromethyl)pyridine-2-carboxamide,
N-{6-(2-hydroxypropan-2-yl)-2-[3-(trifluoromethoxy)propyl]-2H-indazol-5-yl}-6-(trifluoromethyl)pyridine-2-carboxamide,
N-{6-(2-hydroxypropan-2-yl)-2-[3-(2,2,2-trifluoroethoxy)propyl]-2H-indazol-5-yl}-6-(trifluoromethyl)pyridine-2-carboxamide,
5-fluoro-N-[2-(3-hydroxy-3-methylbutyl)-6-(2-hydroxypropan-2-yl)-2H-indazol-5-yl]-6-methylpyridine-2-carboxamide,
N-[2-(3-hydroxy-3-methylbutyl)-6-(2-hydroxypropan-2-yl)-2H-indazol-5-yl]-6-methylpyridine-2-carboxamide, and
6-(2-hydroxypropan-2-yl)-N-[6-(2-hydroxypropan-2-yl)-2-(4,4,4-trifluorobutyl)-2H-indazol-5-yl]pyridine-2-carboxamide.

**15.** The kit according to claim 13 or 14, wherein:
said component A is 2-amino-N-[7-methoxy-8-(3-morpholin-4-ylpropoxy)-2,3-dihydroimidazo[1,2-c]quinazolin-5-yl]pyrimidine-5-carboxamide dihydrochloride,
and
said component B is N-[2-(3-Hydroxy-3-methylbutyl)-6-(2-hydroxypropan-2-yl)-2H-indazol-5-yl]-6-(trifluoromethyl)pyridine-2-carboxamide.
